# EUROPEAN PATENT APPLICATION

(11) **EP 4 467 645 A1**
(43) Date of publication of application: **27.11.2024**
(21) Application number: 23742974.1
(22) Date of filing: 19.01.2023
(51) Int. Cl.: C12N 15/113, A61K 31/713, A61P 3/06, A61K 48/00

(54) **DSRNA, USE THEREOF AND PREPARATION METHOD THEREFOR**

(30) Priority: 20.01.2022 CN 202210064031
(71) Applicant: Tuojie Biotech (Shanghai) Co., Ltd., Shanghai 201203 (CN)
(72) Inventor: LI, Yunfei, Shanghai 201203 (CN); DENG, Yongyan, Shanghai 201203 (CN); LIN, Xiaoyan, Shanghai 201203 (CN); WANG, Yanhui, Shanghai 201203 (CN); MAO, Song, Shanghai 201203 (CN); HUANG, Minyin, Shanghai 201203 (CN); ZHOU, Yaqin, Shanghai 201203 (CN); HUANG, Longfei, Shanghai 201203 (CN)
(74) Representative: Dragotti & Associati S.R.L.
(86) International application number: PCT/CN2023/073166
(87) International publication number: WO 2023/138659

(57) **Abstract**

Provided are a dsRNA, and the use thereof and a preparation method therefor. Further provided are a pharmaceutical composition, a cell or a kit containing the dsRNA. The dsRNA can interfere with the expression of APOC3, and prevent and/or treat related diseases.

## Description

The present disclosure claims priority to Chinese Patent Application No. 202210064031.8, filed on Jan. 20, 2022, which is incorporated herein by reference in its entirety.

### TECHNICAL FIELD

The present disclosure relates to the technical field of using dsRNA to reduce the expression of the target RNA and treating related diseases. In particular, the present disclosure relates to the preparation and use of dsRNA.

### BACKGROUND

RNA interference (RNAi) is an effective way to silence gene expression. Statistically, about more than 80% of the proteins related to diseases in humans are non-druggable proteins as they cannot be targeted by conventional small-molecule drugs and biomacromolecule formulations. By using RNA interference technology, proper dsRNAs can be designed according to the mRNAs encoding these proteins to specifically target and degrade the target mRNAs, thereby inhibiting the production of the related proteins. Therefore, dsRNAs have very important prospects for drug development. However, to achieve the therapeutic RNA interference effect in vivo, delivery to specific cells in vivo is needed.

One effective way of delivering a drug is by conjugating it with a targeting ligand so that it can enter cells through endocytosis using the targeting ligand and the molecular structure of the receptor on the surface of the cell membrane. For example, the asialoglycoprotein receptors (ASGPRs) are receptors specifically expressed by hepatocytes and, on the surface of hepatocytes, are characterized by their high abundance and rapid intracellular and extracellular transformation. Monosaccharide and polysaccharide molecules such as galactose, galactosamine, and N-acetylgalactosamine have high affinities for ASGPR. According to the literature (10.16476/j.pibb.2015.0028), RNA may be efficiently delivered to hepatocytes using clusters of N-acetylgalactosamine (GalNAc) molecules; when GalNAc molecules are designed as trivalent or tetravalent molecular clusters, the ability of monovalent or divalent GalNAc molecules to target hepatocytes can be significantly improved.

Different cluster structures and different modes of connection with RNA significantly affect the in vivo activity of dsRNA. Higher activity means a better therapeutic effect or a lower dosage of medication, and further, a lower dosage of medication that achieves an equivalent therapeutic effect means lower toxicity.

APOC3 is primarily synthesized in the liver and plays an important role in the production, metabolism, and clearance of triglyceride-rich lipoproteins from the plasma. The expression of APOC3 in the liver promotes the secretion of very low-density lipoproteins (VLDL), which are rich in triglycerides. In addition, APOC3 can also inhibit the catabolism of triglyceride-rich lipoproteins by inhibiting the activity of lipoprotein lipase and hepatic lipase, thereby increasing serum triglyceride levels. Furthermore, APOC3 can also inhibit the hepatic clearance of triglyceride-rich lipoproteins and their residual particles by interfering with triglyceride-rich lipoproteins and binding to hepatic receptors.

Elevated APOC3 levels are associated with elevated triglyceride levels and diseases such as cardiovascular disease, metabolic syndrome, obesity, and diabetes. In recent years, APOC3 has become a promising target for the treatment of diseases related to hypertriglyceridemia. Elevated serum triglyceride levels have been identified as an independent risk factor for cardiovascular disease and a contributing factor to the development of atherosclerosis. Individuals with severe hypertriglyceridemia are also at a high risk of developing recurrent pancreatitis.

### SUMMARY

In a first aspect, the present disclosure provides a double-stranded ribonucleic acid (dsRNA) comprising a sense strand and an antisense strand that comprise contiguous nucleotides in a direction from the 5' end to the 3' end, wherein the nucleotides at positions 7, 8, and 9 of the sense strand are 2'-fluoro-modified nucleotides, the nucleotide at position 5 is independently a 2'-methoxy-modified nucleotide or a 2'-fluoro-modified nucleotide, and the nucleotides at the other positions are 2'-methoxy-modified nucleotides; the nucleotides at positions 2 and 14 of the antisense strand are 2'-fluoro-modified nucleotides, the nucleotides at positions 4, 6, 8, 9, 10, 12, 16, and 18 are independently 2'-methoxy or 2'-fluoro-modified nucleotides, and the nucleotides at the other positions are 2'-methoxy-modified nucleotides; the number of 2'-fluoro-modified nucleotides in the antisense strand is 2-7;
the antisense strand comprises a chemical modification represented by formula (I), a tautomer thereof, or a pharmaceutically acceptable salt thereof at at least one of nucleotide positions 2 to 8 of the antisense strand: 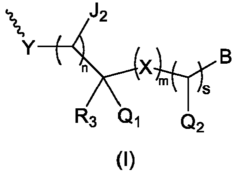
wherein Y is selected from the group consisting of O, NH, and S;
each X is independently selected from the group consisting of CR₄(R₄'), S, NR₅, and NH-CO, wherein R₄, R₄', and R₅ are each independently H or C₁-C₆ alkyl;
J₂ is H or C₁-C₆ alkyl;
n = 0, 1, or 2; m = 0, 1, or 2; s = 0 or 1;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
Q₁ is 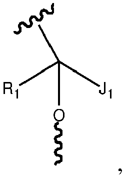 and Q₂ is R₂; or Q₁ is R₂, and Q₂ is 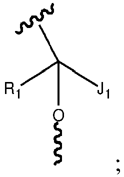
wherein:
   R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3; J₁ is H or C₁-C₆ alkyl;
   R₂ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
   optionally, R₁ and R₂ are directly linked to form a ring;
   B is a base;
   the chemical modification represented by formula (I), the tautomer thereof, or the pharmaceutically acceptable salt thereof is not

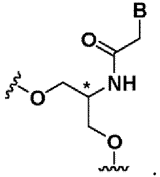

In some embodiments (certain groups or features are defined below, and undefined groups or features are as described in any other embodiment, hereinafter simply "in some embodiments"), when X is NH-CO, R₁ is not H.

In some embodiments, a 2'-methoxy modification is substituted for the chemical modification represented by formula (I), the tautomer thereof, or the pharmaceutically acceptable salt thereof.

In some embodiments, at least one of the nucleotides at positions 2 to 8 of the antisense strand is a 2'-methoxy-modified nucleotide.

In some specific embodiments, the nucleotides at positions 2, 4, 6, 10, 12, 14, and 16 from the 5' end of the antisense strand are 2'-fluoro-modified nucleotides.

In some specific embodiments, the nucleotides at positions 2, 4, 6, 10, 14, and 16 from the 5' end of the antisense strand are 2'-fluoro-modified nucleotides.

In some specific embodiments, the nucleotides at positions 2, 4, 6, 12, 14, and 16 from the 5' end of the antisense strand are 2'-fluoro-modified nucleotides.

In some specific embodiments, the nucleotides at positions 2, 6, 10, 12, 14, and 16 from the 5' end of the antisense strand are 2'-fluoro-modified nucleotides.

In some specific embodiments, the nucleotides at positions 2, 6, 14, and 16 from the 5' end of the antisense strand are 2'-fluoro-modified nucleotides.

In some specific embodiments, the nucleotides at positions 2, 4, 6, 14, and 16 from the 5' end of the antisense strand are 2'-fluoro-modified nucleotides.

In some specific embodiments, the nucleotides at positions 2, 6, 10, 14, and 16 from the 5' end of the antisense strand are 2'-fluoro-modified nucleotides.

In some specific embodiments, the nucleotides at positions 2, 6, 12, 14, and 16 from the 5' end of the antisense strand are 2'-fluoro-modified nucleotides.

In some specific embodiments, the nucleotides at positions 6, 14, and 16 from the 5' end of the antisense strand are 2'-fluoro-modified nucleotides.

In some specific embodiments, the nucleotides at positions 2, 14, and 16 from the 5' end of the antisense strand are 2'-fluoro-modified nucleotides.

In some specific embodiments, the nucleotides at positions 2, 6, and 14 from the 5' end of the antisense strand are 2'-fluoro-modified nucleotides.

In some embodiments, the chemical modification represented by formula (I) is selected from the group consisting of chemical modifications represented by formula (1-1): 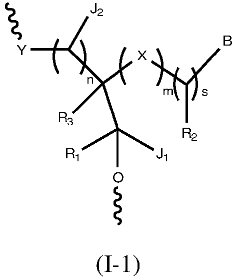
wherein Y is selected from the group consisting of O, NH, and S;
each X is independently selected from the group consisting of CR₄(R₄'), S, NR₅, and NH-CO, wherein R₄, R₄', and R₅ are each independently H or C₁-C₆ alkyl;
each J₁ and each J₂ are each independently H or C₁-C₆ alkyl;
n = 0, 1, or 2; m = 0, 1, or 2; s = 0 or 1;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
R₂ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
optionally, R₁ and R₂ are directly linked to form a ring;
B is as defined in formula (I).

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, the chemical modification represented by formula (I) is selected from the group consisting of chemical modifications represented by formula (I-2): 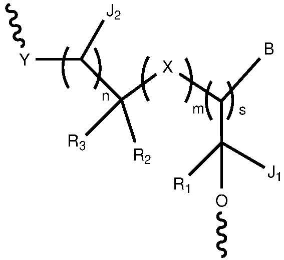 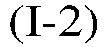
wherein Y is selected from the group consisting of O, NH, and S;
each X is independently selected from the group consisting of CR₄(R₄'), S, NR₅, and NH-CO, wherein R₄, R₄', and R₅ are each independently H or C₁-C₆ alkyl;
n = 0, 1, or 2; m = 0, 1, or 2; s = 0 or 1;
each J₁ and each J₂ are each independently H or C₁-C₆ alkyl;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
R₂ is selected from the group consisting of H, C₁-C₆ alkyl, C₁-C₆ alkoxy, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
optionally, R₁ and R₂ are directly linked to form a ring;
B is as defined in formula (I).

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, each X is independently selected from the group consisting of CR₄(R₄'), S, NR₅, and NH-CO, wherein R₄, R₄', and R₅ are each independently H, methyl, ethyl, n-propyl, or isopropyl;
n = 0, 1, or 2; m = 0, 1, or 2; s = 0 or 1;
each J₁ and each J₂ are each independently H or methyl;
R₃ is selected from the group consisting of H, OH, F, Cl, NH₂, methyl, ethyl, n-propyl, isopropyl, methoxy, ethoxy, n-propoxy, isopropoxy, vinyl, allyl, ethynyl, propargyl, S-CH₃, NCH₃(CH₃), OCH₂CH₂OCH₃, -O-methylamino, -O-ethylamino, and (CH₂)ₚR₆, wherein R₆ is selected from the group consisting of OH, F, Cl, methoxy, ethoxy, N₃, vinyl, allyl, ethynyl, and propargyl, and p = 1 or 2;
R₁ is selected from the group consisting of H, methyl, ethyl, n-propyl, isopropyl, methoxy, ethoxy, n-propoxy, isopropoxy, vinyl, allyl, ethynyl, propargyl, and (CH₂)_{q}R₇, wherein R₇ is selected from the group consisting of OH, F, Cl, methoxy, ethoxy, N₃, vinyl, allyl, ethynyl, and propargyl, and q = 1 or 2;
R₂ is selected from the group consisting of H, OH, F, Cl, NH₂, methyl, ethyl, n-propyl, isopropyl, methoxy, ethoxy, n-propoxy, isopropoxy, vinyl, allyl, ethynyl, propargyl, S-CH₃, NCH₃(CH₃), OCH₂CH₂OCH₃, -O-methylamino, -O-ethylamino, and (CH₂)ᵣR₈ wherein R₈ is selected from the group consisting of OH, F, Cl, methoxy, ethoxy, N₃, vinyl, allyl, ethynyl, and propargyl, and r = 1 or 2;
optionally, R₁ and R₂ are directly linked to form a ring;
B is as defined in formula (I).

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, each X is independently selected from the group consisting of CR₄(R₄'), S, NR₅, and NH-CO, wherein R₄, R₄', and R₅ are each independently H, methyl, ethyl, n-propyl, or isopropyl;
n = 0, 1, or 2; m = 0, 1, or 2; s = 0 or 1;
each J₁ and each J₂ are each independently H or methyl;
R₃ is selected from the group consisting of H, OH, F, Cl, NH₂, methyl, ethyl, n-propyl, isopropyl, methoxy, ethoxy, n-propoxy, isopropoxy, vinyl, allyl, ethynyl, propargyl, S-CH₃, NCH₃(CH₃), OCH₂CH₂OCH₃, -O-methylamino, -O-ethylamino, and (CH₂)ₚR₆, wherein R₆ is selected from the group consisting of OH, F, Cl, methoxy, ethoxy, N₃, vinyl, allyl, ethynyl, and propargyl, and p = 1 or 2;
R₁ is selected from the group consisting of H, methyl, ethyl, n-propyl, isopropyl, methoxy, ethoxy, n-propoxy, isopropoxy, vinyl, allyl, ethynyl, propargyl, and (CH₂)_{q}R₇, wherein R₇ is selected from the group consisting of OH, F, Cl, methoxy, ethoxy, N₃, vinyl, allyl, ethynyl, and propargyl, and q = 1 or 2;
R₂ is selected from the group consisting of H, OH, F, Cl, NH₂, methyl, ethyl, n-propyl, isopropyl, methoxy, ethoxy, n-propoxy, isopropoxy, vinyl, allyl, ethynyl, propargyl, S-CH₃, NCH₃(CH₃), OCH₂CH₂OCH₃, -O-methylamino, -O-ethylamino, and (CH₂)ᵣR₈ wherein R₈ is selected from the group consisting of OH, F, Cl, methoxy, ethoxy, N₃, vinyl, allyl, ethynyl, and propargyl, and r = 1 or 2;
optionally, R₁ and R₂ are directly linked to form a ring;
B is as defined in formula (I).

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, Y is O or NH; each X is independently selected from the group consisting of NH-CO, CH₂, and NH;
n = 0 or 1; m = 0 or 1; s = 0 or 1;
each J₁ and each J₂ are each independently H;
R₁ is selected from the group consisting of H, methyl, and CH₂OH;
R₂ is selected from the group consisting of H, OH, NH₂, methyl, and CH₂OH;
R₃ is selected from the group consisting of H, OH, NH₂, methyl, and CH₂OH;
optionally, R₁ and R₂ are directly linked to form a ring;
B is as defined in formula (I).

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, Y is O or NH; each X is independently selected from the group consisting of NH-CO, CH₂, and NH;
n = 0 or 1; m = 0 or 1; s = 0 or 1;
each J₁ and each J₂ are each independently H;
R₁ is selected from the group consisting of H, methyl, and CH₂OH;
R₂ is selected from the group consisting of H, methyl, and CH₂OH;
R₃ is selected from the group consisting of H, OH, NH₂, methyl, and CH₂OH;
optionally, R₁ and R₂ are directly linked to form a ring;
B is as defined in formula (I).

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, Y is O or NH;
each X is independently selected from the group consisting of CR₄(R₄'), NR₅, and NH-CO, wherein R₄, R₄', and R₅ are each independently H or C₁-C₆ alkyl;
J₂ is H or C₁-C₆ alkyl;
n = 0 or 1; m = 0 or 1; s = 0 or 1;
R₃ is selected from the group consisting of H, OH, NH₂, C₁-C₆ alkyl, C₁-C₆ alkoxy, and (CH₂)ₚR₆, wherein R₆ is selected from the group consisting of OH, methoxy, and ethoxy, and p =1, 2, or 3;
Q₁ is 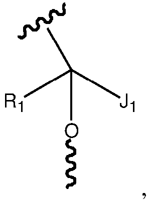 and Q₂ is R₂; or Q₁ is R₂, and Q₂ is 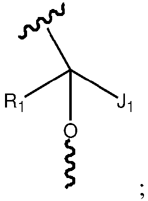
R₁ is selected from H, OH, C₁-C₆ alkyl, C₁-C₆ alkoxy, and (CH₂)_{q}R₇, wherein R₇ is selected from the group consisting of OH, methoxy, and ethoxy, and q =1, 2, or 3;
J₁ is H or C₁-C₆ alkyl;
R₂ is selected from the group consisting of H, OH, C₁-C₆ alkyl, C₁-C₆ alkoxy, and (CH₂)ᵣR₈ wherein R₈ is selected from the group consisting of OH, methoxy, and ethoxy, and r =1, 2, or 3;
optionally, R₁ and R₂ are directly linked to form a 3-6 membered ring;
B is a base;
the chemical modification represented by formula (I), the tautomer thereof, or the pharmaceutically acceptable salt thereof is not

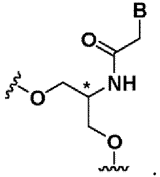

In some embodiments, X is independently selected from the group consisting of CR₄(R₄') and NH-CO.

In some embodiments, X is independently selected from the group consisting of CR₄(R₄'). In some embodiments, R₃ is selected from the group consisting of H, C₁-C₆ alkyl, and (CH₂)ₚR₆.

In some embodiments, R₃ is selected from the group consisting of H and C₁-C₆ alkyl.

In some embodiments, R₁ is selected from the group consisting of H, C₁-C₆ alkyl, and (CH₂)_{q}R₇.

In some embodiments, R₁ is selected from the group consisting of H and C₁-C₆ alkyl.

In some embodiments, R₂ is selected from the group consisting of H, OH, C₁-C₆ alkyl, and (CH₂)ᵣR₈.

In some embodiments, R₂ is selected from the group consisting of H, C₁-C₆ alkyl, and (CH₂)ᵣR₈.

In some embodiments, Y is O;
each X is independently selected from the group consisting of CR₄(R₄') and NH-CO, wherein R₄ and R₄' are each independently H or C₁-C₆ alkyl;
J₂ is H or C₁-C₆ alkyl;
R₃ is selected from the group consisting of H, C₁-C₆ alkyl, and (CH₂)ₚR₆, wherein R₆ is selected from the group consisting of OH, and p =1, 2, or 3;
Q₁ is 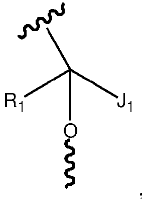 and Q₂ is R₂; or Q₁ is R₂, and Q₂ is 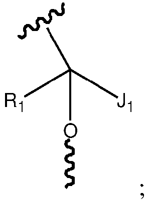
R₁ is selected from the group consisting of H, C₁-C₆ alkyl, and (CH₂)_{q}R₇, wherein R₇ is selected from the group consisting of OH, and q =1, 2, or 3;
J₁ is H or C₁-C₆ alkyl;
R₂ is selected from the group consisting of H, OH, C₁-C₆ alkyl, and (CH₂)ᵣR₈, wherein R₈ is selected from the group consisting of OH, and r =1, 2, or 3;
optionally, R₁ and R₂ are directly linked to form a 5-6 membered ring;
B is a base.

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, Y is O;
each X is independently selected from the group consisting of CR₄(R₄'), wherein R₄ and R₄' are each independently H or C₁-C₆ alkyl;
J₂ is H;
R₃ is selected from the group consisting of H and C₁-C₆ alkyl;
Q₁ is 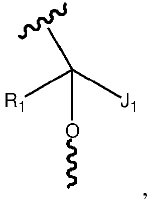 and Q₂ is R₂; or Q₁ is R₂, and Q₂ is 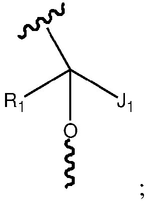
R₁ is selected from the group consisting of H and C₁-C₆ alkyl;
J₁ is H or C₁-C₆ alkyl;
R₂ is selected from the group consisting of H, C₁-C₆ alkyl, and (CH₂)ᵣR₈, wherein R₈ is selected from the group consisting of OH, and r =1, 2, or 3;
optionally, R₁ and R₂ are directly linked to form a 5-6 membered ring;
B is a base.

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, Y is O.

In some embodiments, X is independently selected from the group consisting of CR₄(R₄'), NR₅, and NH-CO, wherein R₄, R₄', and R₅ are each independently H, methyl, ethyl, n-propyl, or isopropyl. In some embodiments, X is independently selected from the group consisting of NH-CO, CH₂, and NH. In some embodiments, X is independently selected from the group consisting of NH-CO and CH₂. In some embodiments, X is CH₂.

In some embodiments, J₂ is H or methyl. In some embodiments, J₂ is H.

In some embodiments, R₃ is selected from the group consisting of H, OH, NH₂, methyl, ethyl, n-propyl, isopropyl, methoxy, ethoxy, n-propoxy, isopropoxy, and (CH₂)ₚR₆, wherein R₆ is selected from the group consisting of OH, methoxy, and ethoxy, and p =1 or 2. In some embodiments, R₃ is selected from the group consisting of H, methyl, ethyl, n-propyl, isopropyl, and (CH₂)ₚR₆, wherein R₆ is selected from the group consisting of OH, and p =1 or 2. In some embodiments, R₃ is selected from the group consisting of H and methyl.

In some embodiments, R₁ is selected from the group consisting of H, OH, methyl, ethyl, n-propyl, isopropyl, methoxy, ethoxy, n-propoxy, isopropoxy, and (CH₂)_{q}R₇, wherein R₇ is selected from the group consisting of OH, and q =1 or 2. In some embodiments, R₁ is selected from the group consisting of H, methyl, ethyl, n-propyl, isopropyl, and (CH₂)_{q}R₇, wherein R₇ is selected from the group consisting of OH, and q =1 or 2. In some embodiments, R₁ is selected from the group consisting of H and methyl.

In some embodiments, R₂ is selected from the group consisting of H, OH, methyl, ethyl, n-propyl, isopropyl, methoxy, ethoxy, n-propoxy, isopropoxy, and (CH₂)ᵣR₈ wherein R₈ is selected from the group consisting of OH, and r =1 or 2. In some embodiments, R₂ is selected from the group consisting of H, OH, methyl, ethyl, n-propyl, isopropyl, and (CH₂)ᵣR₈ wherein R₈ is selected from the group consisting of OH, and r =1 or 2. In some embodiments, R₂ is selected from the group consisting of H, methyl, and CH₂OH.

In some embodiments, R₁ and R₂ are directly linked to form a 5-6 membered ring. In some embodiments, R₁ and R₂ are directly linked to form 3-6 membered cycloalkyl. In some embodiments, R₁ and R₂ are directly linked to form cyclopentyl or cyclohexyl.

In some embodiments, the chemical modification represented by formula (I) is selected from the group consisting of any one of the following structures: 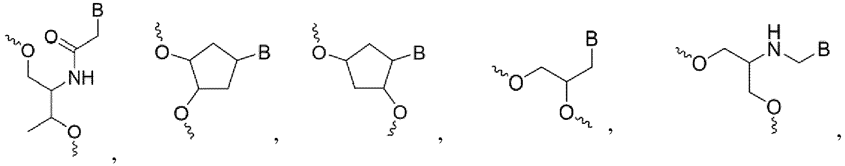 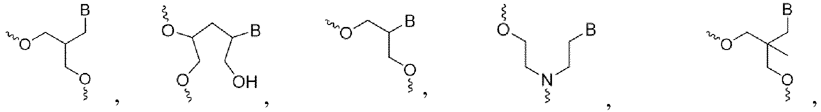 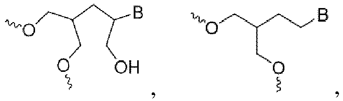 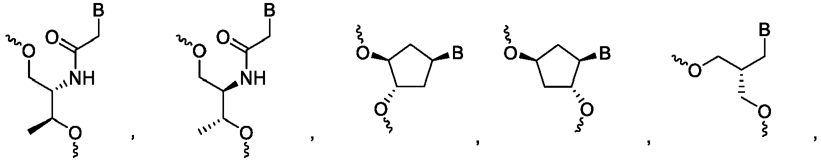 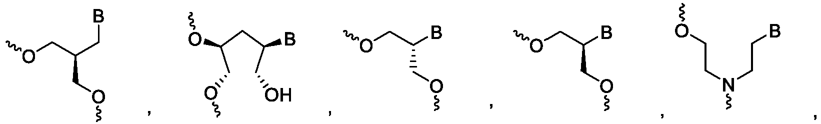 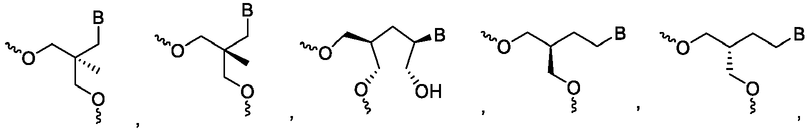 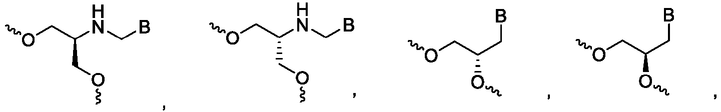 wherein: B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, the chemical modification represented by formula (I) is selected from the group consisting of any one of the following structures: 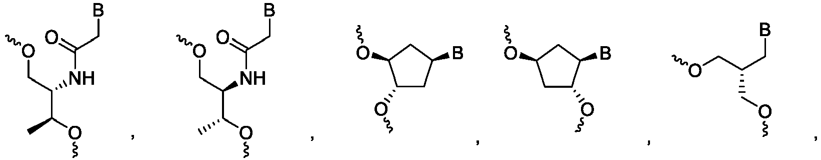 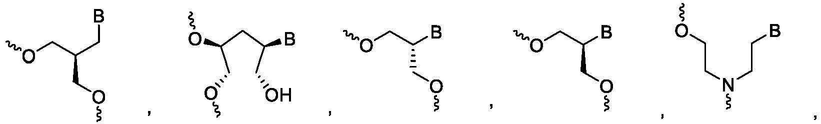 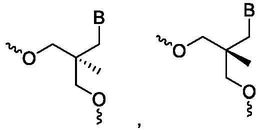 wherein: B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, the chemical modification represented by formula (I) is selected from the group consisting of any one of the following structures: 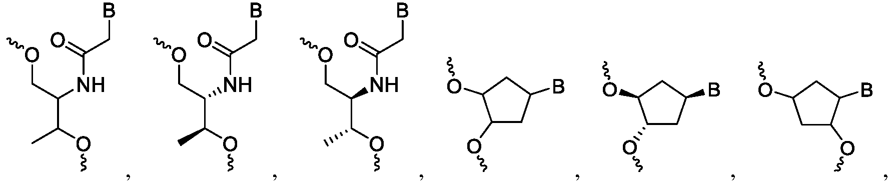 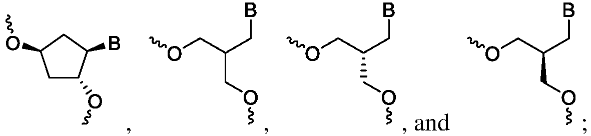 wherein: B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, the chemical modification represented by formula (I) is selected from the group consisting of any one of the following structures: 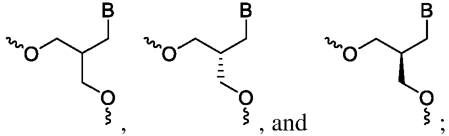 wherein: B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, the nucleotide comprising the chemical modification represented by formula (I), the tautomer thereof, or the pharmaceutically acceptable salt thereof is selected from the group consisting of a nucleotide comprising a chemical modification represented by formula (I'), a tautomer thereof, or a pharmaceutically acceptable salt thereof, 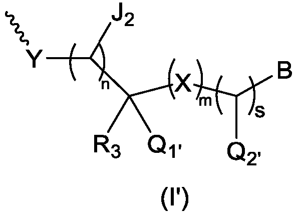
wherein Y is selected from the group consisting of O, NH, and S;
each X is independently selected from the group consisting of CR₄(R₄'), S, NR₅, and NH-CO, wherein R₄, R₄', and R₅ are each independently H or C₁-C₆ alkyl;
J₂ is H or C₁-C₆ alkyl;
n = 0, 1, or 2; m = 0, 1, or 2; s = 0 or 1;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
Q_{1'} is 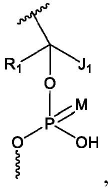 and Q_{2'} is R₂; or Q_{1'} is R₂, and Q_{2'} is 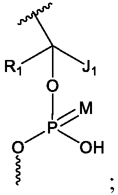
wherein:
   R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
   J₁ is H or C₁-C₆ alkyl;
   R₂ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
   optionally, R₁ and R₂ are directly linked to form a ring;
   B is a base;
   M is O or S;
   the chemical modification represented by formula (I'), the tautomer thereof, or the pharmaceutically acceptable salt thereof is not

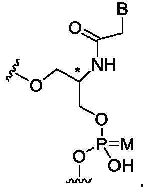

In some embodiments, when X is NH-CO, R₁ is not H.

In some embodiments, a 2'-methoxy modification is substituted for the chemical modification represented by formula (I'), the tautomer thereof, or the pharmaceutically acceptable salt thereof.

In some embodiments, the chemical modification represented by formula (I') is selected from the group consisting of chemical modifications represented by formula (I'-1): 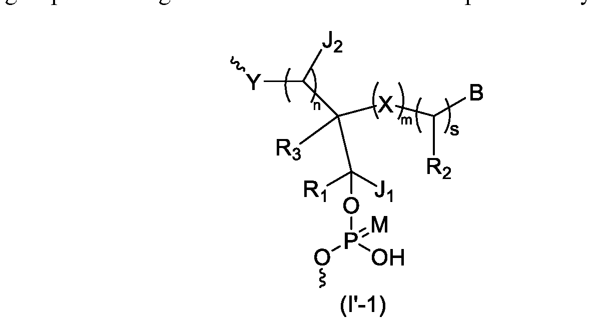
wherein Y is selected from the group consisting of O, NH, and S;
each X is independently selected from the group consisting of CR₄(R₄'), S, NR₅, and NH-CO, wherein R₄, R₄', and R₅ are each independently H or C₁-C₆ alkyl;
each J₁ and each J₂ are each independently H or C₁-C₆ alkyl;
n = 0, 1, or 2; m = 0, 1, or 2; s = 0 or 1;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
R₂ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3; M is O or S;
optionally, R₁ and R₂ are directly linked to form a ring;
B is as defined in formula (I').

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, the chemical modification represented by formula (I') is selected from the group consisting of chemical modifications represented by formula (I'-2): 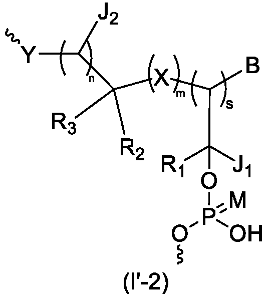
wherein Y is selected from the group consisting of O, NH, and S;
each X is independently selected from the group consisting of CR₄(R₄'), S, NR₅, and NH-CO, wherein R₄, R₄', and R₅ are each independently H or C₁-C₆ alkyl;
n = 0, 1, or 2; m = 0, 1, or 2; s = 0 or 1;
each J₁ and each J₂ are each independently H or C₁-C₆ alkyl;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
R₂ is selected from the group consisting of H, C₁-C₆ alkyl, C₁-C₆ alkoxy, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
optionally, R₁ and R₂ are directly linked to form a ring;
M is O or S;
B is as defined in formula (I').

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, each X is independently selected from the group consisting of CR₄(R₄'), S, NR₅, and NH-CO, wherein R₄, R₄', and R₅ are each independently H or C₁-C₃ alkyl;
n = 0, 1, or 2; m = 0, 1, or 2; s = 0 or 1;
each J₁ and each J₂ are each independently H or C₁-C₃ alkyl;
R₃ is selected from the group consisting of H, OH, halogen, NH₂, C₁-C₃ alkyl, C₁-C₃ alkoxy, C₂-C₄ alkenyl, C₂-C₄ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₃ alkyl, C₁-C₃ alkoxy, C₂-C₄ alkenyl, C₂-C₄ alkynyl, and (CH₂)_{q}R₇, wherein R₇ is selected from the group consisting of OH, halogen, methoxy, ethoxy, N₃, C₂-C₄ alkenyl, and C₂-C₄ alkynyl, and q = 1, 2, or 3;
R₂ is selected from the group consisting of H, OH, halogen, NH₂, C₁-C₃ alkyl, C₁-C₃ alkoxy, C₂-C₄ alkenyl, C₂-C₄ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₄ alkenyl, and C₂-C₄ alkynyl, and r = 1, 2, or 3;
optionally, R₁ and R₂ are directly linked to form a ring;
B is as defined in formula (I').

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, each X is independently selected from the group consisting of CR₄(R₄'), S, NR₅, and NH-CO, wherein R₄, R₄', and R₅ are each independently H, methyl, ethyl, n-propyl, or isopropyl;
n = 0, 1, or 2; m = 0, 1, or 2; s = 0 or 1;
each J₁ and each J₂ are each independently H or methyl;
R₃ is selected from the group consisting of H, OH, F, Cl, NH₂, methyl, ethyl, n-propyl, isopropyl, methoxy, ethoxy, n-propoxy, isopropoxy, vinyl, allyl, ethynyl, propargyl, S-CH₃, NCH₃(CH₃), OCH₂CH₂OCH₃, -O-methylamino, -O-ethylamino, and (CH₂)ₚR₆, wherein R₆ is selected from the group consisting of OH, F, Cl, methoxy, ethoxy, N₃, vinyl, allyl, ethynyl, and propargyl, and p = 1 or 2;
R₁ is selected from the group consisting of H, methyl, ethyl, n-propyl, isopropyl, methoxy, ethoxy, n-propoxy, isopropoxy, vinyl, allyl, ethynyl, propargyl, and (CH₂)_{q}R₇, wherein R₇ is selected from the group consisting of OH, F, Cl, methoxy, ethoxy, N₃, vinyl, allyl, ethynyl, and propargyl, and q = 1 or 2;
R₂ is selected from the group consisting of H, OH, F, Cl, NH₂, methyl, ethyl, n-propyl, isopropyl, methoxy, ethoxy, n-propoxy, isopropoxy, vinyl, allyl, ethynyl, propargyl, S-CH₃, NCH₃(CH₃), OCH₂CH₂OCH₃, -O-methylamino, -O-ethylamino, and (CH₂)ᵣR₈ wherein R₈ is selected from the group consisting of OH, F, Cl, methoxy, ethoxy, N₃, vinyl, allyl, ethynyl, and propargyl, and r = 1 or 2;
optionally, R₁ and R₂ are directly linked to form a ring;
B is as defined in formula (I').

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments of formula (I'), formula (I'-1), or formula (I'-2), Y is O or NH; each X is independently selected from the group consisting of NH-CO, CH₂, and NH;
n = 0 or 1; m = 0 or 1; s = 0 or 1;
each J₁ and each J₂ are each independently H;
R₁ is selected from the group consisting of H, methyl, and CH₂OH;
R₂ is selected from the group consisting of H, OH, NH₂, methyl, and CH₂OH;
R₃ is selected from the group consisting of H, OH, NH₂, methyl, and CH₂OH;
optionally, R₁ and R₂ are directly linked to form a ring;
B is as defined in formula (I').

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments of formula (I'), formula (I'-1), or formula (I'-2), Y is O or NH; each X is independently selected from the group consisting of NH-CO, CH₂, and NH;
n = 0 or 1; m = 0 or 1; s = 0 or 1;
each J₁ and each J₂ are each independently H;
R₁ is selected from the group consisting of H, methyl, and CH₂OH;
R₂ is selected from the group consisting of H, methyl, and CH₂OH;
R₃ is selected from the group consisting of H, OH, NH₂, methyl, and CH₂OH;
optionally, R₁ and R₂ are directly linked to form a ring;
B is as defined in formula (I').

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, Y is O or NH;
each X is independently selected from the group consisting of CR₄(R₄'), NR₅ and NH-CO, wherein R₄, R₄', and R₅ are each independently H or C₁-C₆ alkyl;
J₂ is H or C₁-C₆ alkyl;
n = 0 or 1; m = 0 or 1; s = 0 or 1;
R₃ is selected from the group consisting of H, OH, NH₂, C₁-C₆ alkyl, C₁-C₆ alkoxy, and (CH₂)ₚR₆, wherein R₆ is selected from the group consisting of OH, methoxy, and ethoxy, and p =1, 2, or 3;
Q_{1'} is 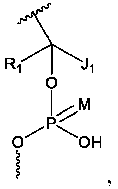 and Q_{2'} is R₂; or Q_{1'} is R₂, and Q_{2'} is 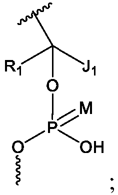
R₁ is selected from H, OH, C₁-C₆ alkyl, C₁-C₆ alkoxy, and (CH₂)_{q}R₇, wherein R₇ is selected from the group consisting of OH, methoxy, and ethoxy, and q =1, 2, or 3;
J₁ is H or C₁-C₆ alkyl;
R₂ is selected from the group consisting of H, OH, C₁-C₆ alkyl, C₁-C₆ alkoxy, and (CH₂)ᵣR₈, wherein R₈ is selected from the group consisting of OH, methoxy, and ethoxy, and r =1, 2, or 3;
optionally, R₁ and R₂ are directly linked to form a 3-6 membered ring;
M is O or S;
B is a base;
the chemical modification represented by formula (I'), the tautomer thereof, or the pharmaceutically acceptable salt thereof is not

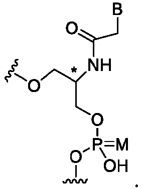

In some embodiments, X is independently selected from the group consisting of CR₄(R₄') and NH-CO.

In some embodiments, X is independently selected from the group consisting of CR₄(R₄'). In some embodiments, R₃ is selected from the group consisting of H, C₁-C₆ alkyl, and (CH₂)ₚR₆.

In some embodiments, R₃ is selected from the group consisting of H and C₁-C₆ alkyl.

In some embodiments, R₁ is selected from the group consisting of H, C₁-C₆ alkyl, and (CH₂)_{q}R₇.

In some embodiments, R₁ is selected from the group consisting of H and C₁-C₆ alkyl.

In some embodiments, R₂ is selected from the group consisting of H, OH, C₁-C₆ alkyl, and (CH₂)ᵣR₈.

In some embodiments, R₂ is selected from the group consisting of H, C₁-C₆ alkyl, and (CH₂)ᵣR₈.

In some embodiments, Y is O;
each X is independently selected from the group consisting of CR₄(R₄') and NH-CO,
wherein R₄ and R₄' are each independently H or C₁-C₆ alkyl;
J₂ is H or C₁-C₆ alkyl;
R₃ is selected from the group consisting of H, C₁-C₆ alkyl, and (CH₂)ₚR₆, wherein R₆ is selected from the group consisting of OH, and p =1, 2, or 3;
Q_{1'} is 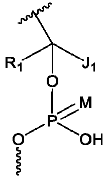 , and Q_{2'} is R₂; or Q_{1'} is R₂, and Q_{2'} is 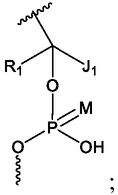
R₁ is selected from the group consisting of H, C₁-C₆ alkyl, and (CH₂)_{q}R₇, wherein R₇ is selected from the group consisting of OH, and q =1, 2, or 3;
J₁ is H or C₁-C₆ alkyl;
R₂ is selected from the group consisting of H, OH, C₁-C₆ alkyl, and (CH₂)ᵣR₈, wherein R₈ is selected from the group consisting of OH, and r =1, 2, or 3;
optionally, R₁ and R₂ are directly linked to form a 5-6 membered ring;
M is O or S;
B is a base.

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, Y is O;
each X is independently selected from the group consisting of CR₄(R₄'), wherein R₄ and R₄' are each independently H or C₁-C₆ alkyl;
J₂ is H;
R₃ is selected from the group consisting of H and C₁-C₆ alkyl;
Q_{1'} is 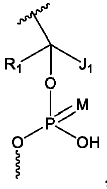 and Q_{2'} is R₂; or Q_{1'} is R₂, and Q_{2'} is 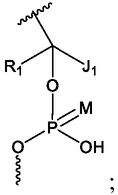
R₁ is selected from the group consisting of H and C₁-C₆ alkyl;
J₁ is H or C₁-C₆ alkyl;
R₂ is selected from the group consisting of H, C₁-C₆ alkyl, and (CH₂)ᵣR₈, wherein R₈ is selected from the group consisting of OH, and r =1, 2, or 3;
optionally, R₁ and R₂ are directly linked to form a 5-6 membered ring;
M is O or S;
B is a base.

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, Y is O.

In some embodiments, X is independently selected from the group consisting of CR₄(R₄'), NR₅, and NH-CO, wherein R₄, R₄', and R₅ are each independently H, methyl, ethyl, n-propyl, or isopropyl. In some embodiments, X is independently selected from the group consisting of NH-CO, CH₂, and NH. In some embodiments, X is independently selected from the group consisting of NH-CO and CH₂. In some embodiments, X is CH₂.

In some embodiments, J₂ is H or methyl. In some embodiments, J₂ is H.

In some embodiments, R₃ is selected from the group consisting of H, OH, NH₂, methyl, ethyl, n-propyl, isopropyl, methoxy, ethoxy, n-propoxy, isopropoxy, and (CH₂)ₚR₆, wherein R₆ is selected from the group consisting of OH, methoxy, and ethoxy, and p =1 or 2. In some embodiments, R₃ is selected from the group consisting of H, methyl, ethyl, n-propyl, isopropyl, and (CH₂)ₚR₆, wherein R₆ is selected from the group consisting of OH, and p =1 or 2. In some embodiments, R₃ is selected from the group consisting of H and methyl.

In some embodiments, R₁ is selected from the group consisting of H, OH, methyl, ethyl, n-propyl, isopropyl, methoxy, ethoxy, n-propoxy, isopropoxy, and (CH₂)_{q}R₇, wherein R₇ is selected from the group consisting of OH, and q =1 or 2. In some embodiments, R₁ is selected from the group consisting of H, methyl, ethyl, n-propyl, isopropyl, and (CH₂)_{q}R₇, wherein R₇ is selected from the group consisting of OH, and q =1 or 2. In some embodiments, R₁ is selected from the group consisting of H and methyl.

In some embodiments, R₂ is selected from the group consisting of H, OH, methyl, ethyl, n-propyl, isopropyl, methoxy, ethoxy, n-propoxy, isopropoxy, and (CH₂)ᵣR₈ wherein R₈ is selected from the group consisting of OH, and r =1 or 2. In some embodiments, R₂ is selected from the group consisting of H, OH, methyl, ethyl, n-propyl, isopropyl, and (CH₂)ᵣR₈ wherein R₈ is selected from the group consisting of OH, and r =1 or 2. In some embodiments, R₂ is selected from the group consisting of H, methyl, and CH₂OH.

In some embodiments, R₁ and R₂ are directly linked to form a 5-6 membered ring. In some embodiments, R₁ and R₂ are directly linked to form 3-6 membered cycloalkyl. In some embodiments, R₁ and R₂ are directly linked to form cyclopentyl or cyclohexyl.

In some embodiments, the chemical modification represented by formula (I') is selected from the group consisting of any one of the following structures: 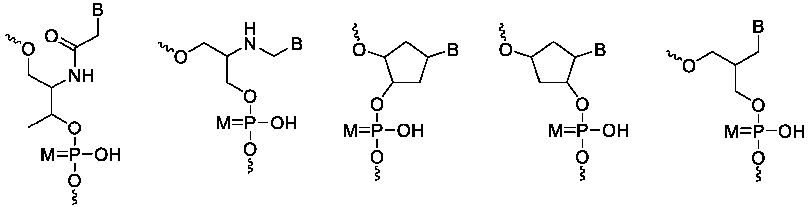 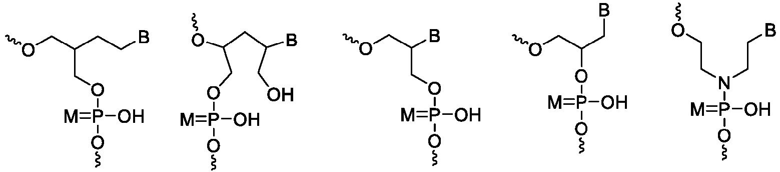 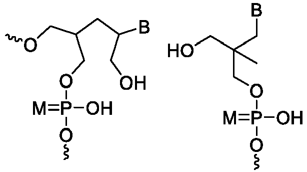 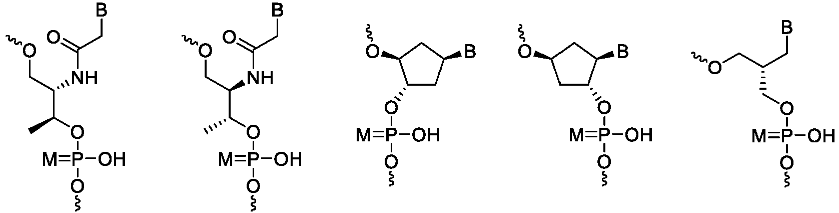 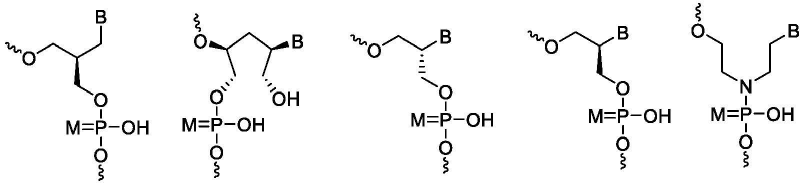 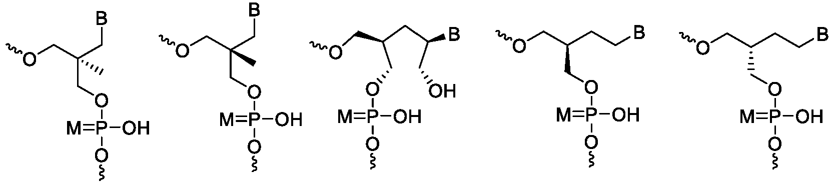 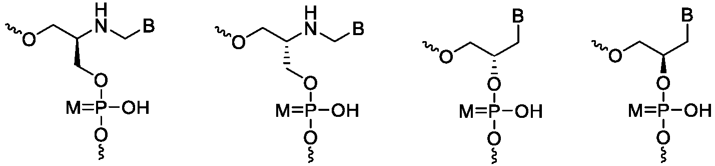
wherein M is O or S;
B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, the chemical modification represented by formula (I') is selected from the group consisting of any one of the following structures: 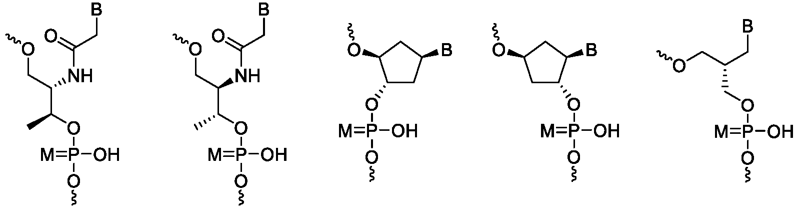 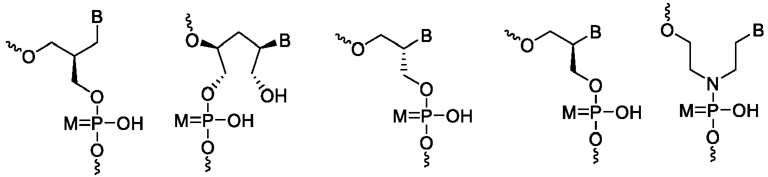 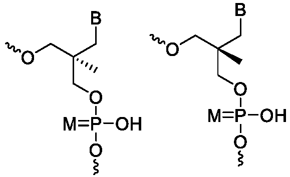
wherein M is O or S;
B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, the chemical modification represented by formula (I') is selected from the group consisting of any one of the following structures: 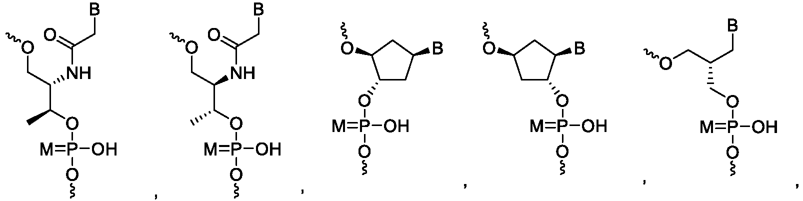 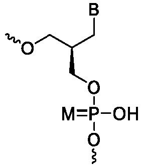
wherein M is O or S;
B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, the chemical modification represented by formula (I') is selected from the group consisting of any one of the following structures: 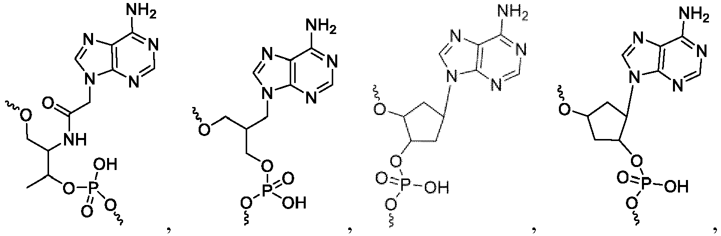 and 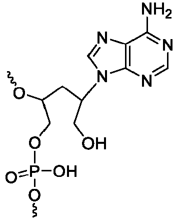 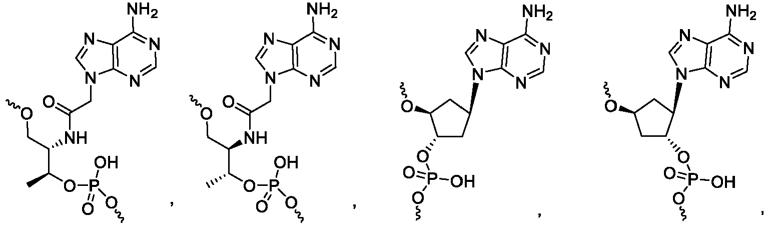 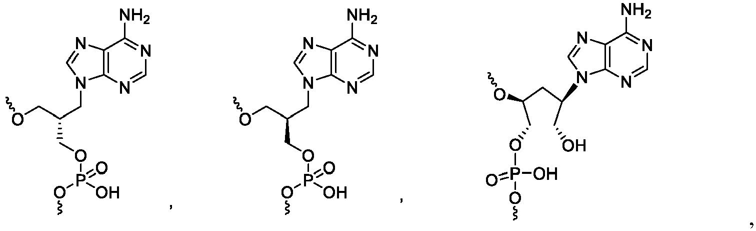 and those where adenine in the structure is replaced with guanine, cytosine, uracil or thymine.

In some embodiments, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at that position of the antisense strand when unmodified.

In some embodiments, the chemical modification represented by formula (I) is

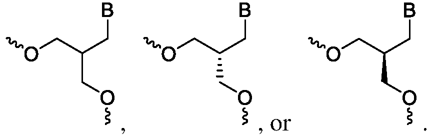

In yet another aspect, the present disclosure provides a dsRNA comprising a sense strand and an antisense strand:
In some embodiments, the antisense strand is at least partially reverse complementary to the target sequence to mediate RNA interference; in some embodiments, there are no more than 5, no more than 4, no more than 3, no more than 2, or no more than 1 mismatch between the antisense strand and the target sequence; in some embodiments, the antisense strand is fully reverse complementary to the target sequence.

In some embodiments, the sense strand is at least partially reverse complementary to the antisense strand so they form a double-stranded region; in some embodiments, there are no more than 5, no more than 4, no more than 3, no more than 2, or no more than 1 mismatch between the sense strand and the antisense strand; in some embodiments, the sense strand is fully reverse complementary to the antisense strand.

In some embodiments, the sense strand and the antisense strand each independently have 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25, 19 to 24, or 19 to 23 nucleotides (e.g., 19, 20, 21, 22, or 23 nucleotides).

In some embodiments, the sense strand and the antisense strand are identical or different in length; the sense strand is 19-23 nucleotides in length, and the antisense strand is 19-26 nucleotides in length. In some embodiments, the ratio of the length of the sense strand to the length of the antisense strand may be 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26. In some embodiments, the ratio of the length of the sense strand to the length of the antisense strand is 19/21, 21/23, or 23/25. In some embodiments, the ratio of the length of the sense strand to the length of the antisense strand is 19/21.

In some embodiments, the dsRNA comprises one or two blunt ends.

In some embodiments, the dsRNA comprises an overhang having 1 to 4 unpaired nucleotides, e.g., 1, 2, 3, or 4 unpaired nucleotides.

In some embodiments, the dsRNA comprises an overhang at the 3' end of the antisense strand.

In some embodiments, the nucleotide comprising the chemical modification represented by formula (I) or formula (I'), the tautomer thereof, or the pharmaceutically acceptable salt thereof is located at position 5, 6, or 7 of the 5' region of the antisense strand.

In some embodiments, the nucleotide comprising the chemical modification represented by formula (I) or formula (I'), the tautomer thereof, or the pharmaceutically acceptable salt thereof is located at position 7 of the 5' region of the antisense strand.

In some embodiments, when the chemical modification represented by formula (I) or formula (I'), the tautomer thereof, or the pharmaceutically acceptable salt thereof is at position 5 of the 5' region, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, when the chemical modification represented by formula (I) or formula (I'), the tautomer thereof, or the pharmaceutically acceptable salt thereof is at position 6 of the 5' region, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, when the chemical modification represented by formula (I) or formula (I'), the tautomer thereof, or the pharmaceutically acceptable salt thereof is at position 7 of the 5' region, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is the same as the base of the nucleotide at position 5 of the 5' region of the antisense strand when unmodified.

In some embodiments, B is the same as the base of the nucleotide at position 6 of the 5' region of the antisense strand when unmodified.

In some embodiments, B is the same as the base of the nucleotide at position 7 of the 5' region of the antisense strand when unmodified.

In some embodiments, the nucleotide at position 5 of the sense strand is a 2'-methoxy-modified nucleotide.

In some embodiments, the nucleotide at position 7 of the antisense strand is a 2'-methoxy-modified nucleotide.

In some embodiments, the sense strand and the antisense strand comprise or are nucleotide sequences represented by the following formulas:
the sense strand: 5'-NₐNₐNₐNₐXNₐN_{b}N_{b}N_{b}NₐNₐNₐNₐNₐNₐNₐNₐNₐNₐ-3';
the antisense strand: 5'-Nₐ'N_{b}'Nₐ'X'Nₐ'X'W'X'X'X'Nₐ'X'Nₐ'N_{b}'Nₐ'X'Nₐ'X'Nₐ'Nₐ'Nₐ'-3';
wherein each X is independently Nₐ or N_{b}, and each X' is independently Nₐ' or N_{b}'; Nₐ and Nₐ' are 2'-methoxy-modified nucleotides, and N_{b} and N_{b}' are 2'-fluoro-modified nucleotides;
W' represents a 2'-methoxy-modified nucleotide or a nucleotide comprising a chemical modification represented by formula (I) or formula (I'), a tautomer thereof, or a pharmaceutically acceptable salt thereof.

In some embodiments, W' represents a 2'-methoxy-modified nucleotide.

In some embodiments, W' represents a nucleotide comprising a chemical modification represented by formula (I) or formula (I'), a tautomer thereof, or a pharmaceutically acceptable salt thereof.

In some embodiments, W' has the following structure:

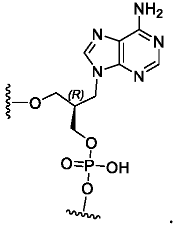

In some embodiments, the sense strand and the antisense strand comprise or are nucleotide sequences represented by the following formulas:
the sense strand: 5'-NₐNₐNₐNₐNₐNₐN_{b}N_{b}N_{b}NₐNₐNₐNₐNₐNₐNₐNₐNₐNₐ-3';
the antisense strand: 5'-Nₐ'N_{b}'Nₐ'X'Nₐ'X'W'X'X'X'Nₐ'X'Nₐ'N_{b}'Nₐ'X'Nₐ'X'Nₐ'Nₐ'Nₐ'-3';
wherein each X' is independently Nₐ' or N_{b}'; Nₐ and Nₐ' are 2'-methoxy-modified nucleotides, and N_{b} and N_{b}' are 2'-fluoro-modified nucleotides;
W' represents a 2'-methoxy-modified nucleotide or a nucleotide comprising a chemical modification represented by formula (I) or formula (I'), a tautomer thereof, or a pharmaceutically acceptable salt thereof.

In some embodiments, W' represents a 2'-methoxy-modified nucleotide.

In some embodiments, W' represents a nucleotide comprising a chemical modification represented by formula (I) or formula (I'), a tautomer thereof, or a pharmaceutically acceptable salt thereof.

In some embodiments, W' has the following structure:

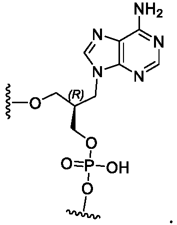

In some embodiments, the sense strand comprises or is a nucleotide sequence represented by the following formula:
5'-NₐNₐNₐNₐXNₐN_{b}N_{b}N_{b}NₐNₐNₐNₐNₐNₐNₐNₐNₐNₐ-3';
wherein X is Nₐ or N_{b}; Nₐ is a 2'-methoxy-modified nucleotide, and N_{b} is a 2'-fluoro-modified nucleotide.

In some embodiments, the sense strand comprises or is a nucleotide sequence represented by the following formula:
5'-NₐNₐNₐNₐNₐNₐN_{b}N_{b}N_{b}NₐNₐNₐNₐNₐNₐNₐNₐNₐNₐ-3';
wherein Nₐ is a 2'-methoxy-modified nucleotide, and N_{b} is a 2'-fluoro-modified nucleotide.

In some embodiments, the antisense strand comprises or is a nucleotide sequence represented by the following formula:
5'-Nₐ'N_{b}'Nₐ'X'Nₐ'X'W'X'X'X'Nₐ'X'Nₐ'N_{b}'Nₐ'X'Nₐ'X'Nₐ'Nₐ'Nₐ'-3';
wherein each X' is independently Nₐ' or N_{b}'; Nₐ' is a 2'-methoxy-modified nucleotide, and N_{b}' is a 2'-fluoro-modified nucleotide;
W' represents a 2'-methoxy-modified nucleotide or a nucleotide comprising a chemical modification represented by formula (I) or formula (I'), a tautomer thereof, or a pharmaceutically acceptable salt thereof.

In some embodiments, W' represents a 2'-methoxy-modified nucleotide.

In some embodiments, W' represents a nucleotide comprising a chemical modification represented by formula (I) or formula (I'), a tautomer thereof, or a pharmaceutically acceptable salt thereof.

In some embodiments, W' has the following structure:

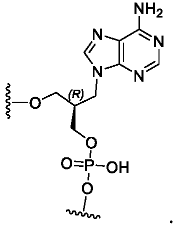

In some embodiments, the chemical modification represented by formula (I) is selected from the group consisting of: 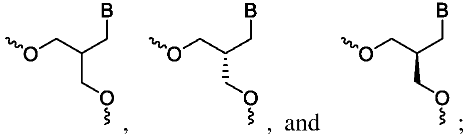 wherein: B is selected from the group consisting of guanine, adenine, cytosine, and uracil; in some specific embodiments, B is the same as the base of the nucleotide at position 7 of the 5' region of the antisense strand when unmodified.

In some embodiments, the chemical modification represented by formula (I) is selected from the group consisting of: 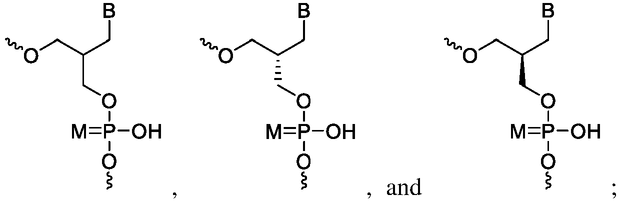 wherein: M is O or S; wherein: B is selected from the group consisting of guanine, adenine, cytosine, and uracil; in some specific embodiments, B is the same as the base of the nucleotide at position 7 of the 5' region of the antisense strand when unmodified.

In some specific embodiments, M is S. In some specific embodiments, M is O.

In some embodiments, the antisense strand comprises or is a nucleotide sequence represented by the following formula:
5'-Nₐ'N_{b}'Nₐ'N_{b}'Nₐ'N_{b}'W'Nₐ'Nₐ'N_{b}'Nₐ'N_{b}'Nₐ'N_{b}'Nₐ'N_{b}'Nₐ'Nₐ'Nₐ'Nₐ'Nₐ'-3';
5'-Nₐ'N_{b}'Nₐ'N_{b}'Nₐ'N_{b}'W'Nₐ'Nₐ'N_{b}'Nₐ'Nₐ'Nₐ'N_{b}'Nₐ'N_{b}'Nₐ'N_{b}'Nₐ'Nₐ'Nₐ'-3';
5'-Nₐ'N_{b}'Nₐ'N_{b}'Nₐ'N_{b}'W'Nₐ'Nₐ'Nₐ'Nₐ'N_{b}'Nₐ'N_{b}'Nₐ'N_{b}'Nₐ'N_{b}'Nₐ'Nₐ'Nₐ'-3';
5'-Nₐ'N_{b}'Nₐ'Nₐ'Nₐ'N_{b}'W'Nₐ'Nₐ'N_{b}'Nₐ'N_{b}'Nₐ'N_{b}'Nₐ'N_{b}'Nₐ'N_{b}'Nₐ'Nₐ'Nₐ'-3';
5'-Nₐ'N_{b}'Nₐ'N_{b}'Nₐ'N_{b}'W'Nₐ'Nₐ'Nₐ'Nₐ'Nₐ'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'N_{b}'W'Nₐ'Nₐ'N_{b}'Nₐ'Nₐ'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'N_{b}'W'Nₐ'Nₐ'Nₐ'Nₐ'N_{b}'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'Nₐ'W'N_{b}'N_{b}'Nₐ'Nₐ'Nₐ'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'N_{b}'W'Nₐ'Nₐ'Nₐ'Nₐ'Nₐ'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'N_{b}'W'Nₐ'Nₐ'Nₐ'Nₐ'Nₐ'Nₐ'N_{b}'Nₐ'Nₐ'Nₐ'Nₐ'Nₐ'Nₐ'Nₐ'-3';
5'-Nₐ'N_{b}'Nₐ'Nₐ'Nₐ'Nₐ'W'Nₐ'Nₐ'Nₐ'Nₐ'Nₐ'Nₐ'N_{b}'Nₐ'N_{b}'Nₐ'Nₐ'Nₐ'Nₐ'Nₐ'-3'; or,
5'-Nₐ'N_{b}'Nₐ'Nₐ'Nₐ'Nₐ'W'Nₐ'Nₐ'Nₐ'Nₐ'Nₐ'Nₐ'N_{b}'Nₐ'Nₐ'Nₐ'Nₐ'Nₐ'Nₐ'Nₐ'-3';
wherein Nₐ' is a 2'-methoxy-modified nucleotide, and N_{b}' is a 2'-fluoro-modified nucleotide;
W' represents a 2'-methoxy-modified nucleotide or a nucleotide comprising a chemical modification represented by formula (I), a tautomer thereof, or a pharmaceutically acceptable salt thereof.

In some embodiments, W' represents a 2'-methoxy-modified nucleotide.

In some embodiments, W' represents a nucleotide comprising a chemical modification represented by formula (I), a tautomer thereof, or a pharmaceutically acceptable salt thereof.

In some embodiments, the chemical modification represented by formula (I) is selected from the group consisting of: 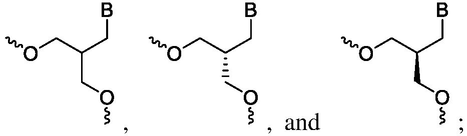 wherein: B is selected from the group consisting of guanine, adenine, cytosine, and uracil; in some specific embodiments, B is the same as the base of the nucleotide at position 7 of the 5' region of the antisense strand when unmodified.

In some embodiments, the chemical modification represented by formula (I) is selected from the group consisting of: 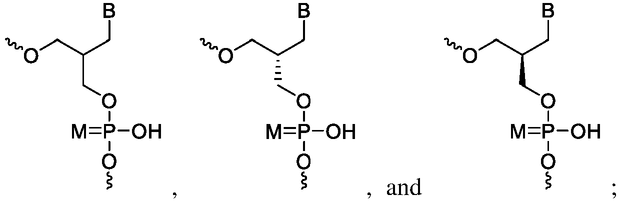 wherein: M is O or S; wherein: B is selected from the group consisting of guanine, adenine, cytosine, and uracil; in some specific embodiments, B is the same as the base of the nucleotide at position 7 of the 5' region of the antisense strand when unmodified.

In some embodiments, W' has the following structure:

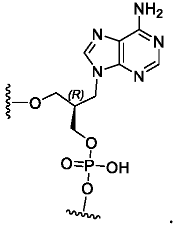

In some specific embodiments, M is S. In some specific embodiments, M is O.

In some embodiments, at least one phosphoester group in the sense strand and/or the antisense strand is a phosphoester group having a modification group that makes the dsRNA have increased stability in a biological sample or environment; in some embodiments, the phosphoester group having a modification group is a phosphorothioate group. In some embodiments, the phosphoester group having a modification group is a phosphorothioate diester group.

In some embodiments, the phosphorothioate diester group is present at at least one of the following positions:
between the first and second nucleotides of the 5' end of the sense strand;
between the second and third nucleotides of the 5' end of the sense strand;
between the first and second nucleotides of the 5' end of the antisense strand;
between the second and third nucleotides of the 5' end of the antisense strand;
between the first and second nucleotides of the 3' end of the antisense strand; and
between the second and third nucleotides of the 3' end of the antisense strand.

In some embodiments, the sense strand and/or the antisense strand comprise a plurality of phosphorothioate diester groups, and the phosphorothioate diester groups are present:
between the first and second nucleotides of the 5' end of the sense strand; and,
between the second and third nucleotides of the 5' end of the sense strand; and,
between the first and second nucleotides of the 5' end of the antisense strand; and,
between the second and third nucleotides of the 5' end of the antisense strand; and,
between the first and second nucleotides of the 3' end of the antisense strand; and,
between the second and third nucleotides of the 3' end of the antisense strand.

In some embodiments, the sense strand comprises or is a nucleotide sequence represented by the following formula:
5'-NmsNmsNmNmNfNmNfNfNfNmNmNmNmNmNmNmNmNmNm-3', or,
5'-NmsNmsNmNmNmNmNfNfNfNmNmNmNmNmNmNmNmNmNm-3',
wherein Nm represents any 2'-methoxy-modified nucleotide, e.g., a 2'-methoxy-modified C, G, U, or A; Nf represents any 2'-fluoro-modified nucleotide, e.g., a 2'-fluoro-modified C, G, U, or A;
the lowercase letter s indicates that the two nucleotides adjacent to the letter s are linked by a phosphorothioate diester group; the lowercase letter s, when being the first at the 3' end, indicates that an end of the nucleotide adjacent to the left of the letter s is a phosphorothioate diester group.

In some embodiments, the sense strand comprises or is a nucleotide sequence represented by the following formula:
5'-NmsNmsNmNmNmNmNfNfNfNmNmNmNmNmNmNmNmNmNm-3',
wherein Nm represents any 2'-methoxy-modified nucleotide, e.g., a 2'-methoxy-modified C, G, U, or A; Nf represents any 2'-fluoro-modified nucleotide, e.g., a 2'-fluoro-modified C, G, U, or A;
the lowercase letter s indicates that the two nucleotides adjacent to the letter s are linked by a phosphorothioate diester group; the lowercase letter s, when being the first at the 3' end, indicates that an end of the nucleotide adjacent to the left of the letter s is a phosphorothioate diester group.

In some embodiments, the antisense strand comprises or is a nucleotide sequence represented by the following formula:
5'-Nm'sNf'sNm'NfNm'NfW'Nm'Nm'NfNm'NfNm'NfNm'NfNm'Nm'Nm'sNm'sNm'-3';
5'-Nm'sNf'sNm'NfNm'NfW'Nm'Nm'NfNm'Nm'Nm'NfNm'NfNm'NfNm'sNm'sNm'-3';
5'-Nm'sNf'sNm'NfNm'NfW'Nm'Nm'Nm'Nm'NfNm'NfNm'NfNm'NfNm'sNm'sNm'-3';
5'-Nm'sNf'sNm'Nm'Nm'NfW'Nm'Nm'NfNm'NfNm'NfNm'NfNm'NfNm'sNm'sNm'-3';
5'-Nm'sNf'sNm'NfNm'NfW'Nm'Nm'Nm'Nm'Nm'Nm'NfNm'NfNm'Nm'Nm'sNm'sNm'-3';
5'-Nm'sNf'sNm'Nm'Nm'NfW'Nm'Nm'NfNm'Nm'Nm'NfNm'NfNm'Nm'Nm'sNm'sNm'-3';
5'-Nm'sNf'sNm'Nm'Nm'NfW'Nm'Nm'Nm'Nm'NfNm'NfNm'NfNm'Nm'Nm'sNm'sNm'-3';
5'-Nm'sNf'sNm'Nm'Nm'Nm'W'NfNfNm'Nm'Nm'Nm'NfNm'NfNm'Nm'Nm'sNm'sNm'-3';
5'-Nm'sNf'sNm'Nm'Nm'NfW'Nm'Nm'Nm'Nm'Nm'Nm'NfNm'NfNm'Nm'Nm'sNm'sNm'-3';
5'-Nm'sNf'sNm'Nm'Nm'Nf'W'Nm'Nm'Nm'Nm'Nm'Nm'Nf'Nm'Nm'Nm'Nm'Nm'sNm'sNm'-3';
5'-Nm'sNf'sNm'Nm'Nm'Nm'W'Nm'Nm'Nm'Nm'Nm'Nm'NfNm'NfNm'Nm'Nm'sNm'sNm'-3'; or,
5'-Nm'sNf'sNm'Nm'Nm'Nm'W'Nm'Nm'Nm'Nm'Nm'Nm'NfNm'Nm'Nm'Nm'Nm'sNm'sNm'-3'; wherein Nm' represents any 2'-methoxy-modified nucleotide, e.g., a 2'-methoxy-modified C, G, U, or A; Nf represents any 2'-fluoro-modified nucleotide, e.g., 2'-fluoro-modified C, G, U, or A;
the lowercase letter s indicates that the two nucleotides adjacent to the letter s are linked by a phosphorothioate diester group, and the lowercase letter s, when being the first at the 3' end, indicates that an end of the nucleotide adjacent to the left of the letter s is a phosphorothioate diester group;
W' represents a 2'-methoxy-modified nucleotide or a nucleotide comprising a chemical modification represented by formula (I) or formula (I'), a tautomer thereof, or a pharmaceutically acceptable salt thereof.

In some embodiments, W' represents a 2'-methoxy-modified nucleotide.

In some embodiments, W' represents a nucleotide comprising a chemical modification represented by formula (I) or formula (I'), a tautomer thereof, or a pharmaceutically acceptable salt thereof.

In some embodiments, the chemical modification represented by formula (I) or formula (I') is selected from the group consisting of: 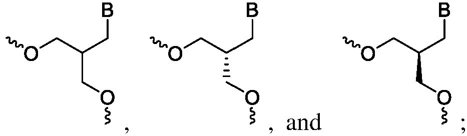 wherein: B is selected from the group consisting of guanine, adenine, cytosine, and uracil; in some embodiments, B is the same as the base of the nucleotide at position 7 of the 5' region of the antisense strand when unmodified.

In some embodiments, the chemical modification represented by formula (I) or formula (I') is selected from the group consisting of: 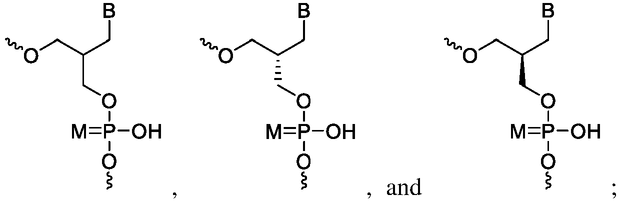 wherein: M is O or S; wherein: B is selected from the group consisting of guanine, adenine, cytosine, and uracil; in some specific embodiments, B is the same as the base of the nucleotide at position 7 of the 5' region of the antisense strand when unmodified.

In some embodiments, M is S. In some specific embodiments, M is O.

In some embodiments, the dsRNA inhibits the expression of apolipoprotein C3 (APOC3). In some embodiments, the sense strand comprises at least 15 contiguous nucleotides that differ from the nucleotide sequence of SEQ ID NO: 1 by no more than 3 nucleotides, and/or,
the antisense strand comprises at least 19 contiguous nucleotides that differ from the nucleotide sequence of SEQ ID NO: 2 by no more than 3 nucleotides;

In some embodiments, the sense strand comprises or is selected from the group consisting of the nucleotide sequence of SEQ ID NO: 1, and/or the antisense strand comprises or is selected from the group consisting of the nucleotide sequence of SEQ ID NO: 2.

In some embodiments, the sense strand and the antisense strand are any one of the following:
the sense strand comprises 5'-UAUUCUCAGUGCUCUCCUZ_{b1}-3' (SEQ ID NO: 21), and the antisense strand comprises 5'-UAGGAGAGCACUGAGAAUACU-3' (SEQ ID NO: 22);
or, the sense strand comprises 5'-GCACCGUUAAGGACAAGUZ_{b2}-3' (SEQ ID NO: 23), and the antisense strand comprises 5'-AACUUGUCCUUAACGGUGCUC-3' (SEQ ID NO: 24);
wherein Z_{b1} is A or G; Z_{b2} is C or U.

In some embodiments, the nucleotide sequence of the sense strand of the dsRNA comprises or is selected from the group consisting of SEQ ID NO: 3, and the nucleotide sequence of the antisense strand comprises or is selected from the group consisting of the nucleotide sequence of any one of SEQ ID NO: 5 to SEQ ID NO: 14;

In some embodiments, the dsRNA is any one of the following:
the sense strand comprises the nucleotide sequence of SEQ ID NO: 3, and the antisense strand comprises the nucleotide sequence of SEQ ID NO: 5;
the sense strand comprises the nucleotide sequence of SEQ ID NO: 3, and the antisense strand comprises the nucleotide sequence of SEQ ID NO: 6;
the sense strand comprises the nucleotide sequence of SEQ ID NO: 3, and the antisense strand comprises the nucleotide sequence of SEQ ID NO: 7;
the sense strand comprises the nucleotide sequence of SEQ ID NO: 3, and the antisense strand comprises the nucleotide sequence of SEQ ID NO: 8;
the sense strand comprises the nucleotide sequence of SEQ ID NO: 3, and the antisense strand comprises the nucleotide sequence of SEQ ID NO: 9;
the sense strand comprises the nucleotide sequence of SEQ ID NO: 3, and the antisense strand comprises the nucleotide sequence of SEQ ID NO: 10;
the sense strand comprises the nucleotide sequence of SEQ ID NO: 3, and the antisense strand comprises the nucleotide sequence of SEQ ID NO: 11;
the sense strand comprises the nucleotide sequence of SEQ ID NO: 3, and the antisense strand comprises the nucleotide sequence of SEQ ID NO: 12;
the sense strand comprises the nucleotide sequence of SEQ ID NO: 3, and the antisense strand comprises the nucleotide sequence of SEQ ID NO: 13;
the sense strand comprises the nucleotide sequence of SEQ ID NO: 3, and the antisense strand comprises the nucleotide sequence of SEQ ID NO: 14.

In some embodiments, the dsRNA is any one of the following:
the sense strand consists of the nucleotide sequence set forth in SEQ ID NO: 3, and the antisense strand consists of the nucleotide sequence set forth in SEQ ID NO: 5;
the sense strand consists of the nucleotide sequence set forth in SEQ ID NO: 3, and the antisense strand consists of the nucleotide sequence set forth in SEQ ID NO: 6;
the sense strand consists of the nucleotide sequence set forth in SEQ ID NO: 3, and the antisense strand consists of the nucleotide sequence set forth in SEQ ID NO: 7;
the sense strand consists of the nucleotide sequence set forth in SEQ ID NO: 3, and the antisense strand consists of the nucleotide sequence set forth in SEQ ID NO: 8;
the sense strand consists of the nucleotide sequence set forth in SEQ ID NO: 3, and the antisense strand consists of the nucleotide sequence set forth in SEQ ID NO: 9;
the sense strand consists of the nucleotide sequence set forth in SEQ ID NO: 3, and the antisense strand consists of the nucleotide sequence set forth in SEQ ID NO: 10;
the sense strand consists of the nucleotide sequence set forth in SEQ ID NO: 3, and the antisense strand consists of the nucleotide sequence set forth in SEQ ID NO: 11;
the sense strand consists of the nucleotide sequence set forth in SEQ ID NO: 3, and the antisense strand consists of the nucleotide sequence set forth in SEQ ID NO: 12;
the sense strand consists of the nucleotide sequence set forth in SEQ ID NO: 3, and the antisense strand consists of the nucleotide sequence set forth in SEQ ID NO: 13;
the sense strand consists of the nucleotide sequence set forth in SEQ ID NO: 3, and the antisense strand consists of the nucleotide sequence set forth in SEQ ID NO: 14.

In some embodiments, the dsRNA is any one of the following:
the dsRNA comprises a sense strand set forth in SEQ ID NO: 3 and an antisense strand set forth in any one of SEQ ID NO: 5 to SEQ ID NO: 14.

In some embodiments, the dsRNA is any one of the following:
the dsRNA is selected from the group consisting of a sense strand set forth in SEQ ID NO: 3 and an antisense strand set forth in any one of SEQ ID NO: 5 to SEQ ID NO: 14.

In the present disclosure, in the 5'-3' direction,
SEQ ID NO: 3 is
   UmsAmsUmUmCmUmCfAfGfUmGmCmUmCmUmCmCmUmAm;
SEQ ID NO: 5 is
   UmsAfsGmGfAmGf(-)hmpNA(A)GmCmAfCmUfGmAfGmAfAmUmAmsCmsUm;
SEQ ID NO: 6 is
   UmsAfsGmGfAmGf(-)hmpNA(A)GmCmAfCmUmGmAfGmAfAmUfAmsCmsUm;
SEQ ID NO: 7 is
   UmsAfsGmGfAmGf(-)hmpNA(A)GmCmAmCmUfGmAfGmAfAmUfAmsCmsUm;
SEQ ID NO: 8 is
   UmsAfsGmGmAmGf(-)hmpNA(A)GmCmAfCmUfGmAfGmAfAmUfAmsCmsUm;
SEQ ID NO: 9 is
   UmsAfsGmGmAmGf(-)hmpNA(A)GmCmAmCmUmGmAfGmAfAmUmAmsCmsUm;
SEQ ID NO: 10 is
   UmsAfsGmGfAmGf(-)hmpNA(A)GmCmAmCmUmGmAfGmAfAmUmAmsCmsUm;
SEQ ID NO: 11 is
   UmsAfsGmGmAmGf(-)hmpNA(A)GmCmAfCmUmGmAfGmAfAmUmAmsCmsUm;
SEQ ID NO: 12 is
   UmsAfsGmGmAmGf(-)hmpNA(A)GmCmAmCmUfGmAfGmAfAmUmAmsCmsUm;
SEQ ID NO: 13 is
   UmsAfsGmGmAmGm(-)hmpNA(A)GfCfAmCmUmGmAfGmAfAmUmAmsCmsUm;
SEQ ID NO: 14 is 
UmsAfsGmGmAmGm(-)hmpNA(A)GmCmAmCmUmGmAfGmAmAmUmAmsCmsU
m;
wherein Af = adenine 2'-F ribonucleoside; Cf = cytosine 2'-F ribonucleoside; Uf = uracil 2'-F ribonucleoside; Gf = guanine 2'-F ribonucleoside; Am = adenine 2'-OMe ribonucleoside; Cm = cytosine 2'-OMe ribonucleoside; Gm = guanine 2'-OMe ribonucleoside; Um = uracil 2'-OMe ribonucleoside;
s indicates that the two nucleotides adjacent to the letter s are linked by a phosphorothioate diester group;
(-)hmpNA(A) represents

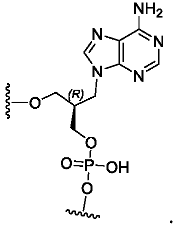

In some embodiments, the dsRNA also comprises a conjugated ligand, wherein the ligand is selected from the group consisting of a targeting ligand that targets the liver; in some embodiments, the ligand binds to asialoglycoprotein receptor (ASGPR); in some embodiments, the ligand comprises a galactose cluster or a galactose derivative cluster, wherein the galactose derivative is selected from the group consisting of N-acetylgalactosamine, N-trifluoroacetylgalactosamine, N-propionylgalactosamine, N-n-butyrylgalactosamine, and N-isobutyrylgalactosamine, and combinations thereof.

In some embodiments, the dsRNA also comprises a conjugated ligand, wherein the ligand is a compound represented by formula (II) or a pharmaceutically acceptable salt thereof, 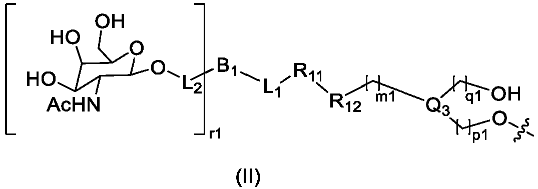
wherein L₁ is a C₁-C₃₀ alkyl chain, or comprises a C₁-C₃₀ alkyl chain interrupted by one or more oxygen, sulfur, or nitrogen atoms or C=O;
R₁₁ and R₁₂ are independently chemical bonds, NR₁₆, C=O or -OC(=O)-;
Q₃ is 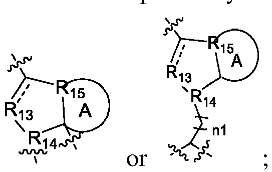 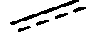
is a single or double bond, and when 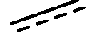 is a single bond, R₁₃ is independently CR₁₇R₁₈, NR₁₆, O, or S; when 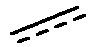 is a double bond, R₁₃ is independently CR₁₉ or N; R₁₄ is independently CR₁₉ or N;
ring A is cycloalkyl, heterocycloalkyl, aryl, or heteroaryl which is present or absent, and when ring A is present, R₁₅ is independently CR₁₉ or N; when ring A is absent, R₁₅ is independently CR₁₇R₁₈, NR₁₆, or O;
R₁₆ and R₁₉ are independently hydrogen, deuterium, alkyl, alkoxy, cycloalkyl, heterocycloalkyl, aryl, heteroaryl, SR', S(=O)R', S(=O)₂R', S(=O)₂NR'(R"), NR'(R"), C(=O)R', C(=O)OR', or C(=O)NR'(R"), wherein the alkyl, alkoxy, cycloalkyl, heterocycloalkyl, aryl, or heteroaryl is optionally substituted with one or more groups selected from the group consisting of halogen, hydroxy, oxo, nitro, cyano, C₁₋₆ alkyl, C₁₋₆ alkoxy, C₃₋₇ cycloalkyl, 3-12 membered heterocycloalkyl, 6-12 membered aryl, 5-12 membered heteroaryl, SR', S(=O)R', S(=O)₂R', S(=O)₂NR'(R"), NR'(R"), C(=O)R', C(=O)OR', and C(=O)NR'(R");
R₁₇ and R₁₈ are independently hydrogen, deuterium, alkyl, alkoxy, cycloalkyl, heterocycloalkyl, aryl, heteroaryl, SR', S(=O)R', S(=O)₂R', S(=O)₂NR'(R"), NR'(R"), C(=O)R', C(=O)OR', or C(=O)NR'(R"), wherein the alkyl, alkoxy, cycloalkyl, heterocycloalkyl, aryl, or heteroaryl is optionally substituted with one or more groups selected from the group consisting of halogen, hydroxy, oxo, nitro, cyano, C₁₋₆ alkyl, C₁₋₆ alkoxy, C₃₋₇ cycloalkyl, 3-12 membered heterocycloalkyl, 6-12 membered aryl, 5-12 membered heteroaryl, SR', S(=O)R', S(=O)₂R', S(=O)₂NR'(R"), NR'(R"), C(=O)R', C(=O)OR', and C(=O)NR'(R");
R' and R" are independently hydrogen, deuterium, hydroxy, alkyl, alkoxy, cycloalkyl, heterocycloalkyl, aryl, or heteroaryl, wherein the alkyl, alkoxy, cycloalkyl, heterocycloalkyl, aryl, or heteroaryl is optionally substituted with one or more substituents selected from the group consisting of halogen, hydroxy, oxo, nitro, and cyano;
m1, n1, p1, and q1 are independently 0, 1, 2, 3, or 4;
B1 is 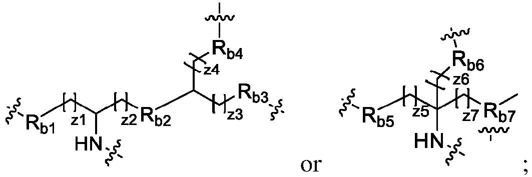
R_{b1}, R_{b2}, R_{b3}, R_{b4}, R_{b5}, R_{b6}, and R_{b7} are independently -C(=O)-, -NHC(=O)-, -C(=O)O-, - C(=O)-(CH₂)_{z8}-O-, or -NHC(=O)-(CH₂)_{z9}-O-;
z1, z2, z3, z4, z5, z6, z7, z8, and z9 are independently integers of 0-10;
L₂ is a C₁-C₃₀ alkyl chain, or comprises a C₁-C₃₀ alkyl chain interrupted by one or more oxygen, sulfur, or nitrogen atoms or C=O;
r1 is an integer of 1-10.

In some embodiments, L₁ is a C₁-C₃₀ alkyl chain, or comprises a C₁-C₃₀ alkyl chain interrupted by one or more oxygen, sulfur, or nitrogen atoms or C=O;
R₁₁ and R₁₂ are independently chemical bonds, NR₁₆, or C=O;
R₁₆ is hydrogen or C₁₋₆ alkyl;
Q₃ is 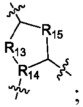
R₁₃ is CR₁₇R₁₈, NR₁₆, O, or S;
R₁₄ is CR₁₉;
R₁₅ is independently CR₁₇R₁₈, NR₁₆, or O;
R₁₇ to R₁₉ are independently hydrogen, deuterium, or alkyl;
m1, p1, and q1 are independently 0, 1, 2, 3, or 4;
B1 is 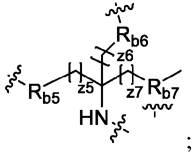
R_{b5}, R_{b6}, and R_{b7} are independently -C(=O)-, -NHC(=O)-, -C(=O)O-, -C(=O)-(CH₂)_{z8}-O-, or -NHC(=O)-(CH₂)_{z9}-O-;
z5, z6, z7, z8, and z9 are independently integers of 0-10;
L₂ is a C₁-C₃₀ alkyl chain, or comprises a C₁-C₃₀ alkyl chain interrupted by one or more oxygen, sulfur, or nitrogen atoms or C=O;
r1 is an integer of 1-10.

In some embodiments, L₁ is -(CH₂)ⱼ₁₁-C(=O)-(CH₂)ⱼ₁₂-;
R₁₁ and R₁₂ are independently chemical bonds, NR₁₆, or C=O;
R₁₆ is hydrogen or C₁₋₆ alkyl;
Q₃ is 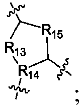
R₁₃ is CR₁₇R₁₈ or O;
R₁₄ is CR₁₉;
R₁₅ is independently CR₁₇R₁₈ or O;
R₁₇ to R₁₉ are independently hydrogen or alkyl;
m1, p1, and q1 are independently 0 or 1;
B1 is 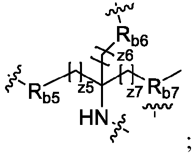
R_{b5}, R_{b6}, and R_{b7} are independently -C(=O)-(CH₂)_{z8}-O- or -NHC(=O)-(CH₂)_{z9}-O-;
z8 and z9 are independently integers of 0-10;
L₂ is -(CH₂)ⱼ₁₅-(OCH₂CH₂)₁₋₄-(CH₂)ⱼ₁₆- or 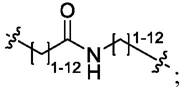
j 15 and j 16 are independently integers of 0-4;
r1 is 3, 4, 5, or 6.

In some embodiments, L₁ may be L₃ or L₃-R₁₁₀-R₁₁₁-L₃, wherein L₃ is independently a C₁-C₁₂ alkyl chain, -(CH₂)ⱼ₁₁-C(=O)-(CH₂)ⱼ₁₂-, or -(CH₂)ⱼ₁₃-(CH₂CH₂O)₁₋₄-(CH₂)ⱼ₁₄-; R₁₁₀ and R₁₁₁ are independently chemical bonds, -NR₁₁₂-, -C(=O)-, or -OC(=O)-; R₁₁₂ is hydrogen or C₁-C₁₂ alkyl; j11, j12, j13, and j14 are independently integers of 0-10. In some embodiments, j11, j12, j13, and j14 are independently integers of 0-2 or 4-10. In some embodiments, j11, j12, j13, and j14 are independently 0, 1, 2, 6, 7, 8, 9, or 10.

In some embodiments, L₁ may be -(CH₂)ⱼ₁₁-C(=O)-(CH₂)ⱼ₁₂-, wherein j11 and j12 are as defined in any one of the preceding embodiments.

In some embodiments, L₁ may be 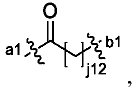 and j 12 is as defined in any one of the preceding embodiments, wherein the a1 end is linked to B₁, and the b1 end is linked to R₁₁.

In some embodiments, L₁ may be 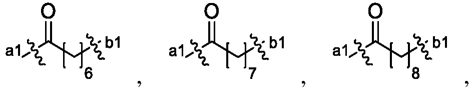 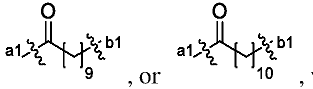 wherein the a1 end is linked to B₁, and the b1 end is linked to R₁₁.

In some embodiments, R₁₁ may be a chemical bond and R₁₂ may be C=O.

In some embodiments, R₁₁ may be a chemical bond and R₁₂ may be NR₁₆, wherein R₁₆ is as defined in any one of the preceding embodiments.

In some embodiments, R₁₁ may be a chemical bond and R₁₂ may be -OC(=O)-.

In some embodiments, R₁₁ may be NR₁₆ and R₁₂ may be C=O, wherein R₁₆ is as defined in any one of the preceding embodiments.

In some embodiments, R₁ may be NR₁₆ and R₁₂ may be -OC(=O)-, wherein R₁₆ is as defined in any one of the preceding embodiments.

In some embodiments, R₁₂ may be NR₁₆ and R₁₁ may be C=O, wherein R₁₆ is as defined in any one of the preceding embodiments.

In some embodiments, R₁₂ may be NR₁₆ and R₁₁ may be -OC(=O)-, wherein R₁₆ is as defined in any one of the preceding embodiments.

In some embodiments, R₁₁ may be NH and R₁₂ may be C=O.

In some embodiments, R₁₂ may be NH and R₁₁ may be C=O.

In some embodiments, R₁₆ may be hydrogen or C₁₋₆ alkyl.

In some embodiments, R₁₆ may be hydrogen, methyl, ethyl, propyl, or isopropyl.

In some embodiments, R₁₆ may be hydrogen.

In some embodiments, R₁₇ and R₁₈ may be hydrogen.

In some embodiments, R₁₉ may be hydrogen.

In some embodiments, when ring A is present, ring A may be C₆₋₁₂ aryl.

In some embodiments, ring A may be phenyl.

In some embodiments, m1 may be 0 or 1.

In some embodiments, m1 may be 3.

In some embodiments, n1 may be 0 or 1.

In some embodiments, p1 and q1 are independently 0 or 1.

In some embodiments, p1 = 1 and q1 = 1.

In some embodiments, p1 = 1 and q1 = 0.

In some embodiments, p1 = 0 and q1 = 1.

In some embodiments, p1 = 0 and q1 = 0.

In some embodiments, z1, z2, z3, z4, z5, z6, z7, z8, and z9 may be independently integers of 0-4. In some embodiments, z1, z2, z3, z4, z5, z6, z7, z8, and z9 may be independently integers of 0, 1, or 2.

In some embodiments, B₁ may be 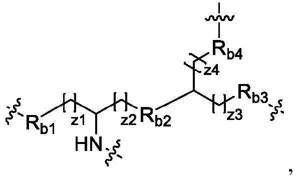 wherein R_{b1}, R_{b2}, R_{b3}, and R_{b4} are independently -C(=O)- or -NHC(=O)-; the N atom is linked to Li; z1, z2, z3, and z4 are as defined in any one of the preceding embodiments.

In some embodiments, B₁ may be 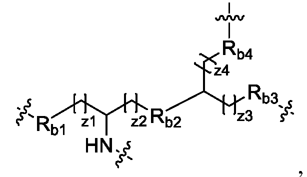 wherein R_{b1}, R_{b2}, R_{b3}, and R_{b4} are independently -C(=O)- or -NHC(=O)-; the N atom is linked to L₁; R_{b1}, R_{b3}, and R_{b4} are identical; z1, z2, z3, and z4 are as defined in any one of the preceding embodiments.

In some embodiments, B₁ may be

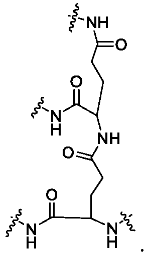

In some embodiments, B₁ may be

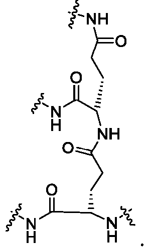

In some embodiments, B₁ may be 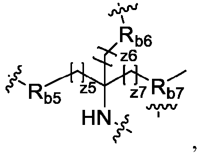 wherein R_{b5}, R_{b6}, and R_{b7} are independently -C(=O)-(CH₂)_{z8}-O- or -NHC(=O)-(CH₂)_{z9}-O-; the N atom is linked to Li; z5, z6, z7, z8, and z9 are as defined in any one of the preceding embodiments.

In some embodiments, B₁ may be 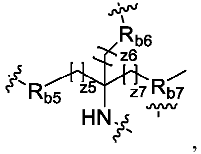 wherein R_{b5}, R_{b6}, and R_{b7} are independently -C(=O)-(CH₂)_{z8}-O- or -NHC(=O)-(CH₂)_{z9}-O-; the N atom is linked to Li; R_{b5}, R_{b6}, and R_{b7} are identical; z5, z6, z7, z8, and z9 are as defined in any one of the preceding embodiments.

In some embodiments, B₁ may be

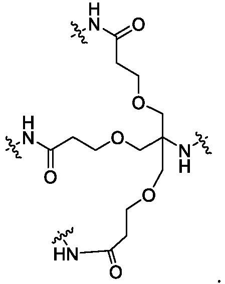

In some embodiments, L₂ may be L₄ or L₄-R₁₃-R₁₄-L₄, wherein L₄ is independently a C₁-C₁₂ alkyl chain or -(CH₂)ⱼ₁₅-(OCH₂CH₂)₁₋₄-(CH₂)ⱼ₁₆-; R₁₃ and R₁₄ are independently chemical bonds, -NR₁₁₅-, -C(=O)-, or -OC(=O)-; R₁₁₅ is independently hydrogen or C₁-C₁₂ alkyl; j 15 and j 16 are independently integers of 0-10. In some embodiments, j 15 and j16 are independently integers of 0-6. In some embodiments, j15 and j16 are independently 0, 1, 2, 3, or 4.

In some embodiments, L₂ may be -(CH₂)ⱼ₁₅-(OCH₂CH₂)₁₋₄-(CH₂)ⱼ₁₆-, wherein j15 and j16 are as defined in any one of the preceding embodiments.

In some embodiments, L₂ may be 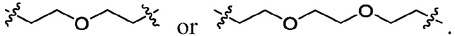 In some embodiments, L₂ may be 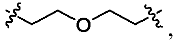 wherein the left side is linked to the O atom, and the right side is linked to B₁.

In some embodiments, L₂ may be a C₁-C₁₂ alkyl chain.

In some embodiments, L₂ may be

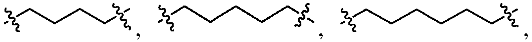

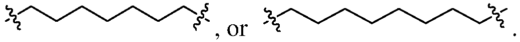

In some embodiments, L₂ may be 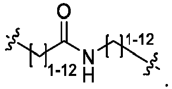 In some embodiments, L₂ may be 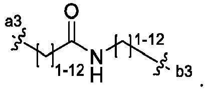 In some embodiments, L₂ may be 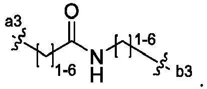 In some embodiments, L₂ may be 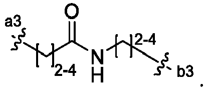 The a3 end is linked to the O atom, and the b3 end is linked to B₁.

In some embodiments, L₂ may be 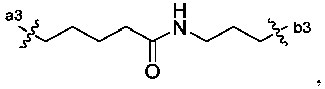 wherein the a3 end is linked to the O atom, and the b3 end is linked to B₁.

In some embodiments, r1 may be 3, 4, 5, or 6. In some embodiments, r1 may be 3.

In some embodiments, Q₃ may be 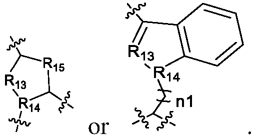 In some embodiments, Q₃ may be 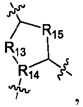 wherein R₁₃, R₁₄, R₁₅, and n1 are as defined in any one of the preceding embodiments.

In some embodiments, 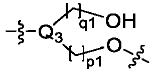 may be 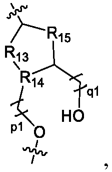 wherein R₁₃, R₁₄, R₁₅, p1, and q1 are as defined in any one of the preceding embodiments.

In some embodiments, 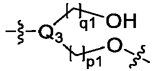 may be 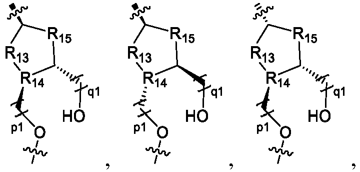 or 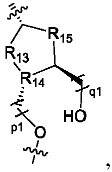 wherein R₁₃, R₁₄, R₁₅, p1, and q1 are as defined in any one of the preceding embodiments.

In some embodiments, 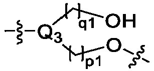 may be

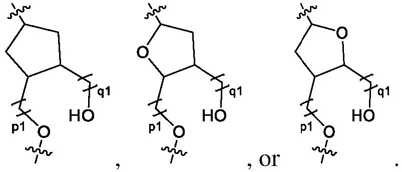

In some embodiments, 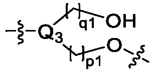 may be 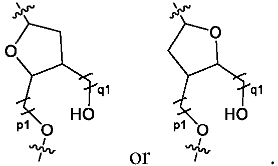 In some embodiments, 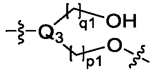 may be 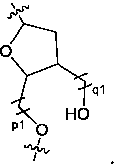 p1 and q1 are as defined in any one of the preceding embodiments.

In some embodiments, 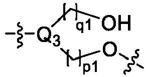 may be 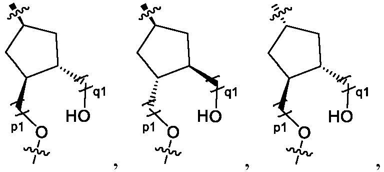 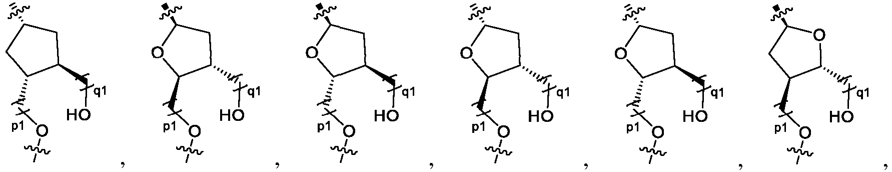 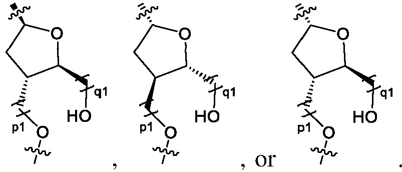 In some embodiments, 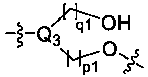 may be 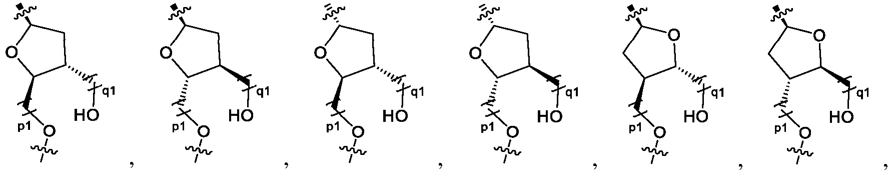 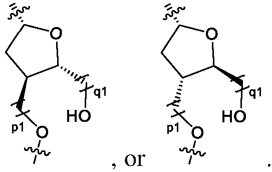 In some embodiments, 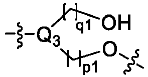 may be 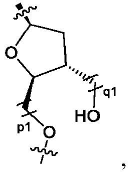 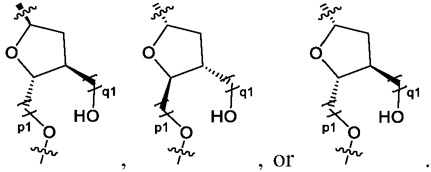 p1 and q1 are as defined in any one of the preceding embodiments.

In some embodiments, 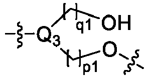 may be 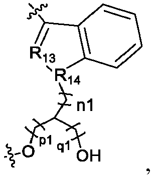 wherein R₁₃, R₁₄, n1, p1, and q1 are as defined in any one of the preceding embodiments.

In some embodiments, 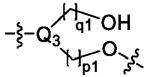 may be 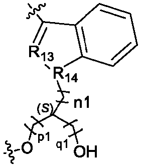 or 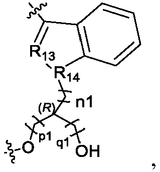 wherein R₁₃, R₁₄, n1, p1, and q1 are as defined in any one of the preceding embodiments.

In some embodiments, 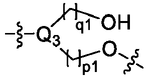 may be 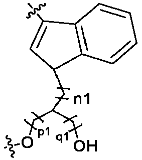 or 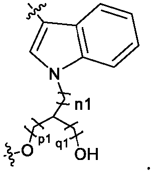 In some embodiments, 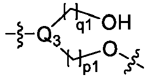 may be 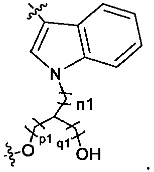 n1, p1, and q1 are as defined in any one of the preceding embodiments.

In some embodiments, 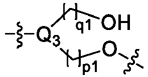 may be 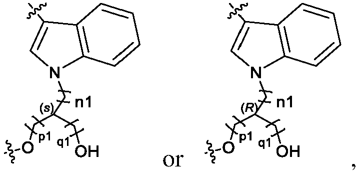 wherein n1, p1, and q1 are as defined in any one of the preceding embodiments.

In some embodiments, the ligand may be any one of the following structures or a pharmaceutically acceptable salt thereof: 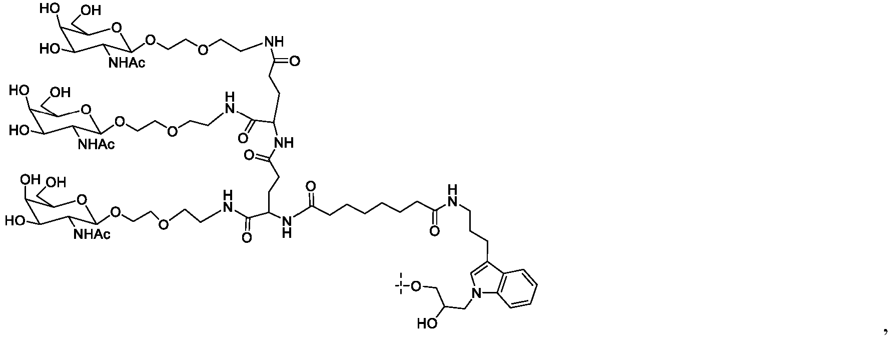 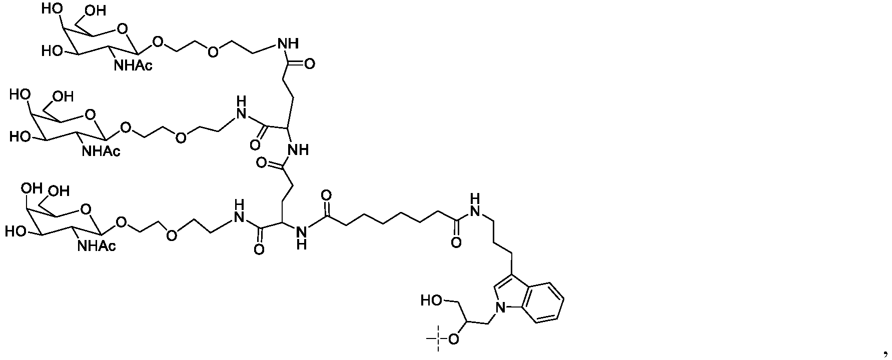 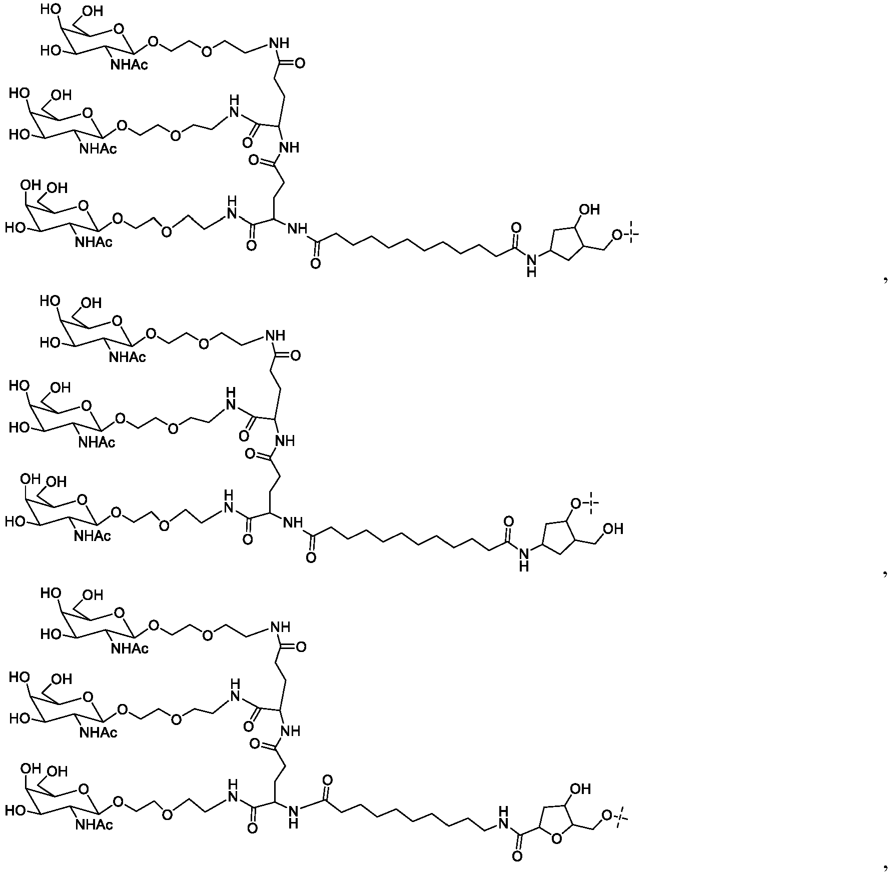 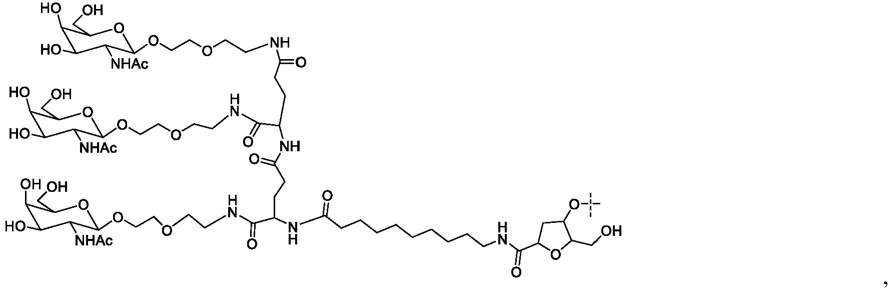 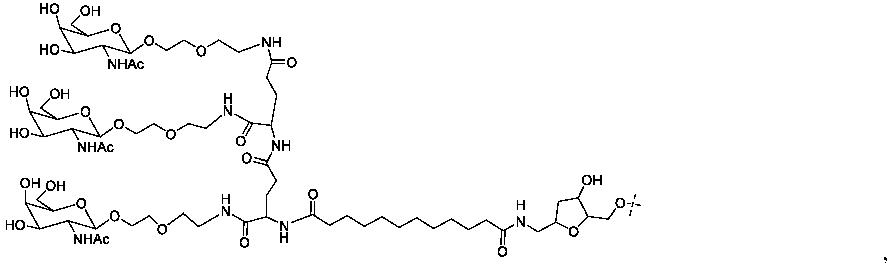 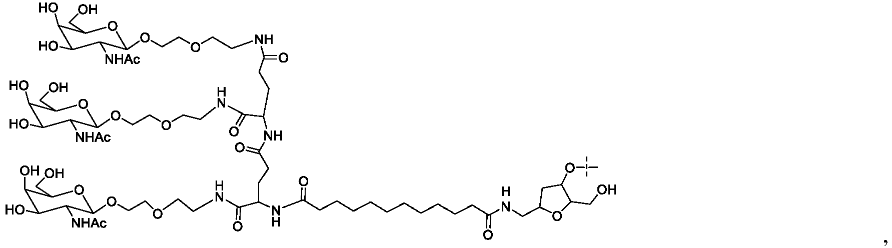 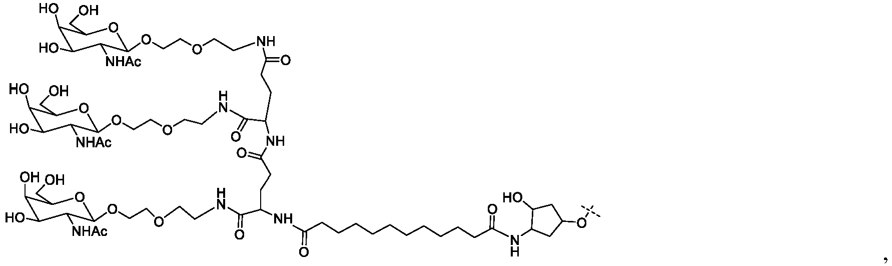 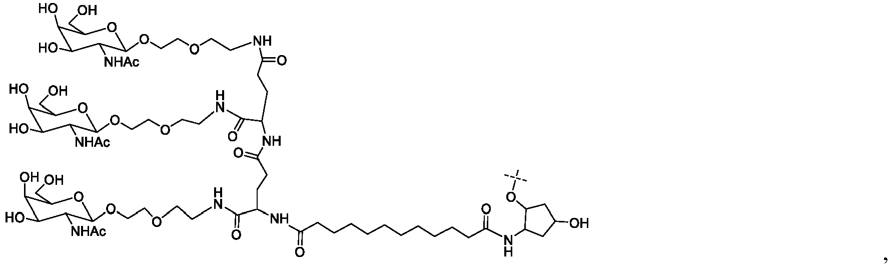 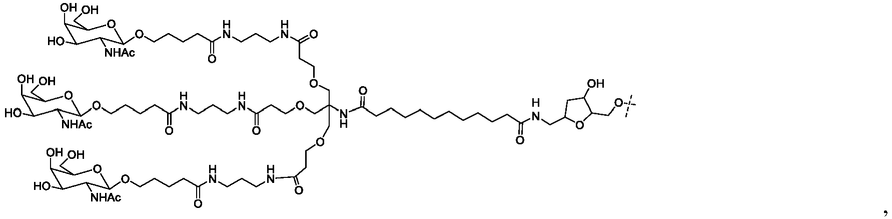 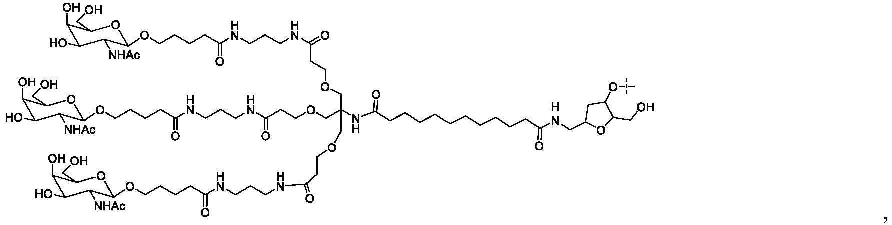 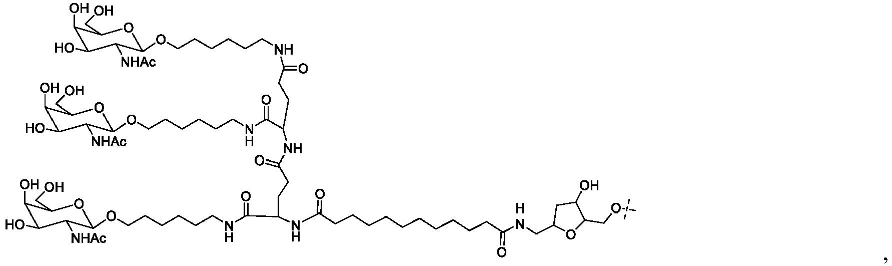 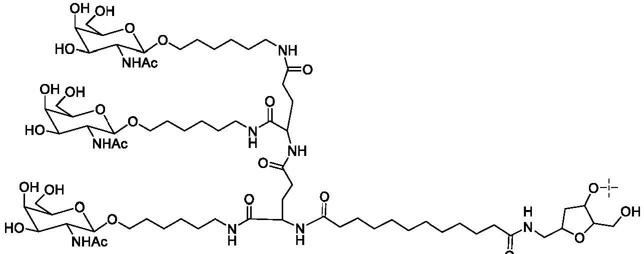 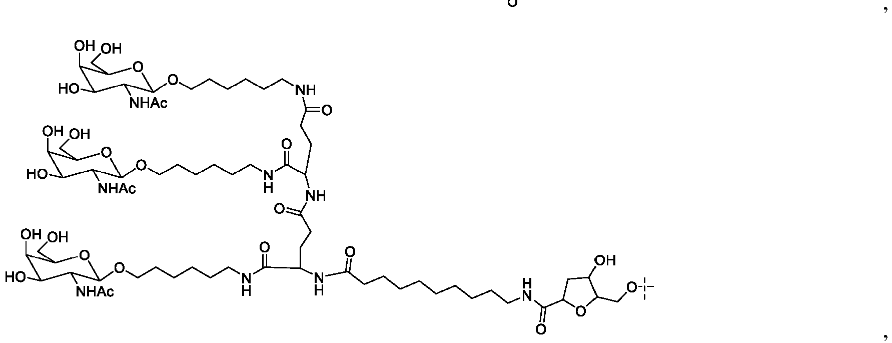 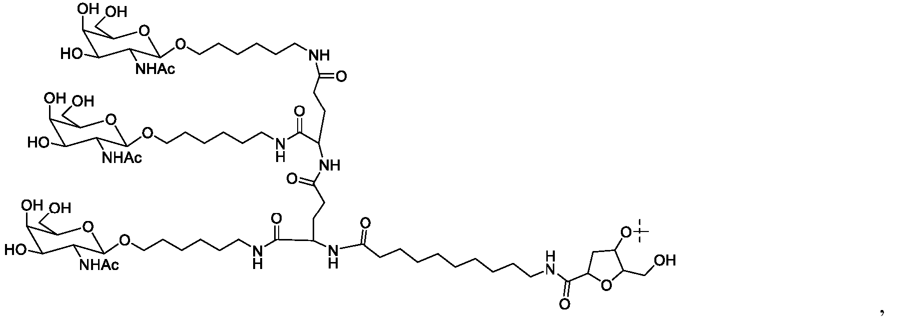 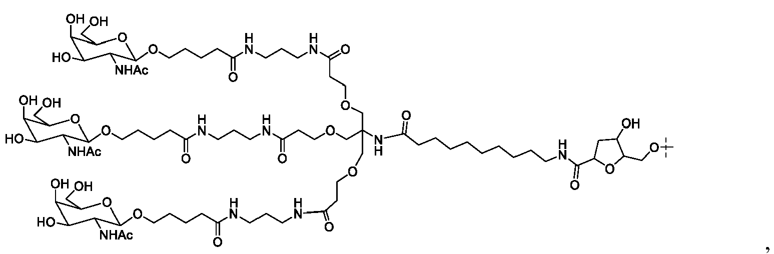 or

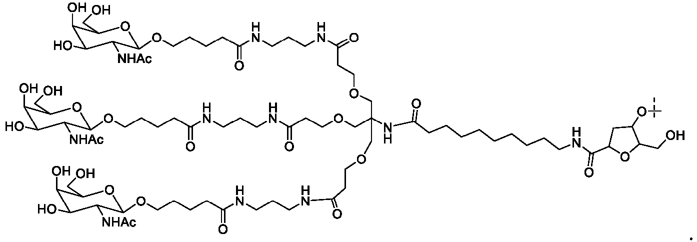

In some embodiments, the ligand may be any one of the following structures or a pharmaceutically acceptable salt thereof: 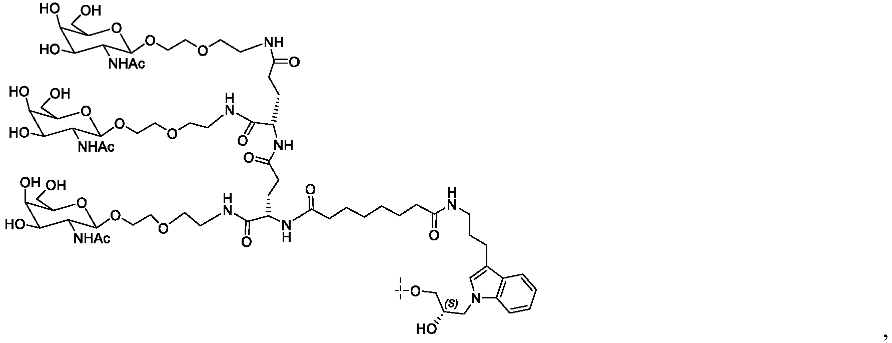 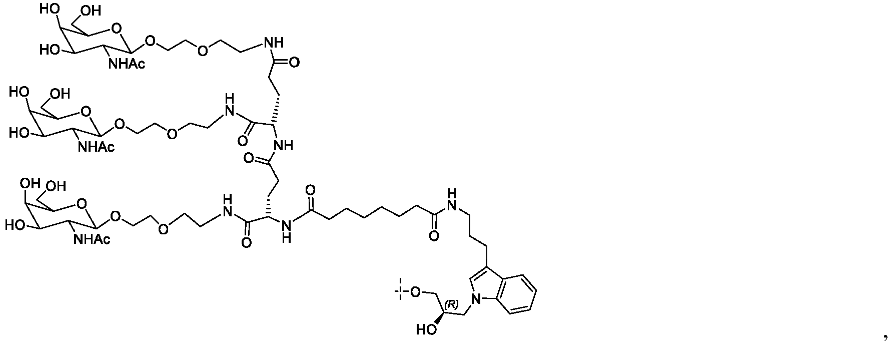 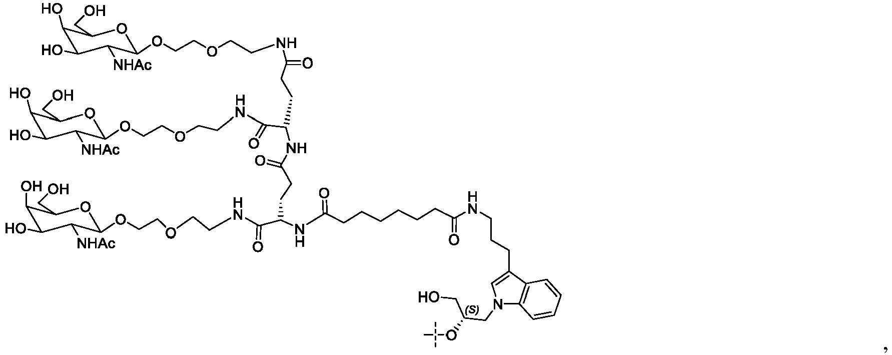 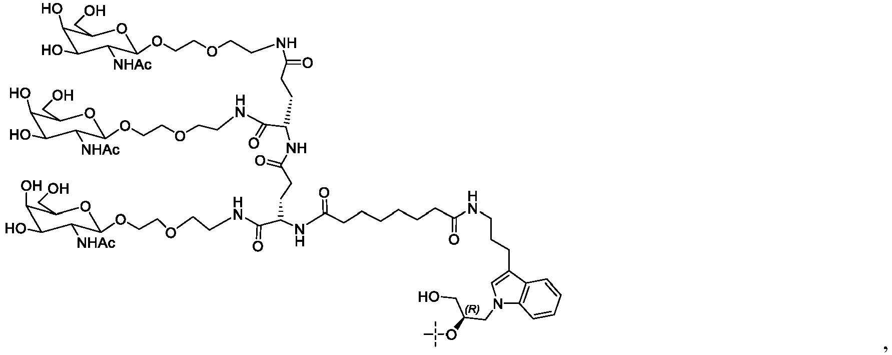 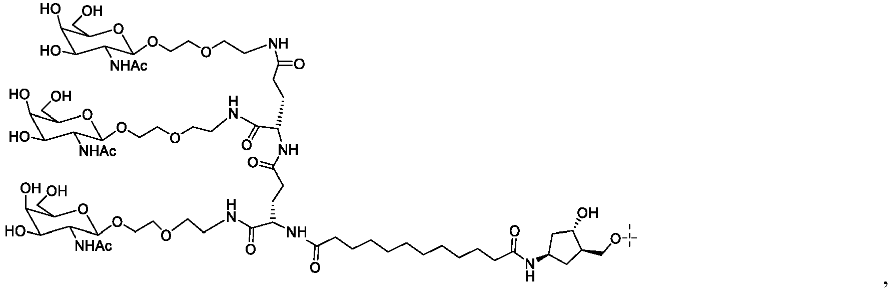 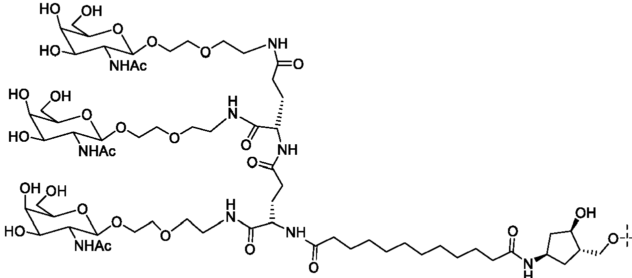 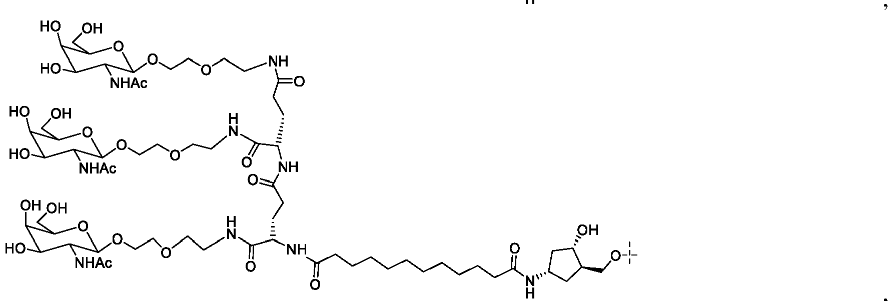 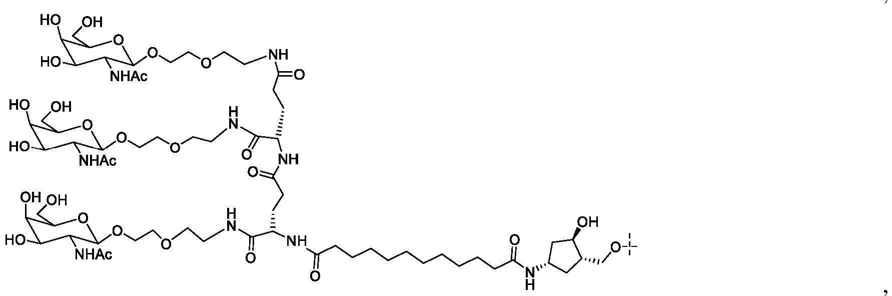 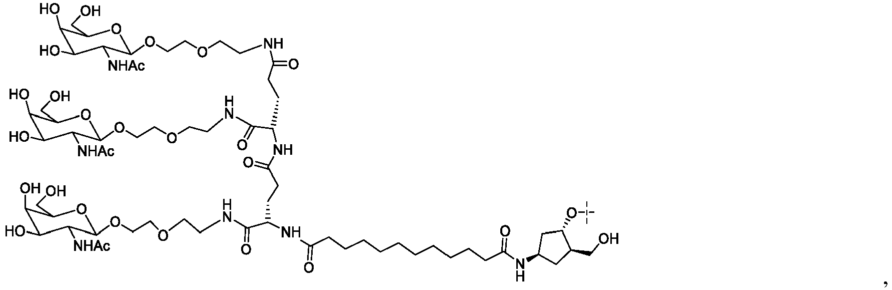 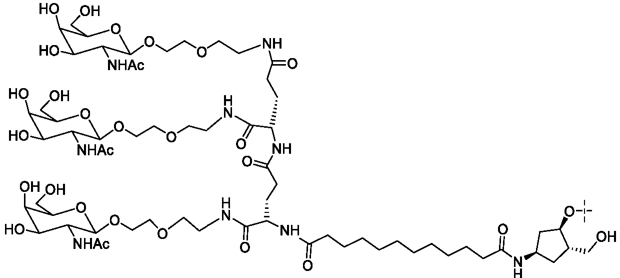 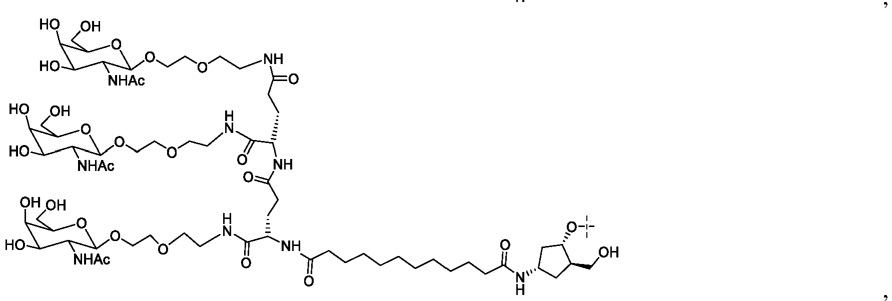 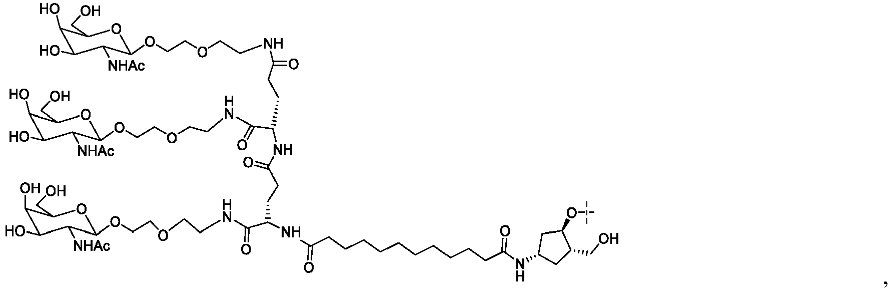 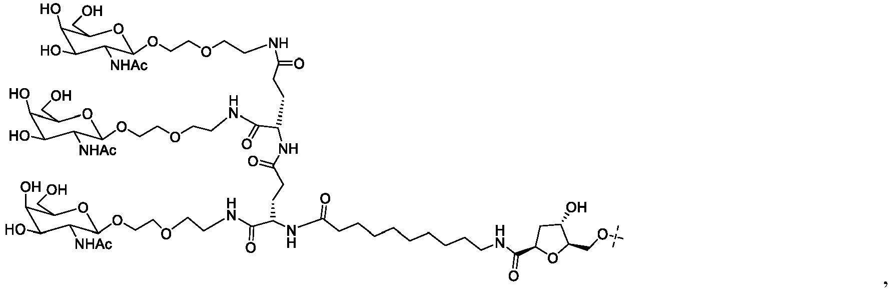 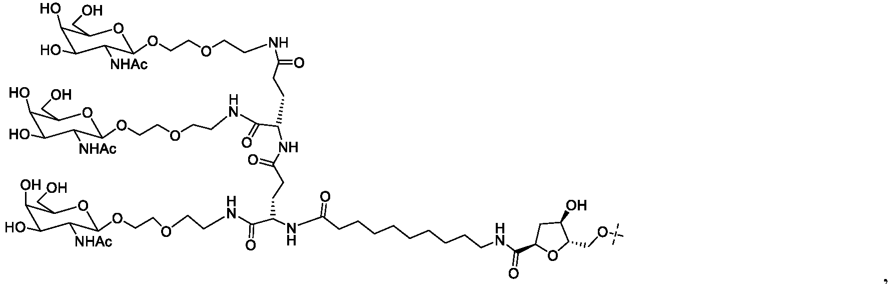 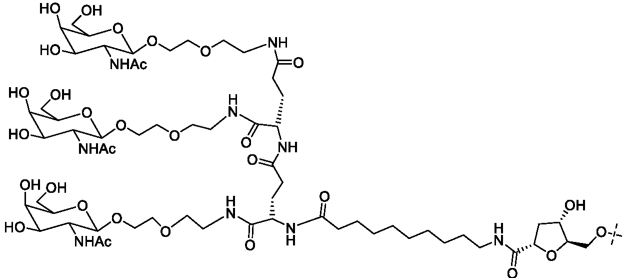 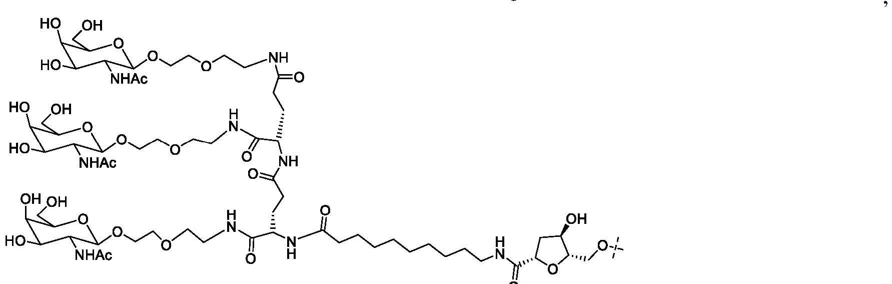 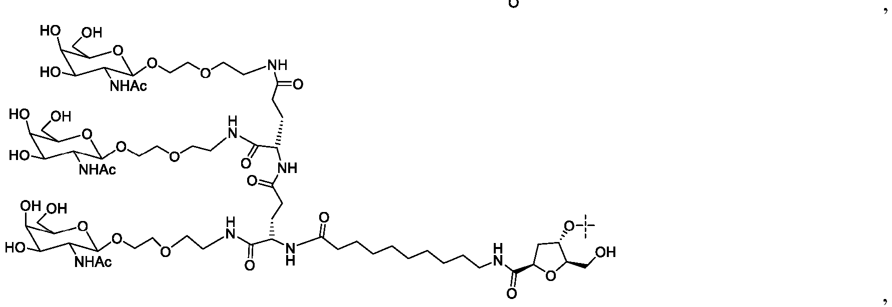 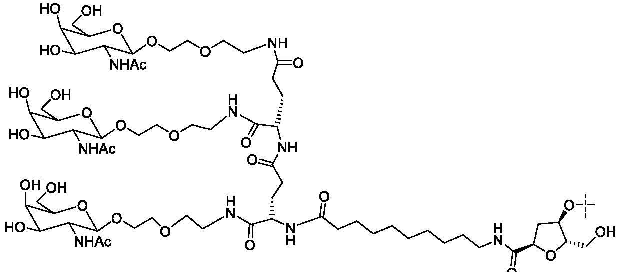 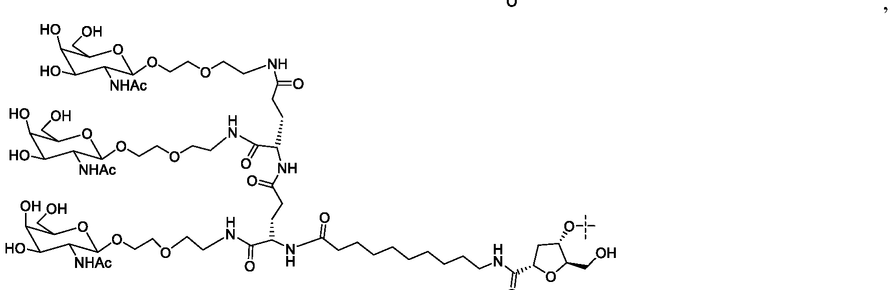 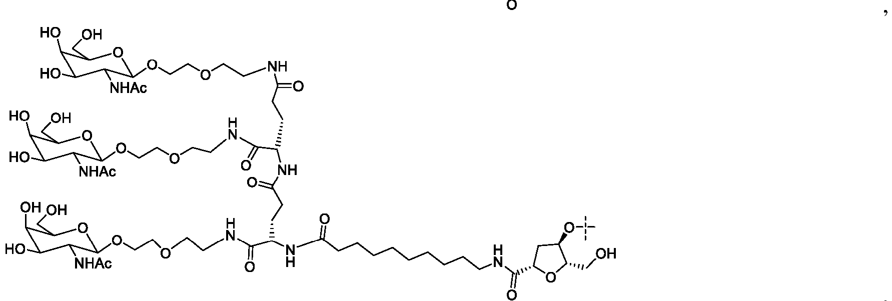 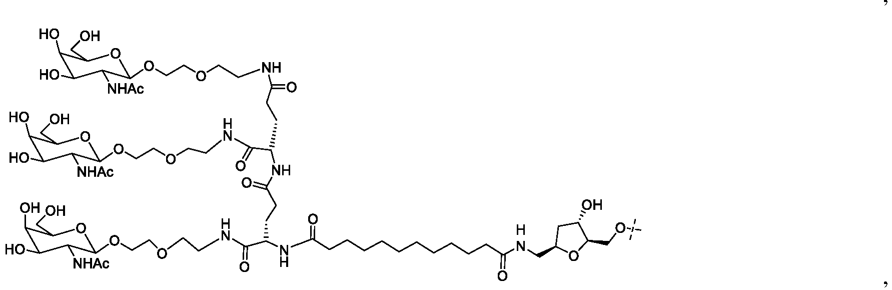 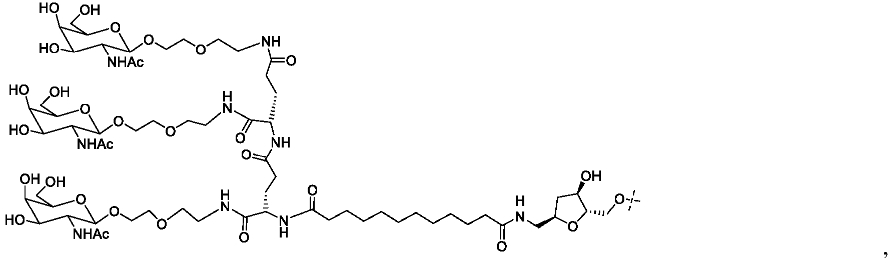 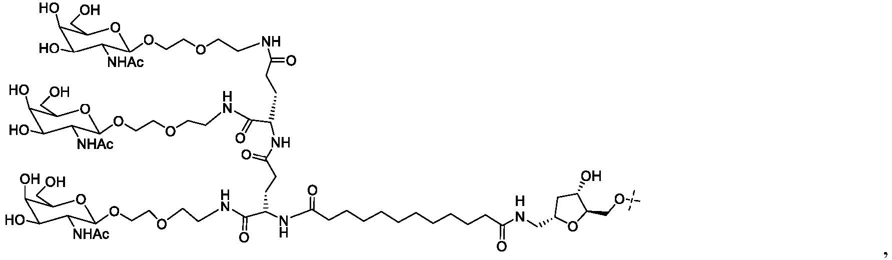 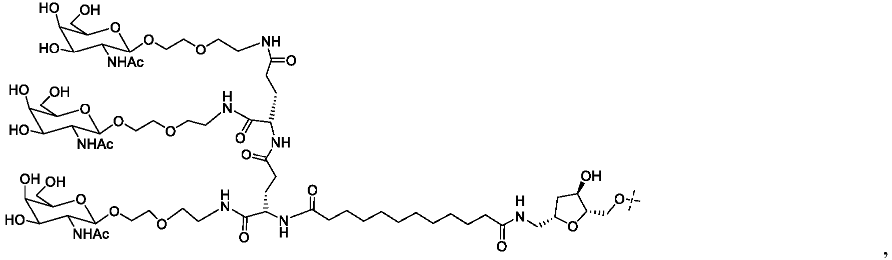 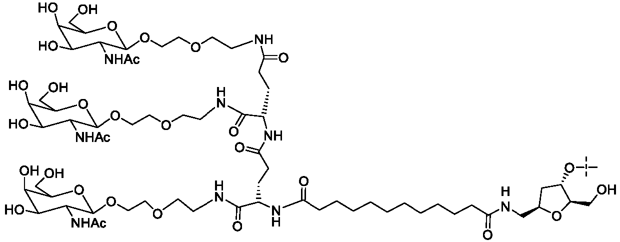 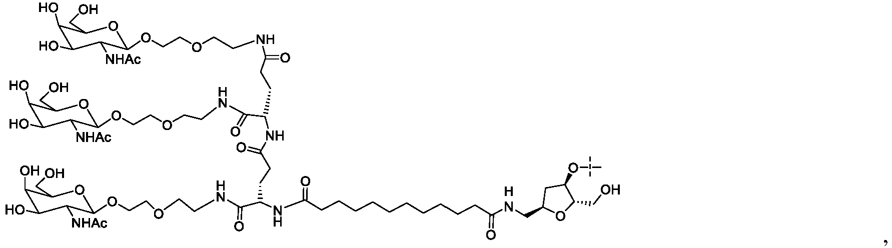 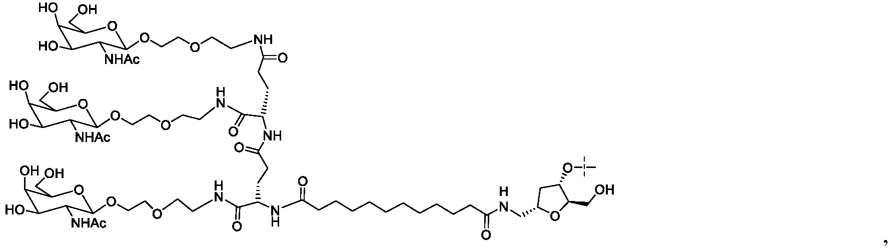 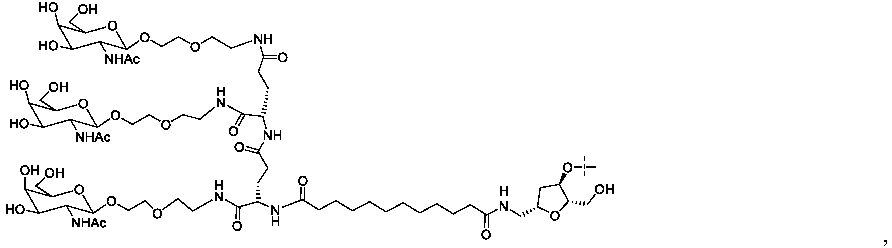 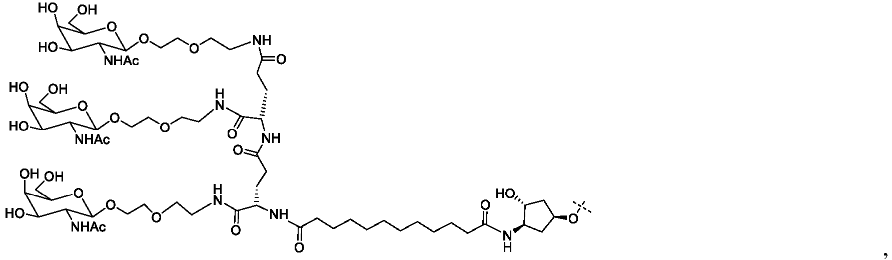 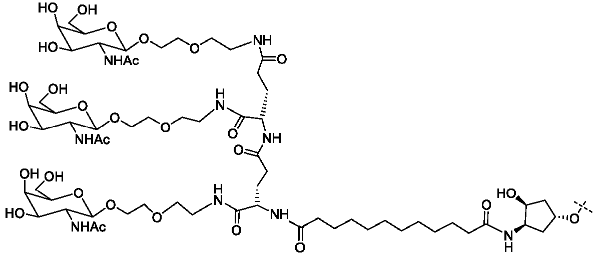 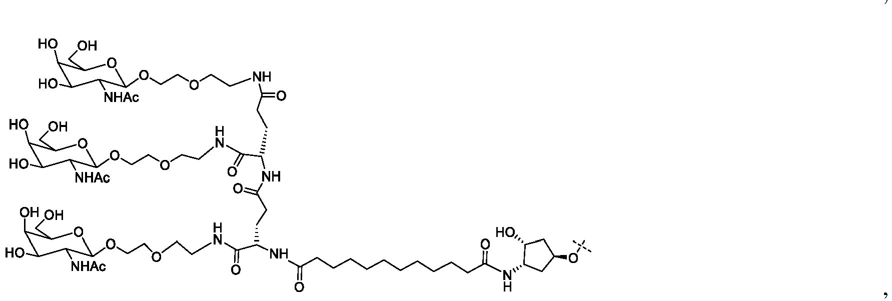 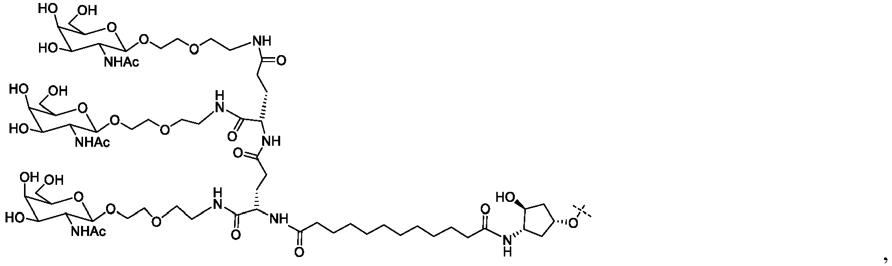 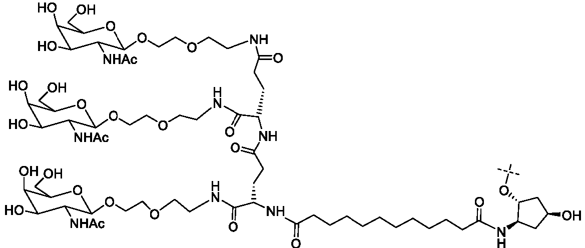 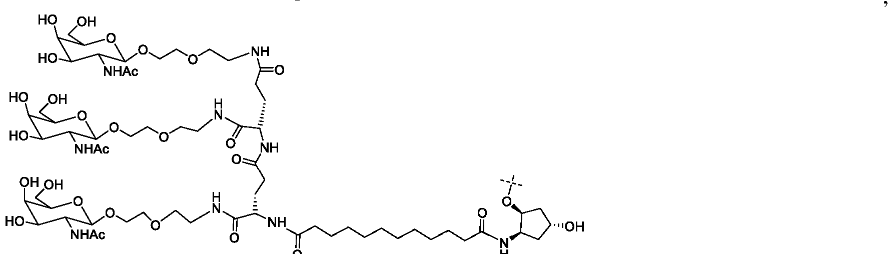 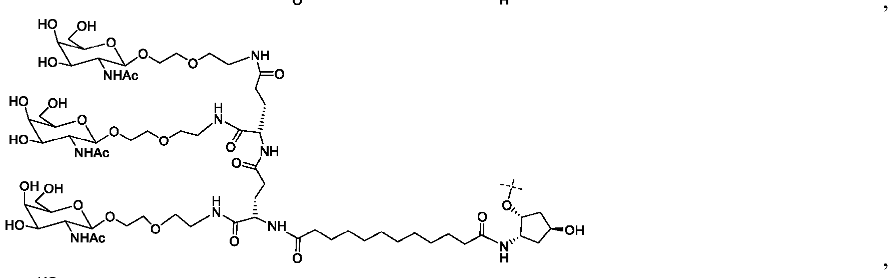 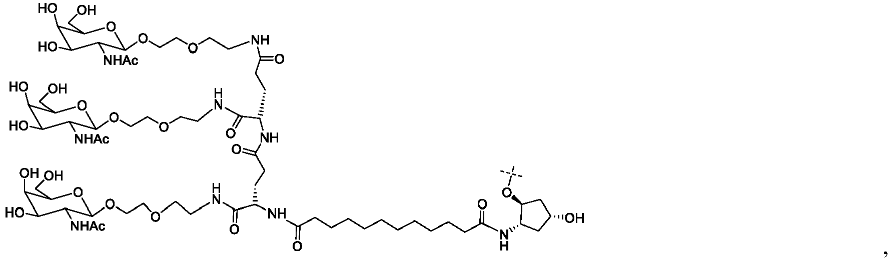 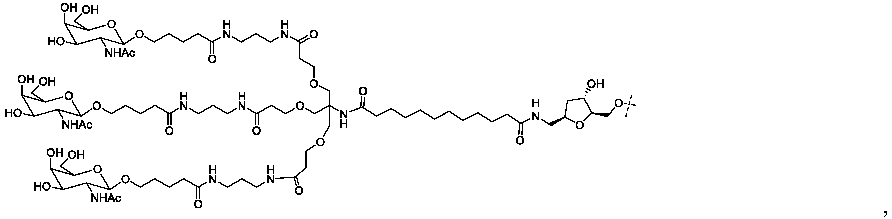 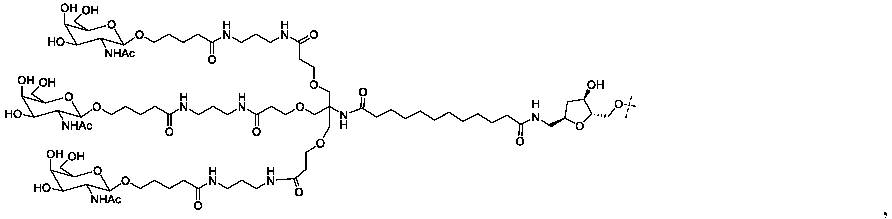 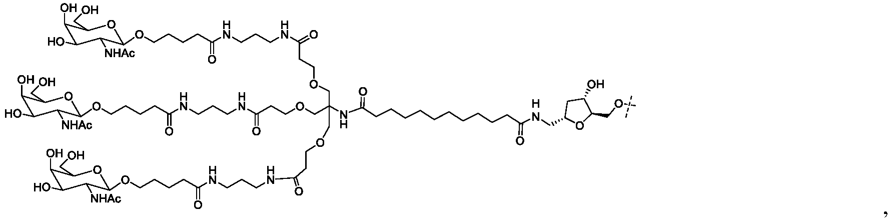 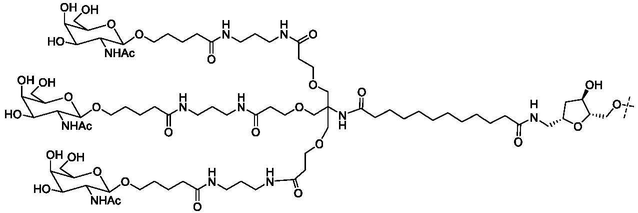 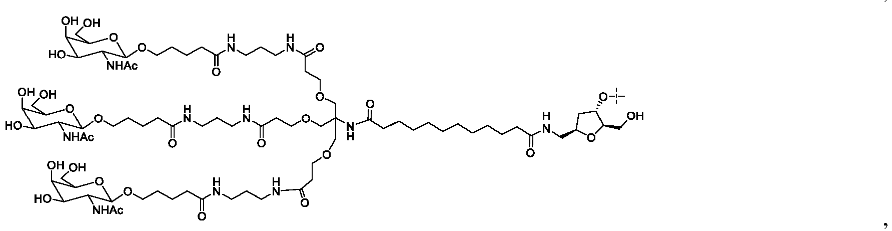 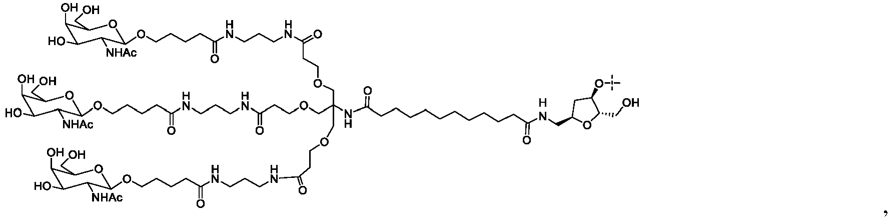 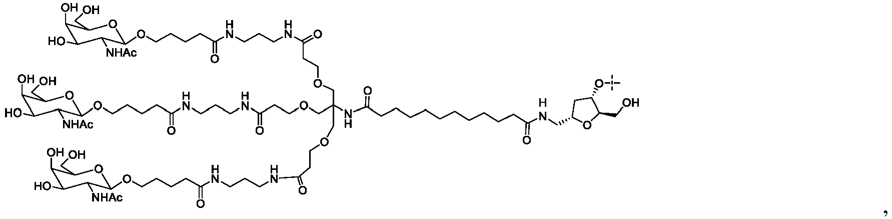 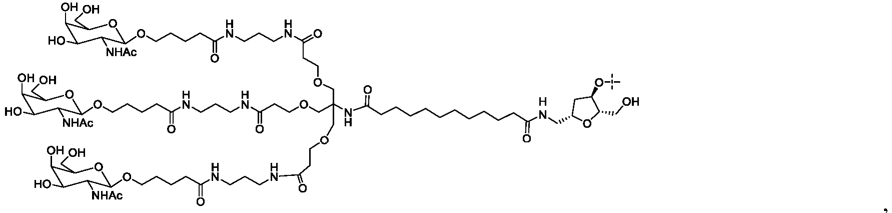 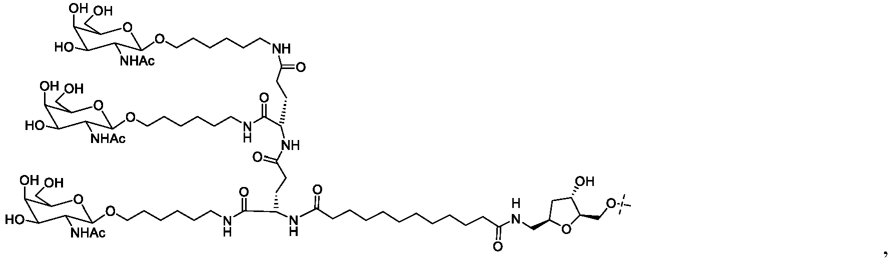 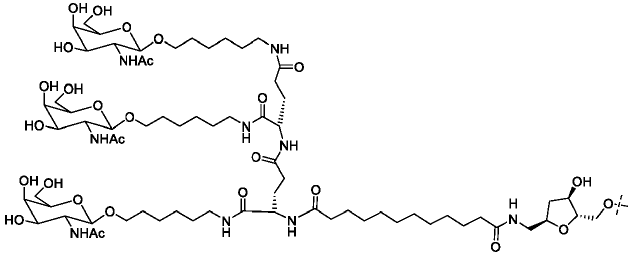 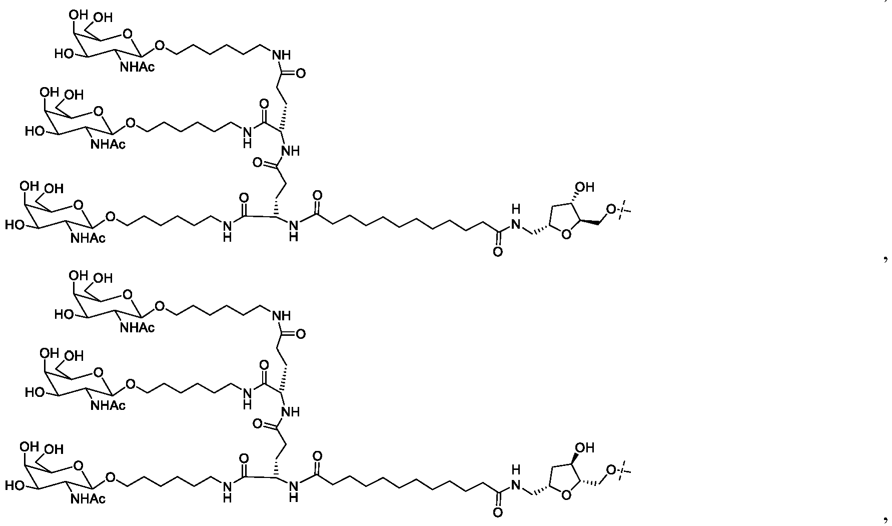 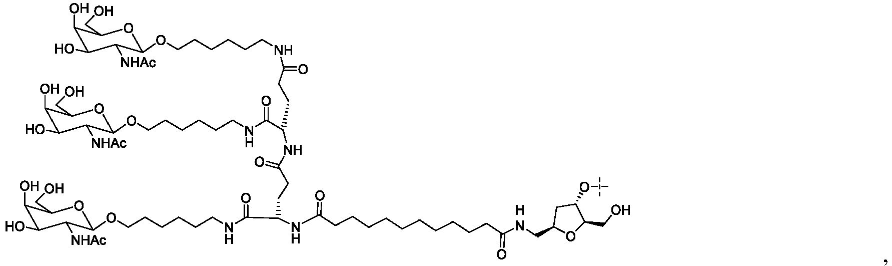 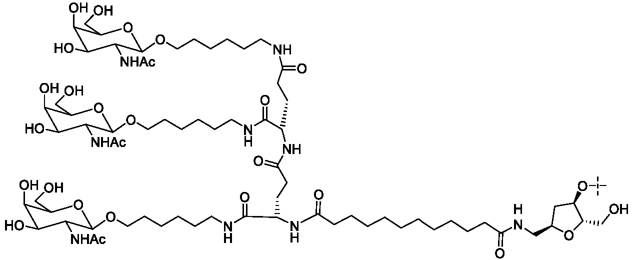 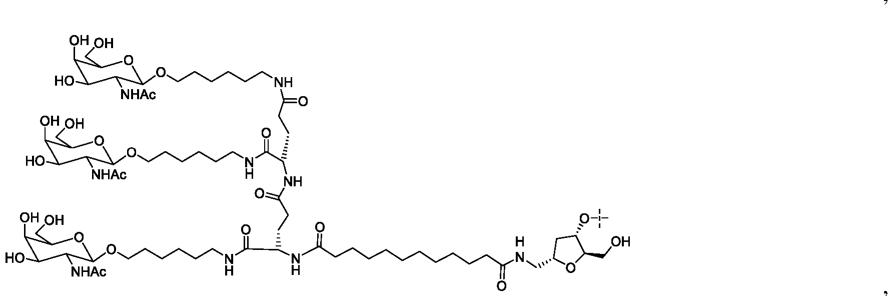 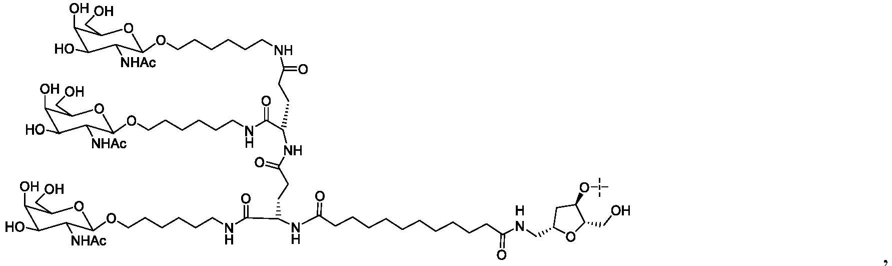 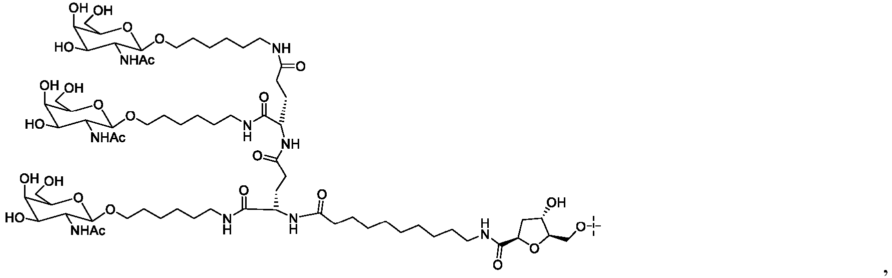 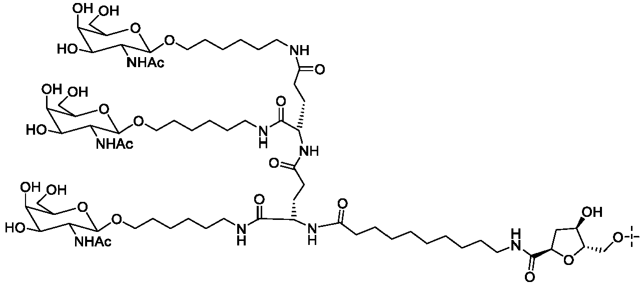 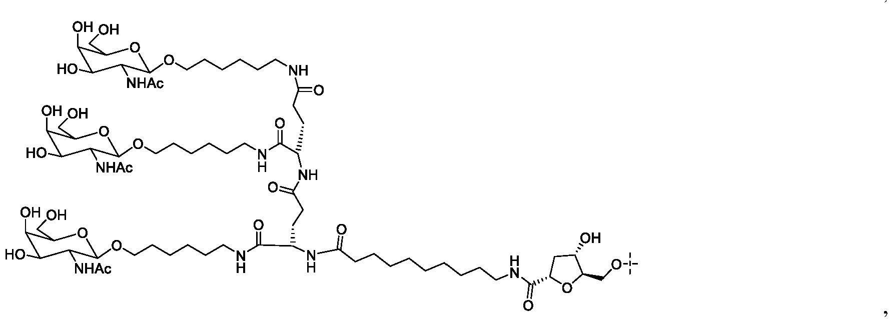 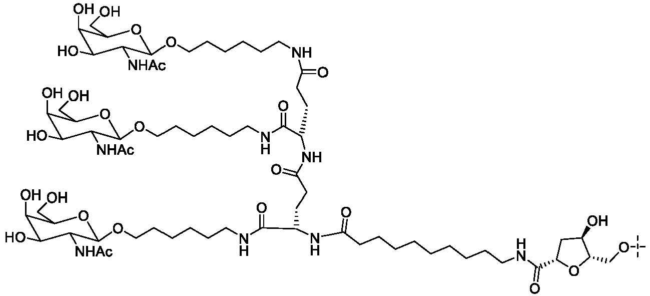 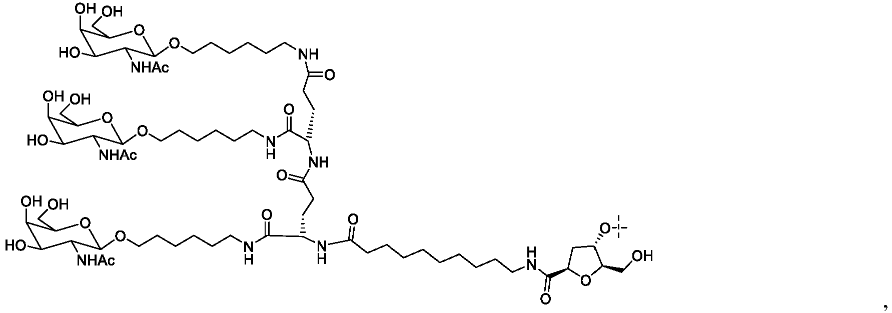 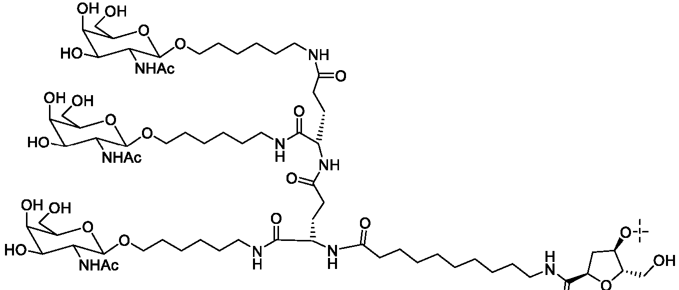 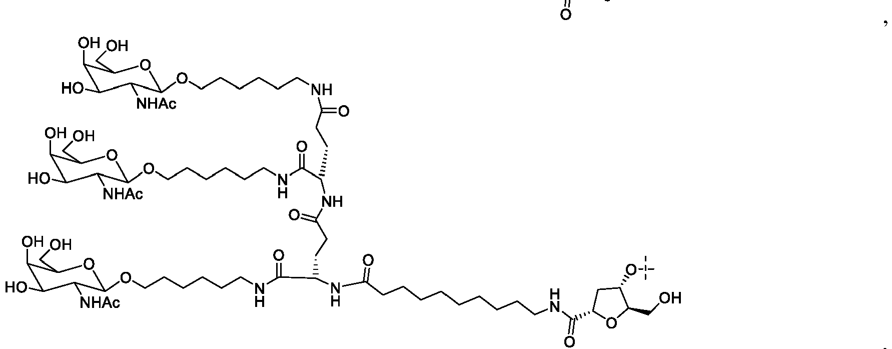 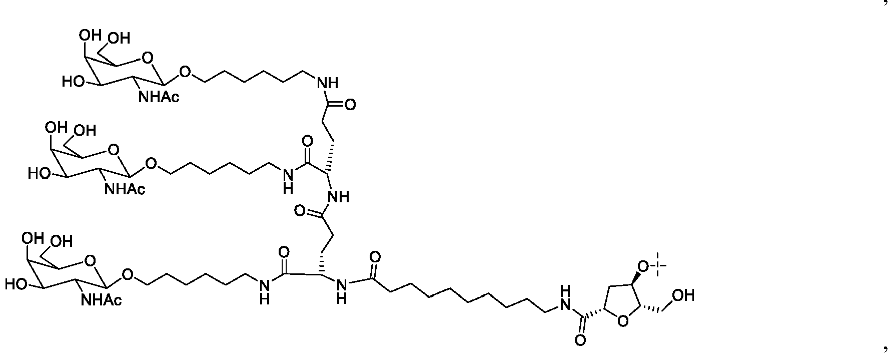 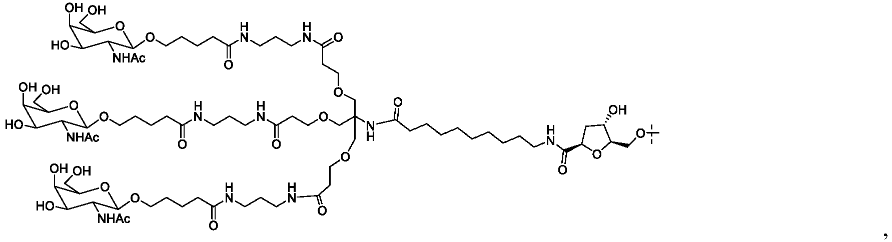 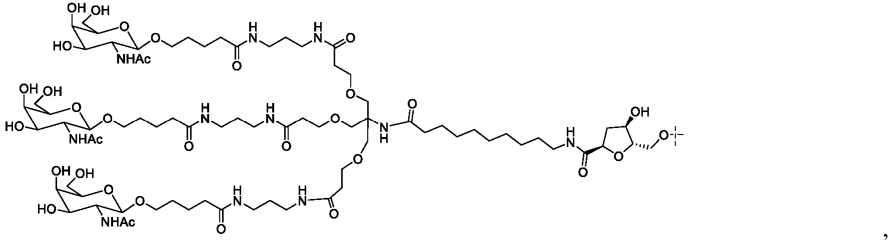 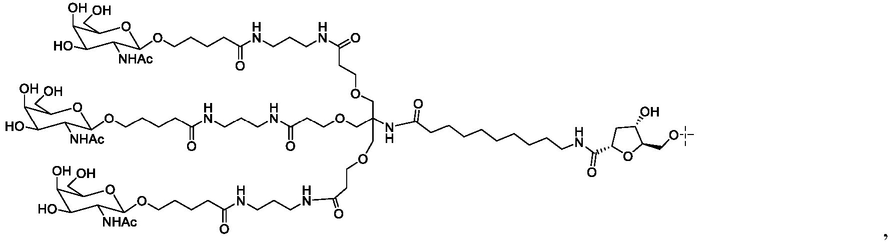 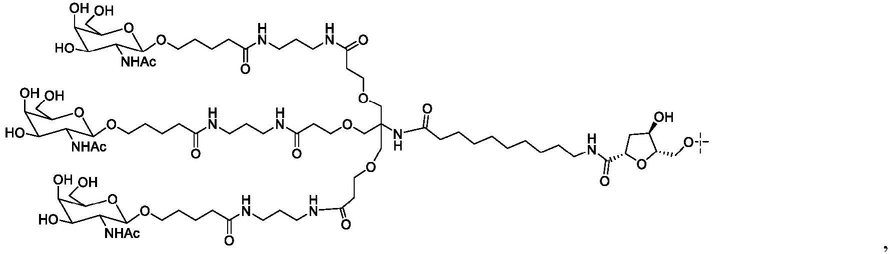 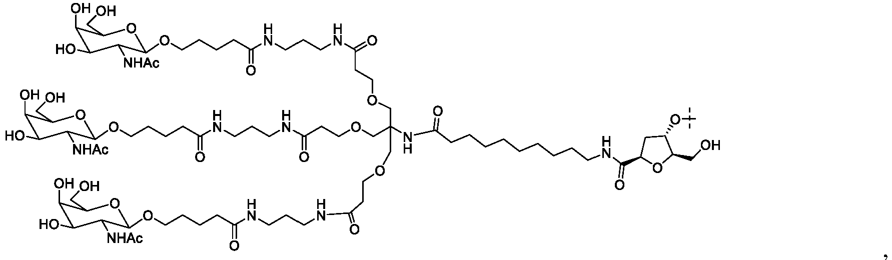 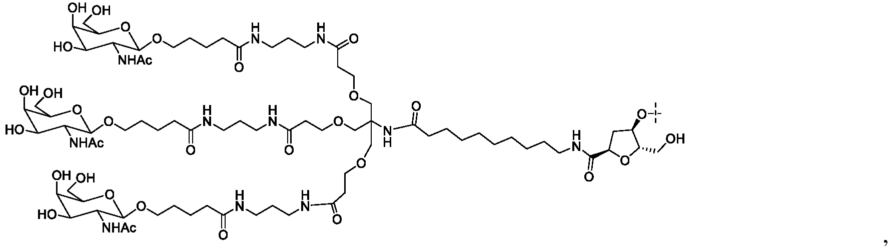 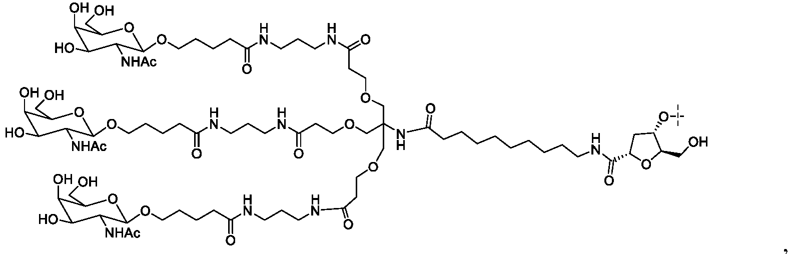 or

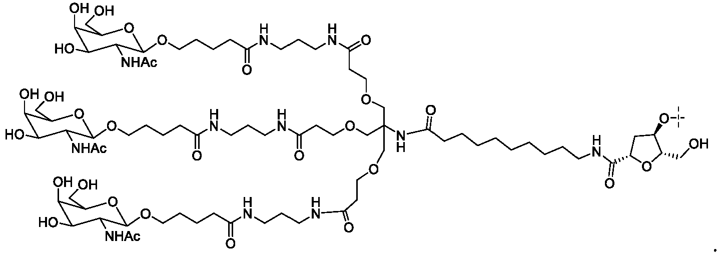

In some embodiments, the ligand may be the following structure or a pharmaceutically acceptable salt thereof:

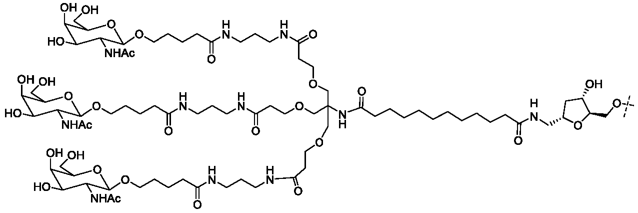

In some embodiments, N-trifluoroacetylgalactosamine, N-propionylgalactosamine, N-n-butyrylgalactosamine, or N-isobutyrylgalactosamine may be substituted for the N-acetylgalactosamine moiety in the above ligand.

In some embodiments, the chemical modification represented by formula (I) is 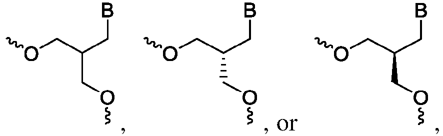 and B is selected from the group consisting of guanine, adenine, cytosine, and uracil;
the ligand is any one of the following structures or a pharmaceutically acceptable salt thereof: 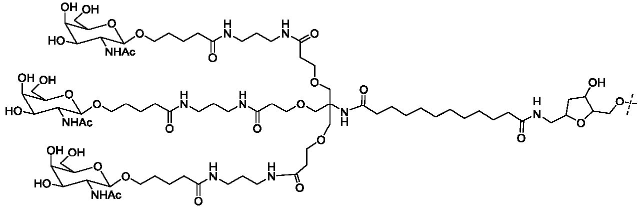 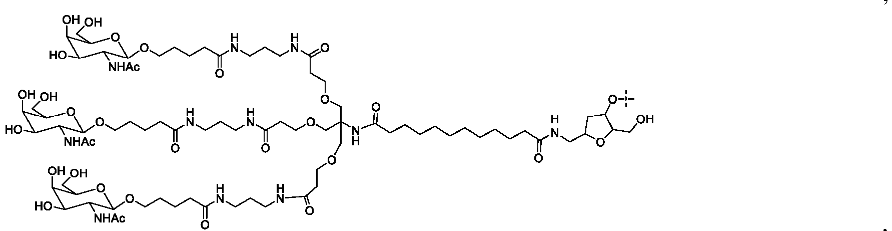 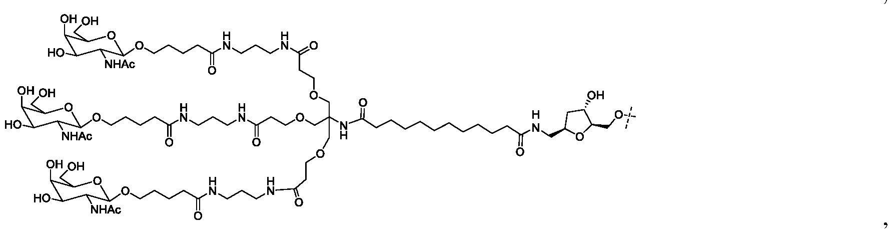 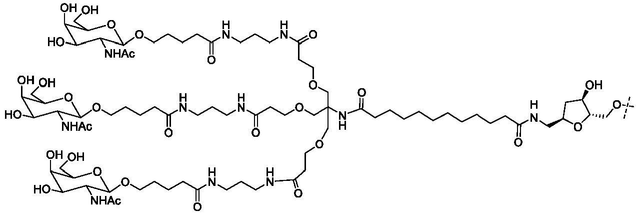 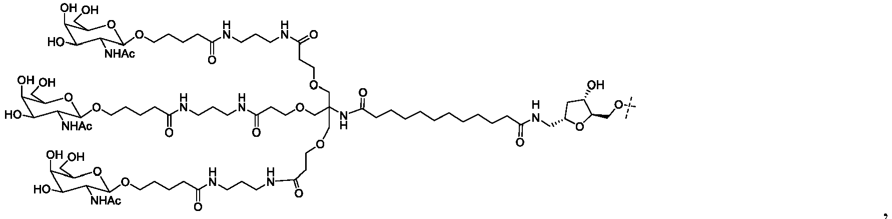 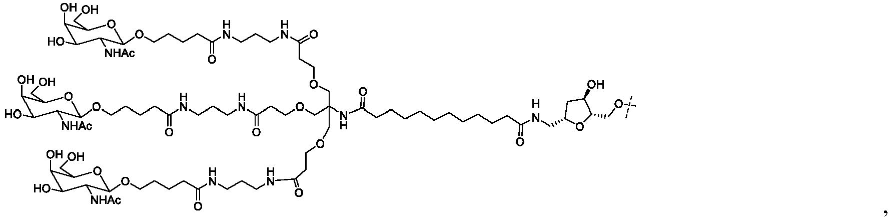 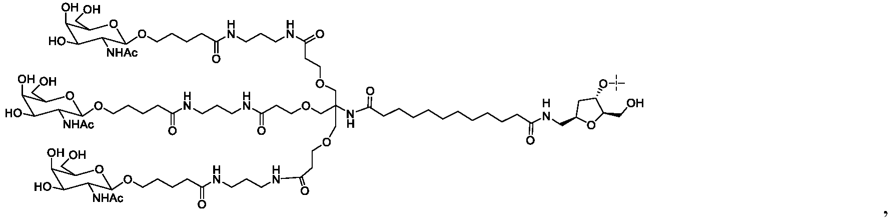 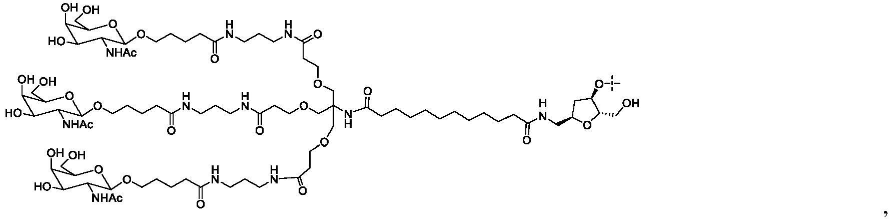 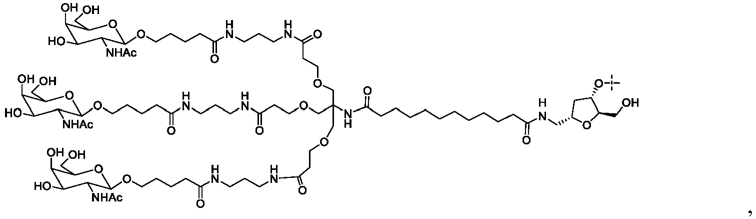 or 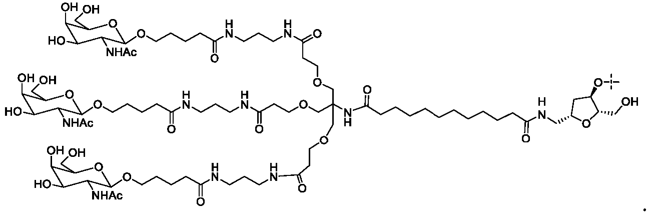 In some embodiments, the sense strand and the antisense strand are as defined in any one of the preceding embodiments.

In some embodiments, the ligand is any one of the following structures or a pharmaceutically acceptable salt thereof: 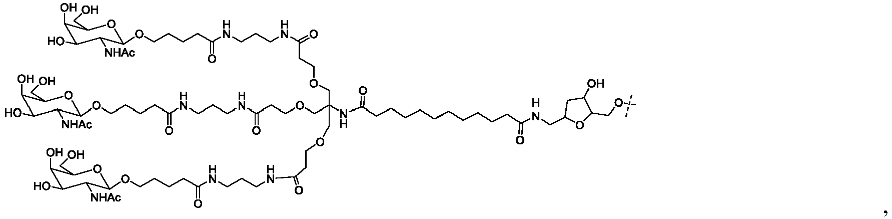 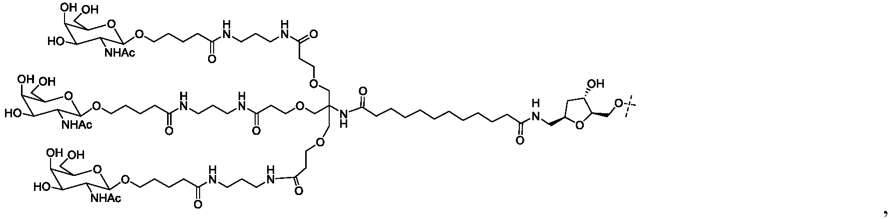 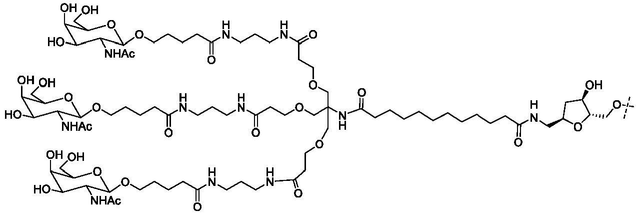 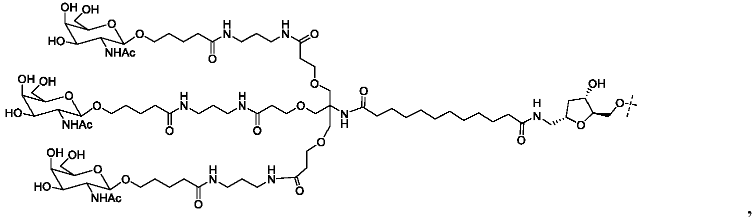 or

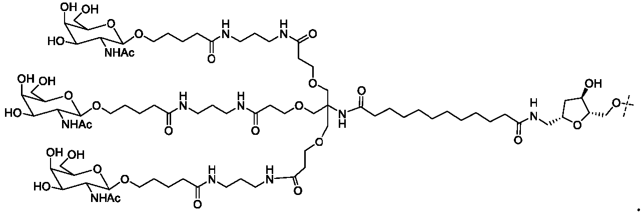

In some embodiments, the sense strand and the antisense strand are as defined in any one of the preceding embodiments.

In some embodiments, the chemical modification represented by formula (I) is 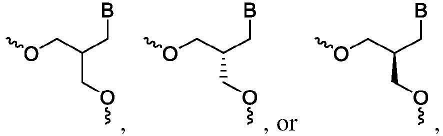 and B is selected from the group consisting of guanine, adenine, cytosine, and uracil;
the ligand is the following structure or a pharmaceutically acceptable salt thereof:

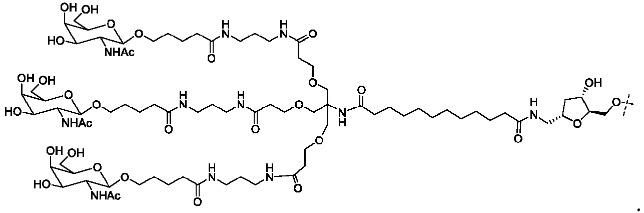

In some embodiments, the sense strand and the antisense strand are as defined in any one of the preceding embodiments.

In some embodiments, the sense strand and/or the antisense strand are/is covalently or non-covalently linked to the ligand.

In some embodiments, the 3' end and/or the 5' end of the sense strand is conjugated to the ligand.

In some embodiments, the 3' end of the sense strand is conjugated to the ligand.

In some embodiments, the ligand is linked to an end of the sense strand and/or the antisense strand by a phosphoester group or a phosphorothioate group.

In some embodiments, the ligand is linked to an end of the sense strand and/or the antisense strand by a phosphodiester group or a phosphorothioate diester group.

In some embodiments, the ligand is linked to an end of the sense strand and/or the antisense strand by a phosphodiester group.

In some embodiments, the ligand is indirectly linked to an end of the sense strand and/or the antisense strand by a phosphoester group or a phosphorothioate group.

In some embodiments, the ligand is directly linked to an end of the sense strand and/or the antisense strand by a phosphoester group or a phosphorothioate group.

In some embodiments, the ligand is directly linked to the 3' end of the sense strand by a phosphoester group or a phosphorothioate group.

In some embodiments, the phosphoester group is a phosphomonoester group or a phosphodiester group. In some embodiments, the phosphoester group is a phosphodiester group.

In some embodiments, the phosphorothioate group is a phosphorothioate monoester group or a phosphorothioate diester group. In some embodiments, the phosphorothioate group is a phosphorothioate diester group.

In some embodiments, to promote entry of the dsRNA into a cell, a lipophilic group such as cholesterol may be introduced to an end of the sense strand, and the lipophilic group is covalently bonded to a small interfering nucleic acid; for example, cholesterol, lipoprotein, vitamin E, etc., are introduced to the end to facilitate the crossing of the cell membrane consisting of a lipid bilayer and the interaction with the mRNA in the cell.

Meanwhile, the dsRNA may also be modified by non-covalent bonding, for example, bonding to a phospholipid molecule, a polypeptide, a cationic polymer, etc, by a hydrophobic bond or an ionic bond to increase stability and biological activity.

In some embodiments, the number of the ligands includes, but is not limited to: 1. 2, 3, or 4. In some embodiments, the number of the ligands is 1.

In some embodiments, the nucleotide sequence of the sense strand of the dsRNA comprises or is selected from the group consisting of SEQ ID NO: 4, and the nucleotide sequence of the antisense strand comprises or is selected from the group consisting of the nucleotide sequence of any one of SEQ ID NO: 5 to SEQ ID NO: 14;

In some embodiments, the dsRNA is any one of the following:
the sense strand comprises the nucleotide sequence of SEQ ID NO: 4, and the antisense strand comprises the nucleotide sequence of SEQ ID NO: 5;
the sense strand comprises the nucleotide sequence of SEQ ID NO: 4, and the antisense strand comprises the nucleotide sequence of SEQ ID NO: 6;
the sense strand comprises the nucleotide sequence of SEQ ID NO: 4, and the antisense strand comprises the nucleotide sequence of SEQ ID NO: 7;
the sense strand comprises the nucleotide sequence of SEQ ID NO: 4, and the antisense strand comprises the nucleotide sequence of SEQ ID NO: 8;
the sense strand comprises the nucleotide sequence of SEQ ID NO: 4, and the antisense strand comprises the nucleotide sequence of SEQ ID NO: 9;
the sense strand comprises the nucleotide sequence of SEQ ID NO: 4, and the antisense strand comprises the nucleotide sequence of SEQ ID NO: 10;
the sense strand comprises the nucleotide sequence of SEQ ID NO: 4, and the antisense strand comprises the nucleotide sequence of SEQ ID NO: 11;
the sense strand comprises the nucleotide sequence of SEQ ID NO: 4, and the antisense strand comprises the nucleotide sequence of SEQ ID NO: 12;
the sense strand comprises the nucleotide sequence of SEQ ID NO: 4, and the antisense strand comprises the nucleotide sequence of SEQ ID NO: 13;
the sense strand comprises the nucleotide sequence of SEQ ID NO: 4, and the antisense strand comprises the nucleotide sequence of SEQ ID NO: 14.

In some embodiments, the dsRNA is any one of the following:
the sense strand consists of the nucleotide sequence set forth in SEQ ID NO: 4, and the antisense strand consists of the nucleotide sequence set forth in SEQ ID NO: 5;
the sense strand consists of the nucleotide sequence set forth in SEQ ID NO: 4, and the antisense strand consists of the nucleotide sequence set forth in SEQ ID NO: 6;
the sense strand consists of the nucleotide sequence set forth in SEQ ID NO: 4, and the antisense strand consists of the nucleotide sequence set forth in SEQ ID NO: 7;
the sense strand consists of the nucleotide sequence set forth in SEQ ID NO: 4, and the antisense strand consists of the nucleotide sequence set forth in SEQ ID NO: 8;
the sense strand consists of the nucleotide sequence set forth in SEQ ID NO: 4, and the antisense strand consists of the nucleotide sequence set forth in SEQ ID NO: 9;
the sense strand consists of the nucleotide sequence set forth in SEQ ID NO: 4, and the antisense strand consists of the nucleotide sequence set forth in SEQ ID NO: 10;
the sense strand consists of the nucleotide sequence set forth in SEQ ID NO: 4, and the antisense strand consists of the nucleotide sequence set forth in SEQ ID NO: 11;
the sense strand consists of the nucleotide sequence set forth in SEQ ID NO: 4, and the antisense strand consists of the nucleotide sequence set forth in SEQ ID NO: 12;
the sense strand consists of the nucleotide sequence set forth in SEQ ID NO: 4, and the antisense strand consists of the nucleotide sequence set forth in SEQ ID NO: 13;
the sense strand consists of the nucleotide sequence set forth in SEQ ID NO: 4, and the antisense strand consists of the nucleotide sequence set forth in SEQ ID NO: 14.

In some embodiments, the dsRNA is any one of the following:
the dsRNA comprises a sense strand set forth in SEQ ID NO: 4 and an antisense strand set forth in any one of SEQ ID NO: 5 to SEQ ID NO: 14.

In some embodiments, the dsRNA is any one of the following:
the dsRNA is selected from the group consisting of a sense strand set forth in SEQ ID NO: 4 and an antisense strand set forth in any one of SEQ ID NO: 5 to SEQ ID NO: 14.

In the present disclosure, in the 5'-3' direction,
SEQ ID NO: 4 is
   UmsAmsUmUmCmUmCfAfGfUmGmCmUmCmUmCmCmUmAm-**NAG0052';**
SEQ ID NO: 5 is
   UmsAfsGmGfAmGf(-)hmpNA(A)GmCmAfCmUfGmAfGmAfAmUmAmsCmsUm;
SEQ ID NO: 6 is
   UmsAfsGmGfAmGf(-)hmpNA(A)GmCmAfCmUmGmAfGmAfAmUfAmsCmsUm;
SEQ ID NO: 7 is
   UmsAfsGmGfAmGf(-)hmpNA(A)GmCmAmCmUfGmAfGmAfAmUfAmsCmsUm;
SEQ ID NO: 8 is
   UmsAfsGmGmAmGf(-)hmpNA(A)GmCmAfCmUfGmAfGmAfAmUfAmsCmsUm;
SEQ ID NO: 9 is
   UmsAfsGmGmAmGf(-)hmpNA(A)GmCmAmCmUmGmAfGmAfAmUmAmsCmsUm
SEQ ID NO: 10 is
   UmsAfsGmGfAmGf(-)hmpNA(A)GmCmAmCmUmGmAfGmAfAmUmAmsCmsUm;
SEQ ID NO: 11 is
   UmsAfsGmGmAmGf(-)hmpNA(A)GmCmAfCmUmGmAfGmAfAmUmAmsCmsUm;
SEQ ID NO: 12 is
   UmsAfsGmGmAmGf(-)hmpNA(A)GmCmAmCmUfGmAfGmAfAmUmAmsCmsUm;
SEQ ID NO: 13 is
   UmsAfsGmGmAmGm(-)hmpNA(A)GfCfAmCmUmGmAfGmAfAmUmAmsCmsUm;
SEQ ID NO: 14 is 
UmsAfsGmGmAmGm(-)hmpNA(A)GmCmAmCmUmGmAfGmAmAmUmAmsCmsU
m;
wherein Af = adenine 2'-F ribonucleoside; Cf = cytosine 2'-F ribonucleoside; Uf = uracil 2'-F ribonucleoside; Gf = guanine 2'-F ribonucleoside; Am = adenine 2'-OMe ribonucleoside; Cm = cytosine 2'-OMe ribonucleoside; Gm = guanine 2'-OMe ribonucleoside; Um = uracil 2'-OMe ribonucleoside;
s indicates that the two nucleotides adjacent to the letter s are linked by a phosphorothioate diester group;
NAG0052' represents 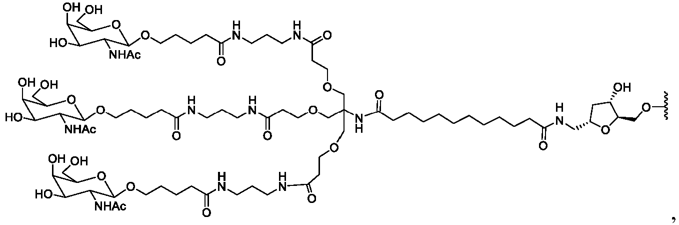 and
(-)hmpNA(A) represents

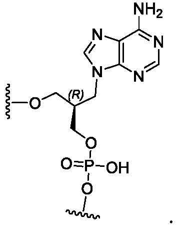

In some embodiments, the dsRNA is selected from the group consisting of the following structures and pharmaceutically acceptable salts thereof: 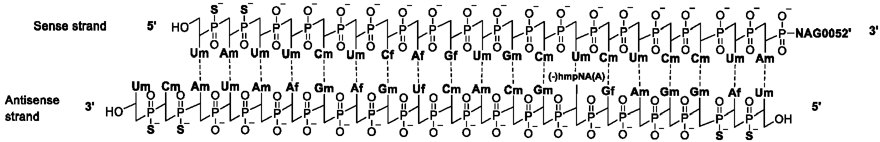 wherein Af = adenine 2'-F ribonucleoside; Cf = cytosine 2'-F ribonucleoside; Uf = uracil 2'-F ribonucleoside; Am = adenine 2'-OMe ribonucleoside; Cm = cytosine 2'-OMe ribonucleoside; Gf = guanine 2'-F ribonucleoside; Gm = guanine 2'-OMe ribonucleoside; Um = uracil 2'-OMe ribonucleoside. 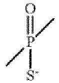 represents a phosphorothioate diester group, 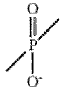 represents a phosphodiester group,
NAG0052' represents 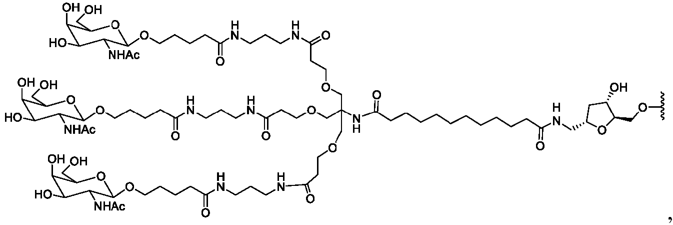 and
(-)hmpNA(A) represents

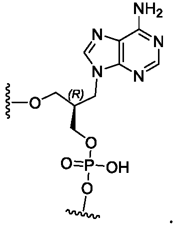

In some embodiments, the pharmaceutically acceptable salt may be a conventional salt in the art, including but not limited to sodium salts, potassium salts, ammonium salts, amine salts, etc.

In some embodiments, the dsRNA is selected from the group consisting of any one of TRD008043, TRD008069, TRD008042, TRD008070, TRD008071, TRD008072, TRD008073, TRD008074, TRD008075, and TRD008076.

In some embodiments, the dsRNA is TRD008043, which is the following structure: 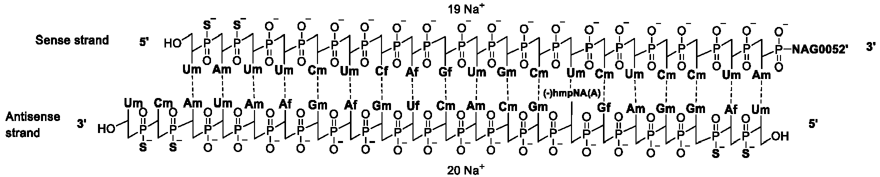 wherein Af = adenine 2'-F ribonucleoside; Cf = cytosine 2'-F ribonucleoside; Uf = uracil 2'-F ribonucleoside; Am = adenine 2'-OMe ribonucleoside; Cm = cytosine 2'-OMe ribonucleoside; Gf = guanine 2'-F ribonucleoside; Gm = guanine 2'-OMe ribonucleoside; Um = uracil 2'-OMe ribonucleoside. 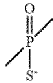 represents a phosphorothioate diester group, 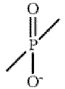 represents a phosphodiester group,
NAG0052' represents 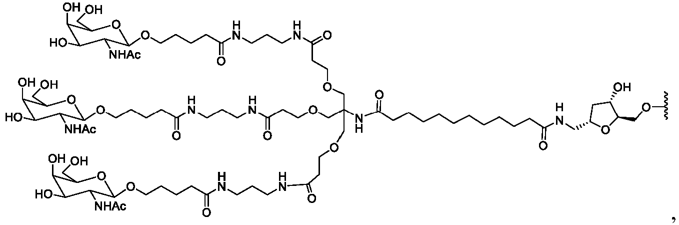 and
(-)hmpNA(A) represents

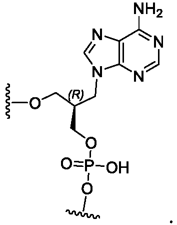

In another aspect, the present disclosure provides a pharmaceutical composition comprising the dsRNA described above.

In some embodiments, the pharmaceutical composition also comprises one or more pharmaceutically acceptable excipients. Various delivery systems are known and can be used for the dsRNA or pharmaceutical composition of the present disclosure, e.g., encapsulation in liposomes, microparticles, microcapsules, recombinant cells capable of expressing the compound, receptor-mediated endocytosis, and construction of a nucleic acid as part of a retroviral vector or other vectors.

In some embodiments, the dsRNA or the pharmaceutical composition of the present disclosure is administered by a conventional mode, which can be administered by local administration (e.g., direct injection or implantation) or systemic administration, or by oral, rectal, or parenteral routes. The parenteral routes include, but are not limited to, subcutaneous injection, intravenous injection, intramuscular injection, intraperitoneal injection, transdermal administration, inhalation administration (e.g., aerosol), mucosal administration (e.g., sublingual or intranasal administration), intracranial administration, etc.

In some embodiments, the dsRNA or the pharmaceutical composition provided by the present disclosure may be administered by injection, for example, intravenous, intramuscular, intradermal, subcutaneous, intraduodenal, or intraperitoneal injection.

In some embodiments, the dsRNA or the pharmaceutical composition provided by the present disclosure may be packaged in a kit.

In some embodiments, an effective amount or effective dose of the dsRNA or the pharmaceutical composition is about 0.001 mg/kg body weight to about 200 mg/kg body weight, about 0.01 mg/kg body weight to about 100 mg/kg body weight, or about 0.5 mg/kg body weight to about 50 mg/kg body weight.

The present disclosure provides use of the dsRNA described above or the pharmaceutical composition described above in the preparation of a medicament.

In some embodiments, the medicament may be used for reducing low-density lipoprotein levels in a subject, or for preventing and/or treating a disease mediated by elevated triglyceride levels or elevated cholesterol levels. In some embodiments, the disease is selected from the group consisting of hypertriglyceridemia, obesity, hyperlipidemia, abnormal lipid and/or cholesterol metabolism, atherosclerosis, cardiovascular disease, coronary artery disease, hypertriglyceridemia-induced pancreatitis, metabolic syndrome, type II diabetes, familial chylomicronemia syndrome, and familial partial lipodystrophy. In some embodiments, the medicament may be used for preventing and/or treating a disease associated with APOC3 gene expression. In some embodiments, the disease is selected from the group consisting of hypertriglyceridemia, obesity, hyperlipidemia, abnormal lipid and/or cholesterol metabolism, atherosclerosis, cardiovascular disease, coronary artery disease, hypertriglyceridemia-induced pancreatitis, metabolic syndrome, type II diabetes, familial chylomicronemia syndrome, and familial partial lipodystrophy. The present disclosure provides a method for preventing and/or treating a disease, comprising administering to a subject an effective amount or effective dose of the dsRNA described above or the pharmaceutical composition described above.

In some embodiments, the disease may be a disease mediated by elevated triglyceride levels or elevated cholesterol levels. In some embodiments, the disease is selected from the group consisting of hypertriglyceridemia, obesity, hyperlipidemia, abnormal lipid and/or cholesterol metabolism, atherosclerosis, cardiovascular disease, coronary artery disease, hypertriglyceridemia-induced pancreatitis, metabolic syndrome, type II diabetes, familial chylomicronemia syndrome, and familial partial lipodystrophy.

In some embodiments, the disease may be a disease associated with APOC3 gene expression. In some embodiments, the disease is selected from the group consisting of hypertriglyceridemia, obesity, hyperlipidemia, abnormal lipid and/or cholesterol metabolism, atherosclerosis, cardiovascular disease, coronary artery disease, hypertriglyceridemia-induced pancreatitis, metabolic syndrome, type II diabetes, familial chylomicronemia syndrome, and familial partial lipodystrophy.

The present disclosure provides a method for silencing the APOC3 gene or an mRNA thereof in a cell in vivo or in vitro, comprising the step of introducing the dsRNA described above or the pharmaceutical composition described above into the cell.

The present disclosure provides a method for inhibiting the expression of the APOC3 gene or an mRNA thereof, comprising administering to a subject an effective amount or effective dose of the dsRNA described above or the pharmaceutical composition described above.

In some embodiments, an effective amount or effective dose of the dsRNA or the pharmaceutical composition is about 0.001 mg/kg body weight to about 200 mg/kg body weight, about 0.01 mg/kg body weight to about 100 mg/kg body weight, or about 0.5 mg/kg body weight to about 50 mg/kg body weight.

The dsRNA or the pharmaceutical composition of the present disclosure may reduce the expression level of the target gene or an mRNA thereof in an object such as a cell, a cell population, a tissue, or a subject, including: administering to the object a therapeutically effective amount of the dsRNA or the pharmaceutical composition described herein to inhibit the expression of the target gene or the mRNA thereof in the object.

In some embodiments, the object has been previously identified as having a pathological upregulation of the target gene or the mRNA thereof in the targeted cell, cell population, tissue, or subject.

The present disclosure provides a method for delivering an oligonucleotide to the liver, comprising administering to a subject an effective amount or effective dose of the dsRNA described above or the pharmaceutical composition described above.

The present disclosure provides an RNA interference (RNAi) reagent comprising the dsRNA described above or the pharmaceutical composition described above.

In another aspect, the present disclosure also provides a cell comprising the dsRNA described above or the pharmaceutical composition described above.

In another aspect, the present disclosure also provides a kit comprising the dsRNA described above or the pharmaceutical composition described above.

In some embodiments, the dsRNA of the present disclosure is selected from the group consisting of an siRNA.

In the present disclosure, when the dsRNA or the pharmaceutical composition described above is in contact with a cell expressing the target gene, the dsRNA or the pharmaceutical composition described above inhibits the expression of the target gene by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry. In the present disclosure, when the dsRNA or the pharmaceutical composition described above is in contact with a cell expressing the target gene, the dsRNA or the pharmaceutical composition described above results in a target gene mRNA remaining expression percentage of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In the present disclosure, when the dsRNA or the pharmaceutical composition described above is in contact with a cell expressing the target gene, the dsRNA reduces off-target activity by at least 20%, at least 25%, at least 30%, at least 35%, at least 40%, at least 45%, at least 50%, at least 55%, at least 60%, at least 65%, at least 70%, or at least 75%, while maintaining on-target activity, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In the present disclosure, when the dsRNA or the pharmaceutical composition described above is in contact with a cell expressing the target gene, the dsRNA reduces off-target activity by at least 20%, at least 25%, at least 30%, at least 35%, at least 40%, at least 45%, at least 50%, at least 55%, at least 60%, at least 65%, at least 70%, or at least 75%, while reducing on-target activity by at most 20%, at most 19%, at most 15%, at most 10%, at most 5%, or more than 1%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In the present disclosure, when the dsRNA or the pharmaceutical composition described above is in contact with a cell expressing the target gene, the dsRNA reduces off-target activity by at least 20%, at least 25%, at least 30%, at least 35%, at least 40%, at least 45%, at least 50%, at least 55%, at least 60%, at least 65%, at least 70%, or at least 75%, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75% or at least 80%, as measured by, for example, psiCHECK activity screening and luciferase reporter gene assay, other mthods such as PCR or branched DNA (bDNA)-based methods, or protein-based methods such as immunofluorescence assay, e.g., western blot or flow cytometry. The compounds of the present disclosure may be present in specific geometric or stereoisomeric forms. The present disclosure contemplates all such compounds, including cis and trans isomers, (-)- and (+)-enantiomers, (*R*)- and (*S*)-enantiomers, diastereomers, (*D*)-isomer, (*L*)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure. The compounds of the present disclosure containing asymmetric carbon atoms may be separated in optically active pure form or in racemic form. The optically active pure form may be isolated from a racemic mixture or synthesized using chiral starting materials or chiral reagents.

Optically active (*R*)- and (*S*)-isomers, and *D*- and *L*-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

In the chemical structures of the compounds of the present disclosure, a bond " 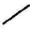 " represents an unspecified configuration; that is, if chiral isomers exist in the chemical structures, the bond " 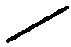 " may be " 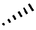 " or " 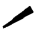 ", or both the configurations of " 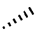 " and " 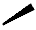 " are included simultaneously. In the chemical structures of the compounds of the present disclosure, a bond " 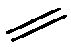 " does not specify a configuration; that is, the configuration for the bond " 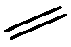 " can be an E configuration or a Z configuration, or includes both the *E* configuration and the *Z* configuration.

In the chemical structural formulas of the present disclosure, " 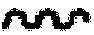 ", " 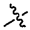 " or " 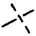 " may be linked to any group or groups in accordance with the scope of the invention described herein; the asterisk "*" indicates a chiral center.

When the configuration is not specified, the compounds and intermediates of the present disclosure can also be present in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

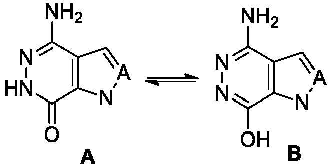

All compounds in the present disclosure can be drawn as form A or form B. All tautomeric forms are within the scope of the present disclosure. The names of the compounds do not exclude any tautomers.

The present disclosure also includes isotopically labeled compounds that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also comprises various deuterated forms of the compounds of formula (I), formula (I'), and formula (II). Each available hydrogen atom linked to a carbon atom may be independently replaced by a deuterium atom. Those skilled in the art are able to synthesize the deuterated forms of the compounds of formula (I), formula (I'), and formula (II) with reference to the relevant literature. Commercially available deuterated starting materials can be used in preparing the deuterated forms of the compounds of formula (I), formula (I') and formula (II), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

The present disclosure also provides a method for preparing a dsRNA or a pharmaceutical composition, comprising: synthesizing the dsRNA or the pharmaceutical composition described in the present disclosure.

In some embodiments, "comprising" may be replaced with "consisting of...". WO2022028462A1, WO2023274395A, and PCT/CN2022/139462 are incorporated in the present disclosure by reference in their entirety.

### Terms and Definitions

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. Unless otherwise specified, "optionally" or "optional" means that the event or circumstance subsequently described may, but does not necessarily, occur, and this description includes instances where the event or circumstance occurs or does not occur. For example, "optionally, R₁ and R₂ are directly linked to form a ring" means that R₁ and R₂ being directly linked to form a ring may occur but does not necessarily exist, and this description includes an instance where R₁ and R₂ are directly linked to form a ring and an instance where R₁ and R₂ do not form a ring.

The terms "about" and "approximately" mean that a numerical value is within an acceptable error range for the specific value determined by those of ordinary skill in the art, and the numerical value depends in part on how the value is measured or determined (i.e., the limitations of the measurement system). For example, "about" may mean a standard deviation within 1 or more than 1. Alternatively, "about" or "substantially comprise" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. Unless otherwise stated, when a specific value is provided in the present application and claims, the meaning of "about" or "substantially comprise" should be assumed to be within an acceptable error range for that specific value.

Unless otherwise specified, the terms "compound", "chemical modification", "ligand", "dsRNA", "nucleic acid" and "RNAi" of the present disclosure can each independently exist in the form of a salt, mixed salts, or a non-salt (e.g., a free acid or free base). When existing in the form of a salt or mixed salts, it can be a pharmaceutically acceptable salt. "Pharmaceutically acceptable salt" may be selected from the group consisting of inorganic or organic salts, and may also include pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-amino salicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts, and magnesium salts; sodium salts are preferred. Salts derived from organic bases include, but are not limited to, salts of the following: primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

"Alkyl" refers to a saturated aliphatic hydrocarbon group that includes, for example, straight-chain and branched-chain groups containing 1 to 30 carbon atoms (C₁-C₃₀ alkyl), for example, alkyl containing 1 to 6 carbon atoms (C₁-C₆ alkyl), and for example, alkyl of 1 to 3 carbon atoms (C₁-C₃ alkyl). Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, various branched-chain isomers thereof, and the like.

The term "alkenyl" refers to a hydrocarbyl group containing at least one double bond. Non-limiting examples of alkenyl include, but are not limited to, ethenyl, 1-propenyl, 2-propenyl, 1-butenyl, or 2-butenyl and various branched-chain isomers thereof.

The term "alkynyl" refers to a hydrocarbyl group containing at least one triple bond. Non-limiting examples of alkynyl include, but are not limited to, ethynyl, 1-propynyl, 2-propynyl, 1-butynyl, or 2-butynyl and various branched-chain isomers thereof.

The term "alkoxy" refers to -O-(alkyl), wherein the alkyl is as defined above. Non-limiting examples of alkoxy include: methoxy, ethoxy, propoxy, and butoxy.

"Cycloalkyl" refers to a saturated or partially unsaturated monocyclic or polycyclic hydrocarbon substituent; the cycloalkyl ring contains 3 to 20 carbon atoms, preferably 3 to 6 carbon atoms, and more preferably 5 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, and the like. Polycyclic cycloalkyl includes spirocycloalkyl, fused cycloalkyl, and bridged cycloalkyl.

"Heterocycloalkyl" refers to a saturated or partially unsaturated, monocyclic or polycyclic cyclic hydrocarbon substituent that contains 3 to 20 ring atoms, one or more of which are heteroatoms selected from the group consisting of nitrogen, oxygen, and S(O)ₘ (where m is an integer of 0 to 2), but does not contain a ring moiety of -O-O-, -O-S-, or -S-S-, and the other ring atoms are carbon atoms. Preferably, it contains 3 to 12 ring atoms, 1 to 4 of which are heteroatoms; more preferably, it contains 3 to 7 ring atoms. Non-limiting examples of "heterocycloalkyl" groups include: 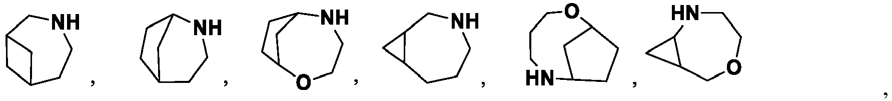 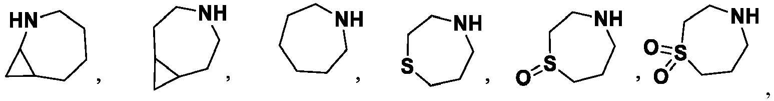 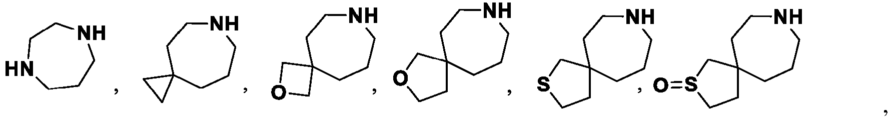 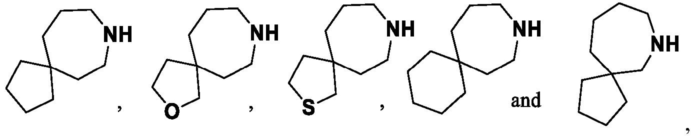 and the like.

The heterocycloalkyl ring may be fused to an aryl or heteroaryl ring, wherein the ring attached to the parent structure is heterocycloalkyl; its non-limiting examples include: 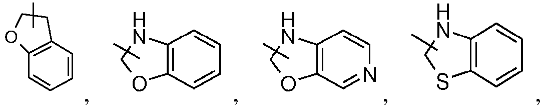 and the like.

"Aryl" refers to a 6- to 14-membered, preferably 6- to 12-membered, all-carbon monocyclic or fused polycyclic (i.e., rings that share a pair of adjacent carbon atoms) group having a conjugated π-electron system, such as phenyl and naphthyl. The aryl ring may be fused to a heteroaryl, heterocycloalkyl, or cycloalkyl ring, wherein the ring attached to the parent structure is the aryl ring; its non-limiting examples include, but are not limited to:

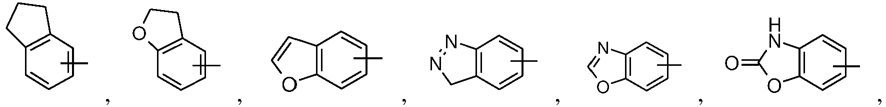

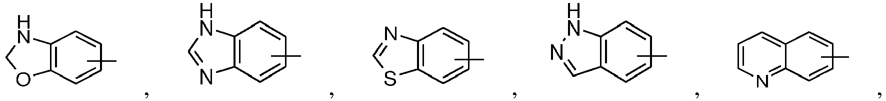

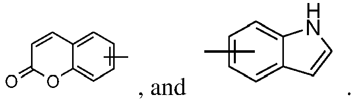

"Heteroaryl" refers to a heteroaromatic system containing 1 to 4 heteroatoms and 5 to 14 ring atoms, wherein the heteroatoms are selected from the group consisting of oxygen, sulfur, and nitrogen. Heteroaryl is preferably 6- to 12-membered, and is more preferably 5- or 6-membered. For example, its non-limiting examples include: imidazolyl, furanyl, thienyl, thiazolyl, pyrazolyl, oxazolyl, isoxazolyl, pyrrolyl, tetrazolyl, pyridinyl, pyrimidinyl, thiadiazole, pyrazinyl, triazolyl, indazolyl, benzimidazolyl, 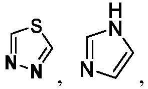 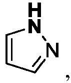 and the like.

The heteroaryl ring may be fused to an aryl, heterocycloalkyl, or cycloalkyl ring, wherein the ring attached to the parent structure is the heteroaryl ring; its non-limiting examples include:

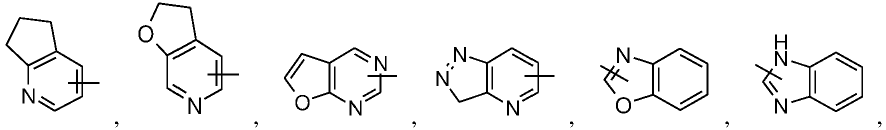

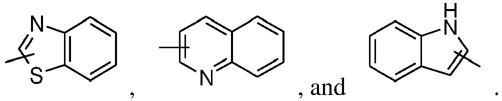

The term "hydroxy" refers to the -OH group.

The term "halogen" refers to fluorine, chlorine, bromine, or iodine.

The term "cyano" refers to -CN.

The term "amino" refers to -NH₂.

The term "nitro" refers to -NO₂.

The term "oxo" refers to the =O substituent.

In the present disclosure, the "phosphoester group" may be a phosphomonoester group, a phosphodiester group, or a phosphotriester group, preferably a phosphodiester group; the "phosphoester group" in the "phosphorothioate group" also has the same meaning.

In the present disclosure, the phosphorothioate diester group refers to a phosphodiester group modified by replacing one non-bridging oxygen atom with a sulfur atom, and is used interchangeably with 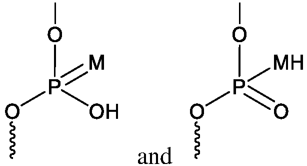 (M is a S atom).

"Substituted" means that one or more, preferably up to 5, and more preferably 1 to 3, hydrogen atoms in the group are independently substituted with a corresponding number of substituents. When the substituent is ketone or oxo (i.e., =O), two (2) hydrogens on the atom are replaced.

In the context of the present disclosure, the 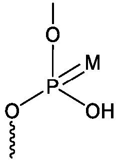 moiety in the group 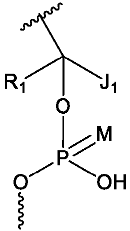 may be replaced with any group capable of linking to an adjacent nucleotide.

The term "link", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly linked" means that a first compound or group is linked to a second compound or group without any atom or group of atoms interposed between.

The term "indirectly linked" means that a first compound or group is linked to a second compound or group by an intermediate group, a compound, or a molecule (e.g., a linking group).

"Pharmaceutical composition" refers to a mixture containing one or more of the compounds or the physiologically and pharmaceutically acceptable salts or pro-drugs thereof described herein, and other chemical components, as well as other components, for example, physiologically and pharmaceutically acceptable carriers and excipients. The pharmaceutical composition is intended to promote administration to an organism and facilitate the absorption of the active ingredient so that it can exert its biological activity.

"Pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent or emulsifier that has been approved by the U.S. Food and Drug Administration (FDA) as acceptable for use in humans or livestock animals.

As used herein, the term "inhibit" is used interchangeably with "decrease", "silence", "down-regulate", "repress", and other similar terms, and includes any level of inhibition.

Inhibition can be assessed in terms of a decrease in the absolute or relative level of one or more of these variables relative to a control level. The control level may be any type of control level used in the art, such as a pre-dose baseline level or a level determined from a similar untreated or control (e.g., buffer-only control or inert agent control) treated subject, cell, or sample. For example, the remaining mRNA expression level can be used to characterize the degree of inhibition of target gene expression by the dsRNA; for example, the remaining expression level of mRNA is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition rate of target gene expression can be measured using Dual-Glo^{®} Luciferase Assay System: the Firefly chemiluminescence value and the Renilla chemiluminescence value are each read, and the relative value Ratio = Ren/Fir and inhibition rate (%) = 1-(Ratio + dsRNA/Ratioreporter only) × 100% are calculated; in the present disclosure, the proportion of remaining expression of mRNA (or residual activity%) = 100% - inhibition rate (%).

"Effective amount" or "effective dose" includes an amount sufficient to ameliorate or prevent a symptom or sign of a medical disorder. An effective amount also refers to an amount sufficient to allow or facilitate diagnosis. The effective amount for a particular patient or veterinary subject may vary depending on factors such as the disorder to be treated, the general health of the patient, the method and route and dosage of administration, and the severity of side effects. An effective amount may be the maximum dose or administration regimen to avoid significant side effects or toxic effects.

As used herein, "object", "patient", "subject" and "individual" are used interchangeably and include human or non-human animals, e.g., mammals, e.g., humans or monkeys.

As used herein, the sense strand (also referred to as SS or SS strand) refers to a strand that comprises a sequence that is identical or substantially identical to a target mRNA sequence; the antisense strand (also referred to as AS or AS strand) refers to a strand having a sequence complementary to a target mRNA sequence.

In the present disclosure, the "5' region", "5' end" and "5' terminus" of the sense or antisense strand are used interchangeably. For example, the nucleotides at positions 2 to 8 of the 5' region of the antisense strand may be replaced with the nucleotides at positions 2 to 8 of the 5' end of the antisense strand. Likewise, the "3' region", "3' terminus" and "3' end" of the sense or antisense strand are also used interchangeably.

In the context of describing the sense strand of the dsRNA described herein, the term "at least 15 contiguous nucleotides of a sequence that differs from the nucleotide sequence of SEQ ID NO: 1 by no more than 3 nucleotides" is intended to mean that the sense strand of the dsRNA described herein comprises at least 15 contiguous nucleotides from the sense strand set forth in SEQ ID NO: 1, or a sequence that differs from at least 15 contiguous nucleotides of the sense strand set forth in SEQ ID NO: 1 by no more than 3 nucleotides, optionally by no more than 2 nucleotides, and optionally by 1 nucleotide. Optionally, the sense strand of the dsRNA described herein comprises at least 16 contiguous nucleotides from the sense strand set forth in SEQ ID NO: 1, or a sequence that differs from at least 16 contiguous nucleotides of the sense strand set forth in SEQ ID NO: 1 by no more than 3 nucleotides, optionally, by no more than 2 nucleotides, and optionally, by 1 nucleotide;

In the context of describing the antisense strand of the dsRNA described herein, the term "at least 15 contiguous nucleotides of a sequence that differs from the antisense strand set forth in SEQ ID NO: 2 by no more than 3 nucleotides" is intended to mean comprising at least 15 contiguous nucleotides of the SEQ ID NO: 2 described herein, or a sequence that differs from at least 15 contiguous nucleotides of the antisense strand set forth in SEQ ID NO: 2 by no more than 3 nucleotides, optionally by no more than 2 nucleotides, and optionally by 1 nucleotide.

Unless otherwise specified, in the context of the present disclosure, "G", "C", "A", "T", and "U" each represent a nucleotide, and comprise the bases of guanine, cytosine, adenine, thymidine, and uracil, respectively. The lowercase letter m indicates that the nucleotide adjacent to the left of the letter m is a methoxy-modified nucleotide; the lowercase letter f indicates that the nucleotide adjacent to the left of the letter f is a fluoro-modified nucleotide; the lowercase letter s indicates that the two nucleotides adjacent to the letter s are linked by a phosphorothioate diester group.

As used in the present disclosure, the term "2'-fluoro (2'-F)-modified nucleotide" refers to a nucleotide in which the hydroxy group at the 2' position of the ribosyl group of the nucleotide is substituted with fluorine, and "non-fluoro-modified nucleotide" refers to a nucleotide or a nucleotide analog in which the hydroxy group at the 2' position of the ribosyl group of the nucleotide is substituted with a non-fluorine group.

As used in the present disclosure, the term "2'-methoxy (2'-OMe)-modified nucleotide" refers to a nucleotide in which the 2'-hydroxy group of the ribosyl group is substituted with a methoxy group.

In the context of the present disclosure, the "nucleotide difference" between one nucleotide sequence and another nucleotide sequence means that the former has a change in the base type of the nucleotide at the same position as compared to the latter; for example, when one nucleotide base in the latter is A, in the case where the corresponding nucleotide base at the same position in the former is U, C, G, or T, it is considered that there is a nucleotide difference at that position between the two nucleotide sequences. In some embodiments, the replacement of a nucleotide at its original position with an abasic nucleotide or an equivalent thereof can also be considered as creating a nucleotide difference at that position.

As used herein, the terms "complementary" and "reverse complementary" are used interchangeably and have the meaning well known to those skilled in the art; that is, in a double-stranded nucleic acid molecule, the bases of one strand are paired with the bases of the other strand in a complementary manner. In DNA, the purine base adenine is always paired with the pyrimidine base thymine (or uracil in RNA), and the purine base guanine is always paired with the pyrimidine base cytosine. Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

The term "dsRNA" refers to a double-stranded RNA molecule capable of RNA interference, comprising a sense strand and an antisense strand.

The term "chemical modification" or "modification" includes all changes made to a nucleotide by chemical means, such as the addition or removal of a chemical moiety, or the substitution of one chemical moiety for another.

The term "base" encompasses any known DNA and RNA bases and base analogs such as purines or pyrimidines, and also includes the natural compounds adenine, thymine, guanine, cytosine, uracil, inosine, and natural analogs. Base analogs can also be universal bases.

The terms "blunt" and "blunt ended" are used interchangeably and mean that there are no unpaired nucleotides or nucleotide analogs at a given terminus of a dsRNA, i.e., no nucleotide overhang. In most cases, a dsRNA whose both ends are blunt ended will be double-stranded over its entire length.

The dsRNA provided by the present disclosure can be obtained using a preparation method conventional in the art (e.g., solid-phase synthesis and liquid-phase synthesis). Solid-phase synthesis has been commercially available as a customization service. A modified nucleotide group can be introduced into the dsRNA of the present disclosure using a nucleoside monomer with a corresponding modification. Methods of preparing a nucleoside monomer with a corresponding modification and introducing a modified nucleotide group into a dsRNA are also well known to those skilled in the art.

### BRIEF DESCRIPTION OF THE DRAWINGS

FIG. 1 shows the expression levels of TTR mRNA at day 7 after administration of TRD002218 and TRD007205.
FIG. 2 shows the expression levels of TTR mRNA at day 28 after administration of TRD002218 and TRD007205.

### DETAILED DESCRIPTION

The present disclosure is further described below with reference to examples; however, these examples are not intended to limit the scope of the present disclosure. Experimental procedures without conditions specified in the examples of the present disclosure were generally conducted under conventional conditions or conditions recommended by the manufacturers of the starting materials or commercial products. Where the source of a reagent is not shown, the reagent may be obtained from any supplier of reagents for molecular biology at a quality/purity level for application in molecular biology.

### Example 1: Preparation of Chemical Modifications

### 1.1. Synthesis of compound 1-1a and compound 1-1b

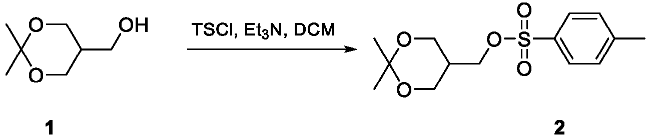

Compound 1 (500 mg, 3.42 mmol) and triethylamine (Et₃N, 692 mg, 6.84 mmol, 0.95 mL) were dissolved in dichloromethane (DCM, 10 mL), and a solution of 4-toluenesulfonyl chloride (TsCl, 717 mg, 3.76 mmol) in dichloromethane (10 mL) was added dropwise under an ice bath. After the dropwise addition was complete, the reaction mixture was stirred at room temperature overnight. After the reaction was complete, the reaction mixture was quenched with water. The aqueous phase was extracted three times with dichloromethane (15 mL). The organic phases were combined, washed first with saturated aqueous sodium bicarbonate solution (10 mL) and then with saturated brine (20 mL), and then concentrated under reduced pressure to evaporate the solvent to give crude **2** (820 mg, 80%), which was directly used in the next step. MS m/z: C₁₄H₂₁O₅S, [M+H]⁺ calculated: 301.10, found: 301.2.

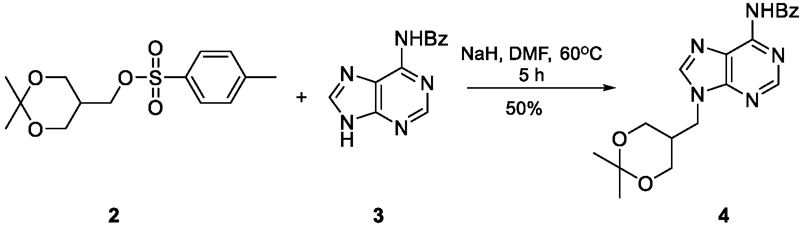

Compound **3** (239 mg, 1.22 mmol) was dissolved in dimethylformamide (DMF, 10 mL), and a solution of NaH (60% in mineral oil, 93 mg, 2.33 mmol) was added under an ice bath. The mixture was stirred for 30 min, and then compound **2** (350 mg, 1.16 mmol) was added dropwise. After the dropwise addition was complete, the mixture was stirred at 60 °C for 5 h. After the reaction was complete, the reaction mixture was quenched with water. The aqueous phase was extracted three times with ethyl acetate (15 mL). The organic phases were combined, washed first with water (10 mL) three times and then with saturated brine (10 mL), and then concentrated under reduced pressure to evaporate the solvent. The residue was purified by preparative reversed-phase HPLC (C¹⁸, condition: 5-50% (A: H₂O, B: CH₃CN), flow rate: 70 mL/min) and lyophilized to give compound **4** (220 mg). MS m/z: C₁₉H₂₁N₅O₃Na, [M+Na]⁺ calculated: 390.16, found: 390.3.

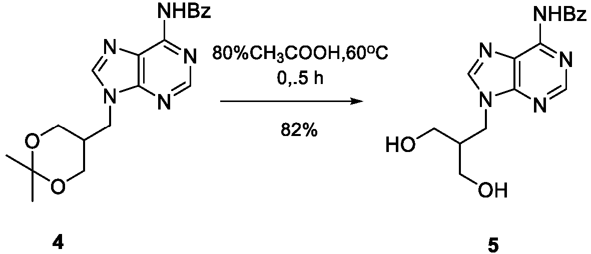

Compound **4** (1.50 g, 4.08 mmol) was dissolved in 20 mL of a mixed solution of acetic acid and water (4:1) at room temperature, and the mixture was stirred at 60 °C for 30 min. After the reaction was complete, the reaction mixture was concentrated under reduced pressure to evaporate the solvent. The residue was purified by preparative reversed-phase HPLC (C¹⁸, condition: 5-25% (A: H₂O, B: CH₃CN), flow rate: 70 mL/min) and lyophilized to give compound **5** (1.10 g). MS m/z: C₁₆H₁₈N₅O₃, [M+H]⁺ calculated: 328.13, found: 328.4.

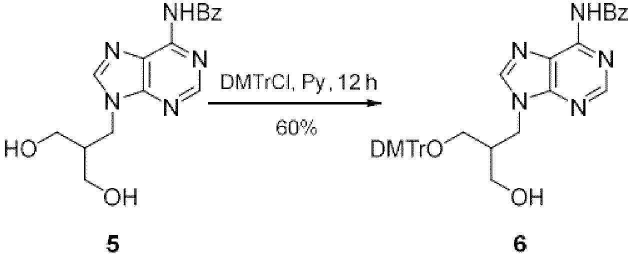

Compound **5** (1.00 g, 3.05 mmol) was dissolved in pyridine (Py, 10 mL), and a solution of 4,4'-dimethoxytrityl chloride (DMTrCl, 1.50 g, 4.58 mmol) in pyridine (5 mL) was added dropwise under an ice bath. After the dropwise addition was complete, the mixture was stirred at room temperature overnight. After the reaction was complete, the reaction mixture was quenched with water and concentrated under reduced pressure to evaporate the solvent. The residue was purified by preparative reversed-phase HPLC (C¹⁸, condition: 5-80% (A: H₂O, B: CH₃CN), flow rate: 70 mL/min) and lyophilized to give compound **6** (1.00 g). MS m/z: C₃₇H₃₆N₅O₅, [M-H]⁺ calculated: 630.26, found: 630.5. The racemate compound **6** was resolved using a chiral column (Daicel CHIRALPAK^{®} IE 250 × 4.6 mm, 5 µm, A: n-hexane, B: ethanol) into 6A(-) (410 mg) and 6B(+) (435 mg).

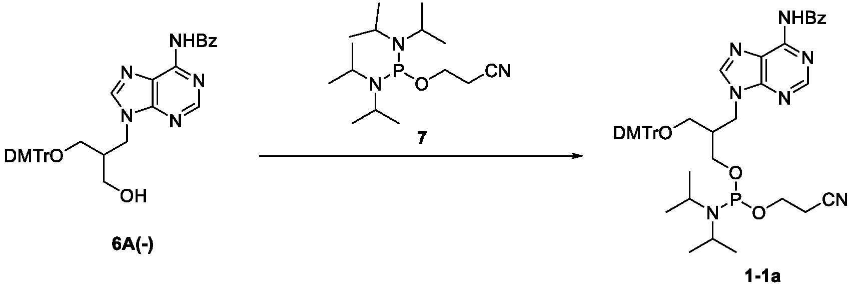

Compound **6A(-)** (200 mg, 0.32 mmol), tetrazole (11 mg, 0.16 mmol), N-methylimidazole (5 mg, 0.06 mmol), and 3A molecular sieve (500 mg) were dissolved in 10 mL of acetonitrile, and compound **7** (144 mg, 0.48 mmol) was added at room temperature. The mixture was stirred at room temperature overnight. After the reaction was complete, the molecular sieve was filtered out, and dichloromethane (30 mL) was added. The mixture was washed with a saturated aqueous sodium bicarbonate solution (10 mL) three times and then with saturated brine (20 mL). The filtrate was concentrated to dryness by rotary evaporation, and the residue was purified by preparative reversed-phase HPLC (C¹⁸, condition: 5-100% (A: water, B: CH₃CN), flow rate: 70 mL/min) and lyophilized to give compound **1-1a** (200 mg). MS m/z: C₄₀H₃₉N₆O₇P, [M-diisopropyl+OH]⁺ calculated: 747.26, found: 747.6. 1H NMR (400 MHz, Acetonitrile-*d₃*) 6 7.56, 7.54 (2s, 1H), 7.36-7.27 (m, 2H), 7.24-7.21 (m, 7H), 6.83-6.80 (m, 4H), 4.12-4.10 (m, 2H), 3.75-3.68 (m, 10H), 3.20-2.80 (m, 2H), 2.68-2.54 (m, 4H), 1.22-1.04 (m, 18H).

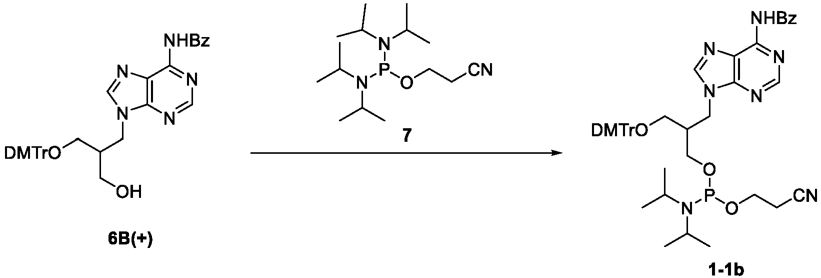

Compound **6B(+)** (200 mg, 0.32 mmol), tetrazole (11 mg, 0.16 mmol), N-methylimidazole (5 mg, 0.06 mmol), and 3A molecular sieve (500 mg) were dissolved in 10 mL of acetonitrile, and compound **7** (144 mg, 0.48 mmol) was added at room temperature. The mixture was stirred at room temperature overnight. After the reaction was complete, the molecular sieve was filtered out, and dichloromethane (30 mL) was added. The mixture was washed with a saturated aqueous sodium bicarbonate solution (10 mL) three times and then with saturated brine (20 mL). The filtrate was concentrated to dryness by rotary evaporation, and the residue was purified by preparative reversed-phase HPLC (C¹⁸, condition: 5-100% (A: water, B: CH₃CN), flow rate: 70 mL/min) and lyophilized to give compound **1-1b** (200 mg). MS m/z: C₄₀H₃₉N6O₇P, [M-diisopropyl+OH]⁺ calculated: 747.26, found: 747.5.

### 1.2. Synthesis of compound 1-6a

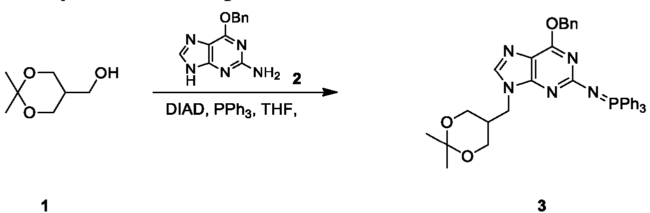

Compound 1 (10 g, 68.404 mmol), compound 2 (15 g, 62.186 mmol), and triphenylphosphine (32.62 g, 124.371 mmol) were dissolved in anhydrous THF (30 mL). DIAD (24.656 mL, 124.371 mmol) was slowly added dropwise at 0 °C. The reaction mixture was left to react at 25 °C for 12 h. LCMS analysis showed the reaction had been complete. The reaction mixture was extracted with ethyl acetate (200 mL) and water (200 mL). The organic phase was dried. The filtrate was concentrated, and the resulting residue was purified using a normal-phase column (DCM/MeOH = 10/1) to give the target product 3 (20 g).

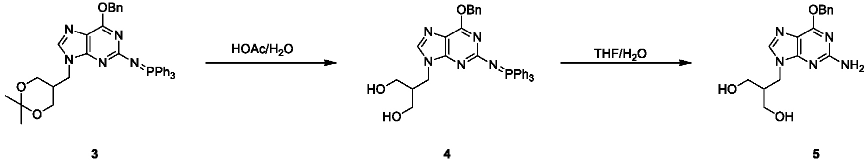

Compound **3** (20 g, 28.585 mmol) was dissolved in acetic acid (24 mL, 426.016 mmol) and H₂O (12 mL), and the solution was stirred at 60 °C for 1 h. The reaction mixture was then concentrated to dryness by rotary evaporation, and THF (12 mL) and H₂O (12 mL) were added. The mixture was stirred at 80 °C for 7 h. LCMS analysis showed the reaction had been complete. The reaction mixture was added to ethyl acetate (200 mL) and water (100 mL) for extraction. Solid sodium carbonate was added to the aqueous phase until a large amount of solid precipitated from the aqueous phase. The solid was collected by filtration and washed with water. The filter cake was dried using an oil pump to give the target compound 5 (9 g).

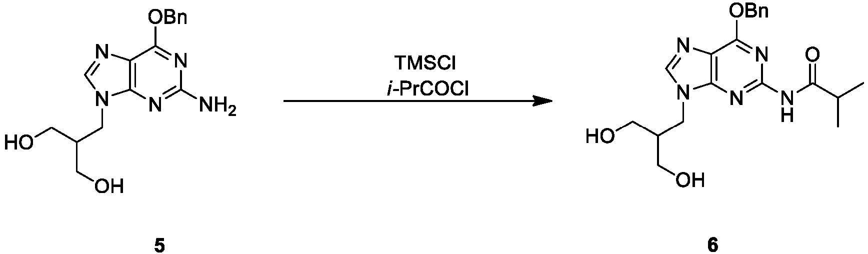

Compound 5 (6.8 g, 18.581 mmol) was dissolved in pyridine (80 mL) in a nitrogen atmosphere, and TMSCl (14.250 mL, 111.489 mmol) was slowly added at 0 °C. The mixture was stirred for 2 h. Isobutyryl chloride (2.044 mL, 19.511 mmol) was then added at 0 °C, and the mixture was stirred at 25 °C for 1 h. LCMS analysis showed the reaction had been complete. The mixture was extracted with dichloromethane (200 mL) and water (200 mL). The organic phase was dried and concentrated to dryness by rotary evaporation, and a sample to be purified was then prepared. The sample was purified using a normal-phase column (elution with DCM:MeOH = 10:1, peak at 4.8%) to give compound 6 as a yellow oil (12 g).

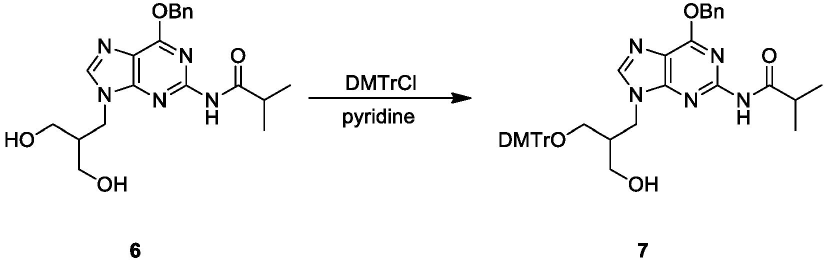

Compound 6 (5.5 g, 12.392 mmol) was dissolved in pyridine (30 mL) in a nitrogen atmosphere. MOLECULAR SIEVE 4A 1/16 (7 g, 12.392 mmol) was added, and solid DMTrCl (5.04 g, 14.870 mmol) was then added in batches at 0 °C. The mixture was left to react at 25 °C for 2 h. TLC analysis (PE:EtOAc = 1:1, Rf = 0.69) showed the reaction had been complete. The reaction mixture and TJN200879-040-P1 were combined and treated together. The reaction mixture was extracted with ethyl acetate (200 mL) and water (200 mL). The organic phase was dried and concentrated to dryness by rotary evaporation, and a sample to be purified was then prepared. The sample was purified using a normal-phase column (elution with PE:EtOAc, peak at 84%) to give compound 7 as a yellow oil (12 g).

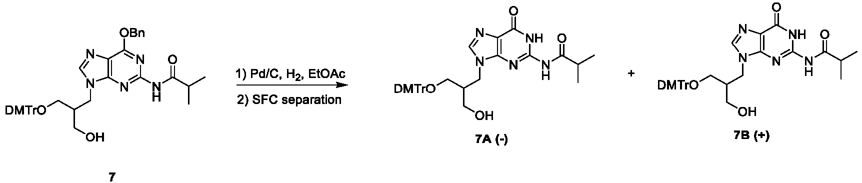

Compound 7 (12 g, 15.389 mmol) was dissolved in EtOAc (140 mL), and wet palladium on carbon Pd/C (7 g, 15.389 mmol) was added. The reaction mixture was left to react at 25 °C in a hydrogen atmosphere (15 Psi) for 2 h. TLC analysis (PE:EtOAc = 0:1, Rf = 0.09) showed the reaction had been complete. The reaction mixture was filtered. After the filter cake was rinsed three times with ethyl acetate (30 mL), the filtrate was collected. After the filtrate was concentrated to dryness by rotary evaporation, 50 mL of dichloromethane and 2 mL of triethylamine were added to prepare a sample to be purified. The sample was purified using a normal-phase column (elution with DCM:MeOH = 10:1, peak at 0.5%) to give 9 g (a yellow foamy solid). The resulting racemic compound was resolved by SFC into the target compound 7A(-) (3.9 g) and the target compound 7B(+) (3.8 g).

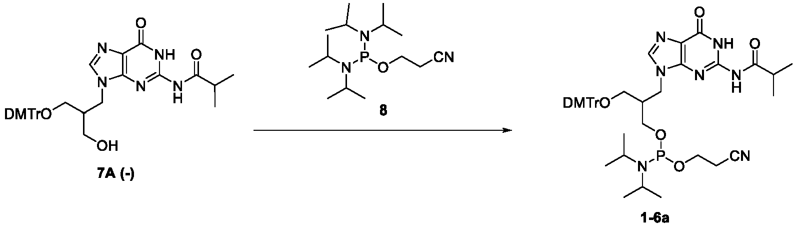

Compound 7A(-) (3.30 g, 5.40 mmol), tetrazole (190 mg, 2.70 mmol), 1-methylimidazole (90 mg, 1.10 mmol), and 3A molecular sieve (500 mg) were dissolved in 30 mL of acetonitrile, and compound 8 (2.50 g, 8.10 mmol) was added at room temperature. The mixture was stirred at room temperature for 2 h. After the reaction was complete, the molecular sieve was filtered out, and DCM (150 mL) was added. The mixture was washed with a saturated aqueous sodium bicarbonate solution (30 mL × 3) and then with saturated brine (30 mL). The filtrate was concentrated to dryness by rotary evaporation, and the residue was purified by preparative reversed-phase HPLC (C18, condition: 5-100% (A: water, B: CH3CN), flow rate: 70 mL/min) and lyophilized to give compound **1-6a** (2.9 g, 66%). MS m/z: C43H55N7O7P [M+H]+, calculated: 812.38, found: 812.5. 1H NMR (400 MHz, Acetonitrile-d3) δ 7.56, 7.54 (2s, 1H), 7.36-7.27 (m, 2H), 7.24-7.21 (m, 7H), 6.83-6.80 (m, 4H), 4.12-4.10 (m, 2H), 3.75-3.68 (m, 10H), 3.20-2.80 (m, 2H), 2.68-2.54 (m, 4H), 1.22-1.04 (m, 18H).

### 1.3. Synthesis of compound 1-7a

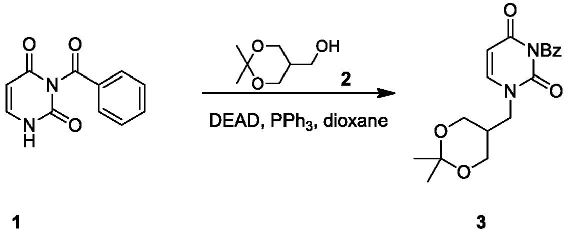

Compound 1 (5 g, 23.1272 mmol), compound 2 (6.76 g, 46.254 mmol), and triphenylphosphine (7.28 g, 27.753 mmol) were dissolved in 30 mL of dioxane in a nitrogen atmosphere, and DEAD (5.502 mL, 27.753 mmol) was slowly added dropwise at 0 °C. After the dropwise addition was complete, the reaction mixture was slowly warmed to 25 °C and left to react for another hour. The reaction mixture was extracted with 100 mL of H₂O and 100 mL of EtOAc. The organic phases were combined, dried, filtered, and concentrated, and a sample to be purified was then prepared. The sample was purified using a normal-phase column (elution with PE:EtOAc =1:1) to give the target product (4 g).

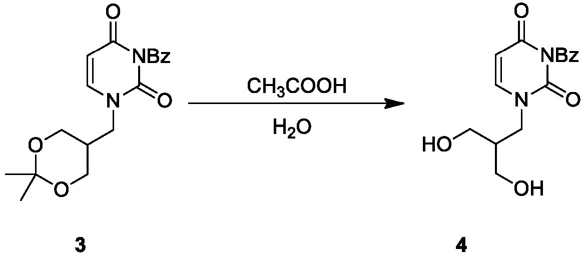

Compound 3 (3.3 g) was dissolved in HOAc (16 mL) and H₂O (4 mL), and the mixture was heated in an oil bath at 60 °C for 0.5 h. The reaction mixture was concentrated to dryness by rotary evaporation, and the resulting residue was purified using a normal-phase column (elution with PE:EtOAc = 0:1) to give the target product 4 (3 g).

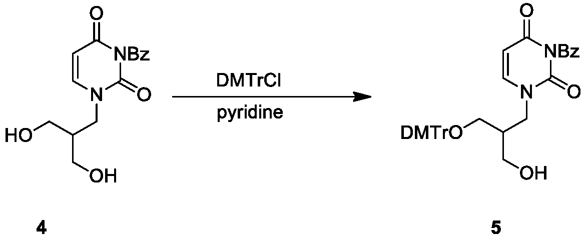

Compound 4 (3 g, 8.873 mmol) was dissolved in 5 mL of pyridine, and a solution of DMTrCl (3.91 g, 11.535 mmol) in 10 mL of pyridine was slowly added dropwise at 0 °C in a nitrogen atmosphere. After the dropwise addition was complete, the mixture was warmed to 25 °C and left to react for another hour. The reaction mixture was extracted with 50 mL of water and 100 mL of ethyl acetate. The aqueous phase was extracted three more times with 100 mL of ethyl acetate. The organic phases were combined, dried, filtered, concentrated, and purified using a normal-phase column (with PE:EtOAc = 2:1) to give the target product 5 (4 g).

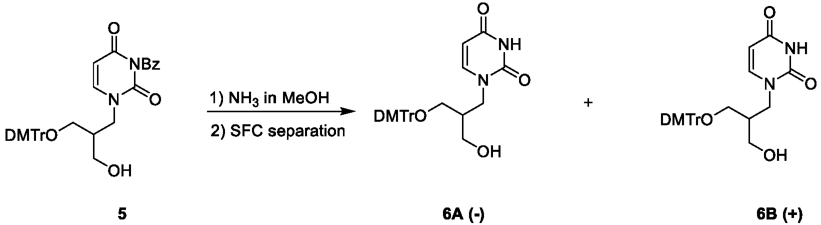

Compound 5 (4 g, 5.769 mmol) was dissolved in methanol (10 mL), and a saturated solution of NH3 in methanol (40 mL) was added. The mixture was left to react at 0 °C for 6 h. The reaction mixture was concentrated to dryness by rotary evaporation, and the residue was purified using a normal-phase column (PE:EtOAc = 0:1) to give a racemic compound (2.4 g). The racemic compound was resolved by SFC into the target product 6A (750 mg, 100% purity) and the target product 6B (400 mg, 99.16% purity).

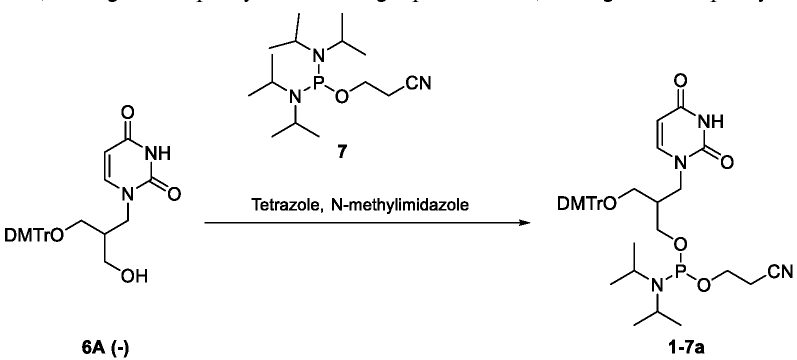

Compound 6A(-) (700 mg, 1.40 mmol), tetrazole (50 mg, 0.70 mmol), 1-methylimidazole (23 mg, 0.28 mmol), and 3A molecular sieve (500 mg) were dissolved in 10 mL of acetonitrile, and compound 7 (630 mg, 2.10 mmol) was added at room temperature. The mixture was stirred at room temperature for 2 h. After the reaction was complete, the molecular sieve was filtered out, and DCM (50 mL) was added. The mixture was washed with a saturated aqueous sodium bicarbonate solution (10 mL × 3) and then with saturated brine (20 mL). The filtrate was concentrated to dryness by rotary evaporation, and the residue was purified by preparative reversed-phase HPLC (C18, condition: 5-100% (A: water, B: CH₃CN), flow rate: 70 mL/min) and lyophilized to give compound **1-7a** (700 mg, 72%). MS m/z: C38H47N4O7PNa [M+Na]+, calculated: 725.32, found: 725.5.

### 1.4. Synthesis of compound 1-8a

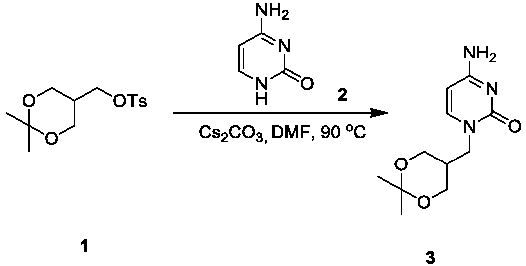

Compound 1 (8.5 g, 76.508 mmol) and compound 2 (30.64 g, 91.809 mmol) were dissolved in DMF (150 mL), and CS2CO3 (29.91 g, 91.809 mmol) was added. The mixture was left to react at 90 °C for 12 h in a nitrogen atmosphere. LCMS analysis showed the reaction had been complete. The reaction mixture was filtered, concentrated to dryness by rotary evaporation using an oil pump, and separated and purified using a normal-phase column (80 g, DCM/MeOH = 10/1 to 5/1) to give the target product 3 (13.5 g, 80% purity).

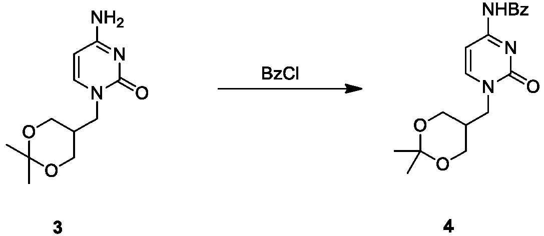

Compound 3 (10.5 g, 35.105 mmol) was dissolved in pyridine (65 mL) and CH₃CN (65 mL), and BzCl (4.894 mL, 42.126 mmol) was added dropwise to the solution. The mixture was left to react at 25 °C for 2 h. LCMS analysis showed most of the starting material had been consumed. The reaction mixture was quenched with H₂O (100 mL) and extracted with EtOAc (100 mL × 3). The organic phase was dried and concentrated to dryness by rotary evaporation, and the residue was separated (combined with TJN200872-101) and purified by column chromatography (80 g, PE/EtOAc = 10/1 to 0/1, DCM/MeOH = 10/1) to give the target product **4** (14 g, 90% purity).

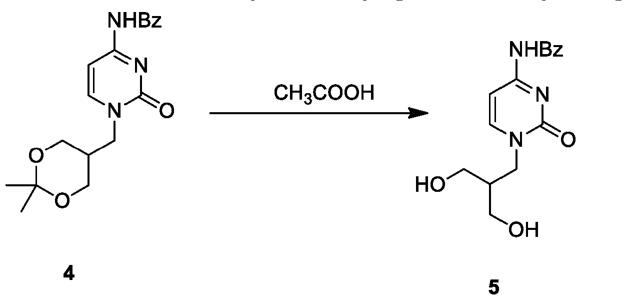

Compound 4 (14 g, 36.694 mmol) was dissolved in HOAc (56 mL, 314.796 mmol) and H₂O (14 mL). The solution was left to react at 60 °C for 2 h, and LCMS analysis showed the reaction had been complete. The reaction mixture was concentrated using an oil pump and separated using a normal-phase column (40 g, DCM/MeOH = 1/0 to 5/1) to give the target product **5** (8.4 g, 90% purity & 2.4 g, 80% purity).

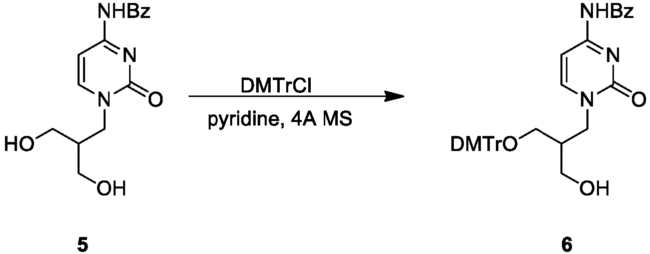

Compound 5 (7.4 g, 21.957 mmol), DMAP (0.54 g, 4.391 mmol), and MOLECULAR SIEVE 4A (11.1 g, 2.967 mmol) were dissolved in pyridine (60 mL). The solution was stirred in an ice bath for 10 min, and DMTrCl (8.93 g, 26.348 mmol) was then added. The mixture was stirred for 1.8 h. LCMS analysis showed about 19% of the starting material remained and about 60% was target MS. The reaction mixture and TJN200872-105&106 were combined and purified together. H₂O (50 mL) was added to the reaction mixture, and extraction was performed with DCM (50 mL × 3). The organic phase was dried and concentrated to dryness by rotary evaporation, and the residue was separated by column chromatography (120 g, PE/(EA:DCM:TEA = 1:1:0.05) = 1/0 to 0/1 to DCM/MeOH = 10/1) to give the target compound 6 (11 g, 89% purity, TJN200872-105&106&107). The starting material was recovered 3.0 g, 70% purity).

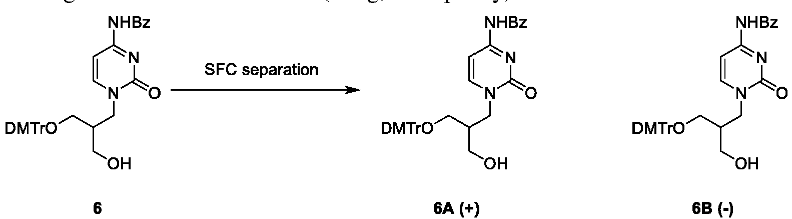

Compound 6 (15 g, 22.041 mmol) was resolved by SFC (DAICEL CHIRALPAK AD (250 mm × 50 mm, 10 µm); 0.1% NH₃ H₂O EtOH, B: 45%-45%; 200 mL/min) to give the target product 6A(+) (5.33 g, 94.29% purity) and the target product 6B(-) (6.14 g, 97.91% purity). 1.0 g of compound 6B(-) was recovered.

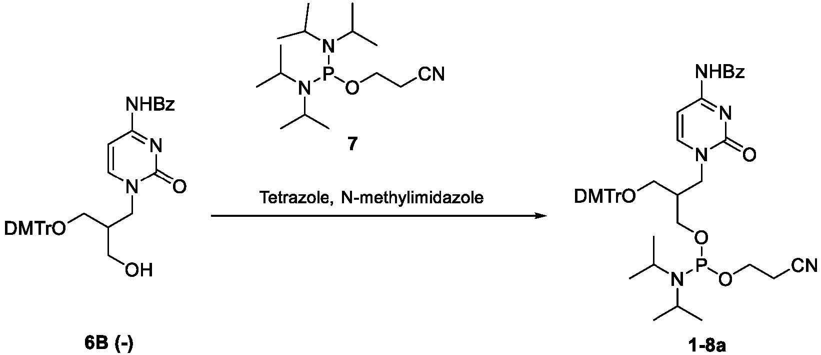

Compound 6B(-) (5.4 g, 8.92 mmol), tetrazole (312 mg, 4.46 mmol), 1-methylimidazole (146 mg, 1.78 mmol), and 3A molecular sieve (500 mg) were dissolved in 40 mL of acetonitrile, and compound 7 (4 g, 13.4 mmol) was added at room temperature. The mixture was stirred at room temperature for 2 h. After the reaction was complete, the molecular sieve was filtered out, and DCM (200 mL) was added. The mixture was washed with a saturated aqueous sodium bicarbonate solution (30 mL × 3) and then with saturated brine (50 mL). The filtrate was concentrated to dryness by rotary evaporation, and the residue was purified by preparative reversed-phase HPLC (C18, condition: 5-100% (A: water, B: CH₃CN), flow rate: 70 mL/min) and lyophilized to give compound **1-8a** (5.8 g, 80%). MS m/z: C45H51N5O7P, [M+H]+, calculated: 804.36, found: 804.4.

### Example 2: Characterization of Different Chemical Modifications

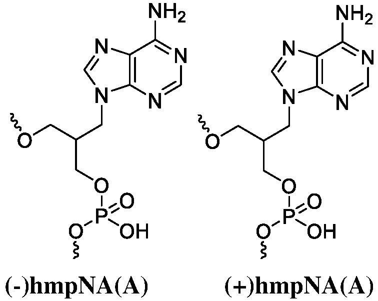

wherein the nucleotide synthesized using 2-hydroxymethyl-1,3-propanediol as the starting material was defined as hmpNA;
(+)hmpNA(A) was obtained by solid-phase synthesis using the nucleoside phosphoramidite monomer **1-1b** of example section 1.1, and its absolute configuration was (S)-hmpNA(A);
(-)hmpNA(A) was obtained by solid-phase synthesis using the nucleoside phosphoramidite monomer 1-1a of example section 1.1, and its absolute configuration was (R)-hmpNA(A).

Similarly, the following structures were obtained by solid-phase synthesis and by changing the base species of hmpNA, and their absolute configurations were determined:
(+)hmpNA(G), with the absolute configuration (S)-hmpNA(G);
(-)hmpNA(G), with the absolute configuration (R)-hmpNA(G);
(+)hmpNA(C), with the absolute configuration (S)-hmpNA(C);
(-)hmpNA(C), with the absolute configuration (R)-hmpNA(C);
(+)hmpNA(U), with the absolute configuration (R)-hmpNA(U); and
(-)hmpNA(U), with the absolute configuration (S)-hmpNA(U).

The absolute configurations (S)-hmpNA(G), (R)-hmpNA(G), (S)-hmpNA(C), (R)-hmpNA(C), (S)-hmpNA(U), and (R)-hmpNA(U) were determined from their intermediates or derivatives by X-Ray diffraction.

The structures of the intermediates or derivatives are:

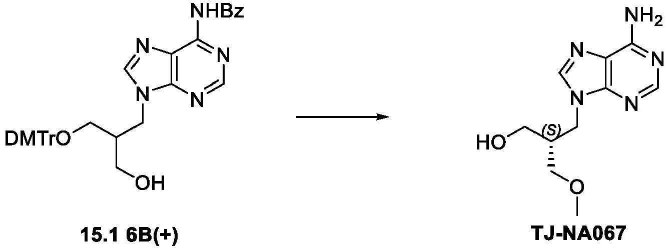

**TJ-NA067:** determined as a colorless massive crystal (0.30 × 0.10 × 0.04 mm3), belonging to the monoclinic crystal system with a P21 space group. Lattice parameter a = 16.0496(5) Å, b = 4.86260(10) Å, c = 16.4686(5) Å, α = 90°, *β* = 118.015(4)°, *γ* = 90°, V = 1134.65(7) Å3, Z = 4. Calculated density Dc = 1.389 g/cm3; the number of electrons in a unit cell *F*(000) = 504.0; linear absorption coefficient of a unit cell *µ* (Cu Kα) = 0.840 mm-1; diffraction experiment temperature T = 150.00(11) K.

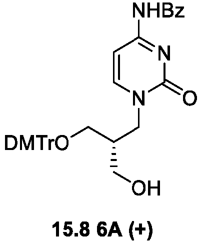

**6A(+):** determined as a colorless massive crystal (0.30 × 0.20 × 0.10 mm3), belonging to the monoclinic crystal system with a P21 space group. Lattice parameter a = 22.6688(7) Å, b = 8.5595(2) Å, c = 23.3578(5) Å, α = 90°, *β* = 113.876(3)°,*γ* = 90°, V = 4144.3(2) Å3, Z = 2. Calculated density Dc = 0.999 g/cm3; the number of electrons in a unit cell *F*(000) = 1318.0; linear absorption coefficient of a unit cell *µ* (Cu Kα) = 0.570 mm-1; diffraction experiment temperature T = 100.01(18) K.

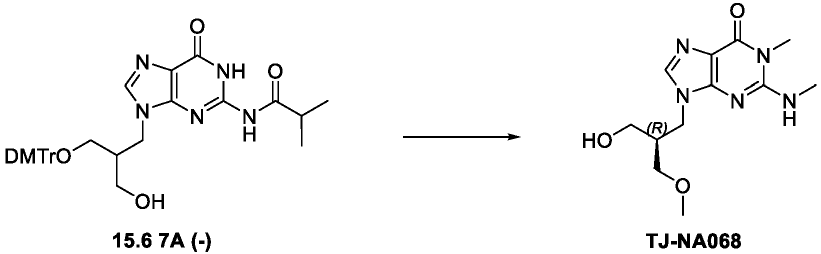

**TJ-NA048:** determined as a colorless acicular crystal (0.30 × 0.04 × 0.04 mm3), belonging to the monoclinic crystal system with a P1 space group. Lattice parameter a = 7.6165(4) Å, b = 11.3423(5) Å, c = 17.3991(8) Å, α = 85.007(4)°, *β* = 88.052(4)°, *γ* = 70.532(4)°, V = 1411.75(12) Å3, Z = 2. Calculated density Dc = 1.366 g/cm3; the number of electrons in a unit cell *F*(000) = 620.0; linear absorption coefficient of a unit cell *µ* (Cu Kα) = 0.856 mm-1; diffraction experiment temperature T = 150.00(13) K.

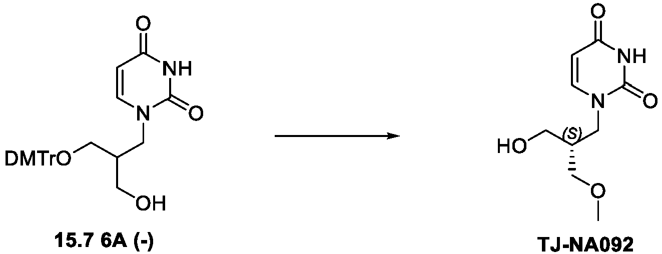

**TJ-NA092:** determined as a colorless prismatic crystal (0.30 × 0.10 × 0.10 mm3), belonging to the triclinic system with a P1 space group. Lattice parameter a = 5.17960(10) Å, b = 8.0667(2) Å, c = 12.4077(2) Å, α = 93.146(2)°, *β* = 101.266(2)°, *γ* = 96.134(2)°, V = 503.993(18) Å3, Z = 2. Calculated density Dc = 1.412 g/cm3; the number of electrons in a unit cell *F*(000) = 228.0; linear absorption coefficient of a unit cell *µ* (Cu Kα) = 0.945 mm-1; diffraction experiment temperature T = 100.00(10) K.

### Example 3. Preparation of NAG0052 and L96

The compounds NAG0024 and NAG0026 were purchased from WuXi AppTec (Tianjin) Co. Ltd. Unless otherwise specified, all reagents used in the following examples were commercially available.

### Synthesis of compound NAG0052

The starting material compound 1 was purchased from Jiangsu Beida Pharmatech Ltd. The synthesis scheme for compound NAG0052 is as follows:

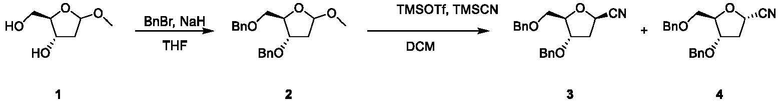

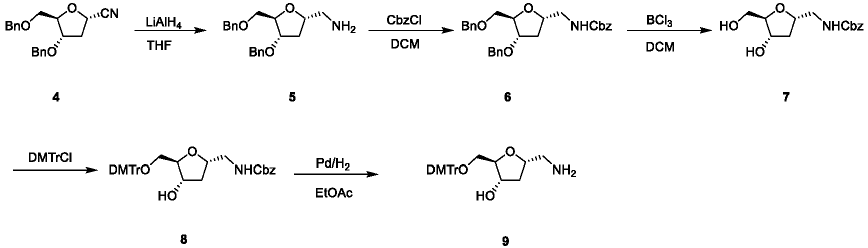

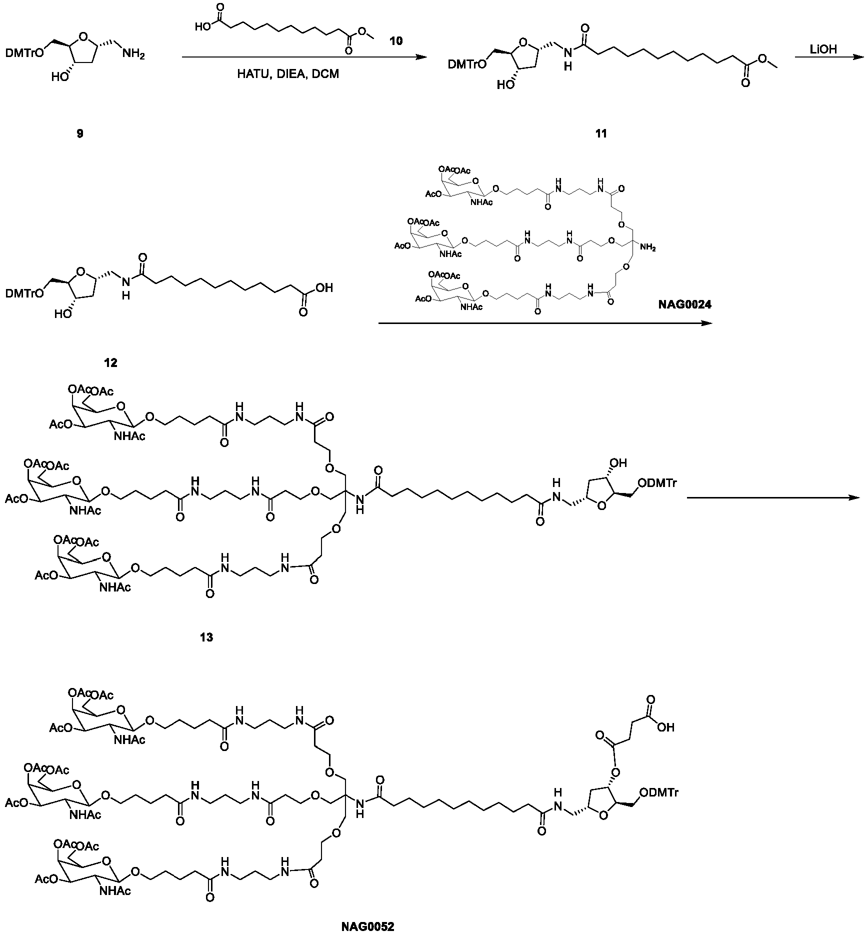

The synthesis and identification of specific intermediates and final products involved in the above scheme are as follows:

### Compound 2

NaH (12.2 g, 304 mmol, purity 60%) was added in batches to a solution of compound **1** (12.3 mL, 101 mmol) in THF (300 mL) at 0 °C in a nitrogen atmosphere. The mixture was stirred at 20 °C for 1 h and then cooled to 0 °C again. Benzyl bromide (36.3 mL, 304 mmol) was then added dropwise to the system, and the mixture was stirred at 20 °C for 12 h. The reaction mixture was quenched with H₂O (100 mL) and then extracted with EtOAc (200 mL × 2). The organic phases were combined, washed with saturated brine (100 mL), dried over Na₂SO₄, filtered, and concentrated, and the resulting residue was separated by silica gel column chromatography to give the target compound **2** (20.0 g, 51.8 mmol, yield 51%).

**LCMS:** t_{R} = 2.615 and 2.820 min in 30-90AB_7 min_220&254_Shimadzu.lcm (Xtimate C18, 3 µm, 2.1 × 30 mm), MS (ESI) m/z = 351.2 [M+Na]⁺.

**¹H** NMR: (400 MHz, CDCl₃) *δ* ppm 7.35-7.12 (m, 10H), 5.06-4.95 (m, 1H), 4.51-4.39 (m, 4H), 4.24-3.87 (m, 2H), 3.50-3.40 (m, 2H), 3.38-3.20 (m, 3H), 2.20-1.91 (m, 2H).

### Compounds 3 and 4

TMSCN (13.5 mL, 101 mmol) was added in one go to a solution of compound **2** (13.0 g, 33.6 mmol) in DCM (300 mL) at 20 °C in a nitrogen atmosphere, and a solution of TMSOTf (9.14 mL, 50.5 mmol) in DCM (30 mL) was then added dropwise. The reaction mixture was stirred at 20 °C for 15 h. After the reaction was complete, the system was quenched with a saturated aqueous NaHCOs solution (80 mL) and extracted with DCM (150 mL × 2). The organic phases were combined, washed with saturated brine (80 mL), dried over Na₂SO₄, filtered, and concentrated. The residue was separated by silica gel column chromatography to give the target compound **3** (3.30 g, 9.18 mmol, yield 27%) and compound **4** (8.50 g, 9.18 mmol, yield 70%) as a pale yellow oily liquid.

### Compound 3

**¹H NMR:** (400 MHz, CDCl₃) *δ* ppm 7.42-7.29 (m, 10H), 4.81 (t, *J* = 7.8 Hz, 1H), 4.65-4.49 (m, 4H), 4.30-4.21 (m, 2H), 3.65-3.57 (m, 1H), 3.57-3.49 (m, 1H), 2.49-2.40 (m, 2H).

### Compound 4

**¹H NMR:** (400 MHz, CDCl₃) *δ* ppm 7.42-7.26 (m, 10H), 4.93-4.87 (m, 1H), 4.65-4.48 (m, 4H), 4.43-4.38 (m, 1H), 4.21-4.17 (m, 1H), 3.79-3.70 (m, 1H), 3.54 (d, *J* = 4.0 Hz, 1H), 2.45-2.37 (m, 2H).

### Compound 5

A solution of compound **4** (3.00 g, 9.28 mmol) in THF (15 mL) was added dropwise to a solution of LiAlH₄ (0.79 g, 20.9 mmol) in THF (15 mL) at 0 °C in a nitrogen atmosphere. After the dropwise addition was complete, the system was left to react at 0 °C for 1 h. TLC monitoring (PE:EtOAc = 3:1) showed the starting material was completely consumed. Sodium sulfate decahydrate was slowly added to the reaction mixture until no more bubbles were produced. The reaction mixture was then filtered, and the filter cake was washed three times with dichloromethane (60 mL). The filtrate was collected and concentrated to dryness by rotary evaporation to give the target compound **5** (3.00 g, yield 90%).

**¹H NMR:** (400 MHz, DMSO-*d*₆) *δ* ppm 7.40-7.14 (m, 10H), 4.54-4.38 (m, 4H), 4.06-3.99 (m, 2H), 3.91 (q, *J =* 6.4 Hz, 1H), 3.48-3.37 (m, 2H), 2.67-2.52 (m, 2H), 2.21-2.18 (m, 1H), 1.77-1.73 (m, 1H).

### Compound 6

Compound **5** (3.00 g, 8.25 mmol) was dissolved in DCM (30 mL) in a nitrogen atmosphere, and TEA (3.44 mL, 24.7 mmol) and CbzCl (1.76 mL, 12.4 mmol) were added. The mixture was left to react at 20 °C for 2 h. LCMS analysis showed the reaction had been complete. The reaction mixture was added to dichloromethane (30 mL) and water (60 mL) for extraction. The organic phase was washed three times with water (60 mL × 3), dried over anhydrous sodium sulfate, and concentrated, and the residue was purified using a normal-phase column (PE:EtOAc = 1:1) to give the target compound **6** (2.5 g, yield 90%).

**LCMS:** t_{R} = 0.810 min in 5-95AB_1min, MS (ESI) m/z = 462.2 [M+H]⁺.

**¹H NMR:** (400 MHz, CDCl₃) *δ* ppm 7.39-7.29 (m, 15H), 5.35 (s, 1H), 5.15-5.01 (m, 2H), 4.72 (d, *J =* 6.0 Hz, 1H), 4.54-4.40 (m, 3H), 4.26 (s, 1H), 4.23-4.18 (m, 1H), 4.11-4.04 (m, 1H), 3.54-3.41 (m, 3H), 3.37-3.25 (m, 1H), 2.34-2.23 (m, 1H), 1.85-1.79 (m, 1H).

### Compound 7

Compound **6** (2.00 g, 3.90 mmol) was dissolved in DCM (5 mL) in a nitrogen atmosphere, and a solution of BCl₃ in THF (1 M, 27.3 mL) was added at -78 °C. The mixture was left to react for 1 h. TLC monitoring (DCM:MeOH = 10:1) showed the starting material was completely consumed. The reaction mixture was quenched with methanol (20 mL) at - 78 °C and concentrated, and the residue was purified using a normal-phase column (DCM:MeOH = 10:1) to give the target compound **7** (2.00 g, yield 60%).

**¹H NMR:** (400 MHz, CD₃OD) *δ* ppm 7.41-7.23 (m, 5H), 5.08 (s, 2H), 4.25-4.07 (m, 2H), 3.85-3.75 (m, 1H), 3.63-3.56 (m, 1H), 3.54-3.48 (m, 1H), 3.30-3.27 (m, 2H), 2.34-2.21 (m, 1H), 1.71-1.64 (m, 1H).

### Compound 8

Compound **7** (0.50 g, 1.78 mmol) was dissolved in pyridine (5 mL) in a nitrogen atmosphere, and 4A molecular sieve (500 mg) and DMTrCl (0.66 mL, 2.13 mmol) were added at 0 °C. Then the mixture was warmed to 20 °C and left to react for 1.5 h. TLC monitoring (PE:EtOAc = 2:1) showed the starting material was completely consumed. The reaction mixture was added to ethyl acetate (60 mL) and water (60 mL) for extraction. The organic phase was washed three times with water (60 mL × 3), then dried over anhydrous sodium sulfate, and concentrated, and the residue was purified using a normal-phase column (PE:EtOAc = 1:1) to give the target compound **8** (800 mg, yield 90%).

**¹H NMR:** (400 MHz, CDCl₃) *δ* ppm 7.44 (d, J = 7.6 Hz, 2H), 7.37-7.23 (m, 11H), 7.22-7.15 (m, 1H), 6.84 (d, J = 8.8 Hz, 4H), 5.09 (s, 2H), 4.31-4.17 (m, 2H), 4.02-3.91 (m, 1H), 3.84-3.73 (m, 6H), 3.33 (s, 1H), 3.28 (s, 1H), 3.19-3.01 (m, 2H), 2.34-2.25 (m, 1H), 1.70-1.62 (m, 1H).

### Compound 9

Compound **8** (800 mg, 1.234 mmol) was dissolved in EtOAc (5 mL), and Pd/C 10% (800 mg, 7.517 mmol) was added. The mixture was left to react in a H₂ atmosphere (15 Psi) at 20 °C for 1 h. LCMS analysis showed the reaction had been complete. The reaction mixture was filtered, and the filter cake was washed three times with dichloromethane (100 mL) and methanol (100 mL). The filtrate was concentrated, and the residue was separated using a reversed-phase column to give compound **9** (300 mg, 54%).

**LCMS:** t_{R} = 2.586 min in 10-80CD_3min MS (ESI) m/z = 450.2 [M+H]⁺.

### Compound 11

Compound **10** (435 mg, 1.780 mmol) was dissolved in DCM (10 mL), and DIEA (0.441 mL, 2.67 mmol) and HATU (677 mg, 1.78 mmol) were added. Compound **9** (400 mg, 0.890 mmol) was then added, and the mixture was left to react at 20 °C for 1 h. TLC monitoring (DCM:MeOH = 10:1) showed the reaction had been complete. The reaction mixture was added to dichloromethane (60 mL) and water (60 mL) for extraction. The organic phase was washed three times with water (60 mL × 3), dried over anhydrous sodium sulfate, and concentrated, and the residue was purified using a normal-phase column (elution with PE:EtOAc = 0:1, product peak at 100%) to give the target compound **11** (600 mg, yield 90%).

**LCMS:** t_{R} = 2.745 min in 30-90CD_3min, MS (ESI) m/z =698.4 [M+Na]⁺.

**¹H NMR:** (400 MHz, CD₃OD) *δ* ppm 7.46-7.38 (m, 2H), 7.35-7.24 (m, 6H), 7.22-7.16 (m, 1H), 6.90-6.78 (m, 4H), 4.29-4.21 (m, 2H), 4.02-3.95 (m, 1H), 3.77 (s, 6H), 3.66-3.62 (m, 3H), 3.41 (s, 1H), 3.18-3.04 (m, 2H), 2.36-2.17 (m, 5H), 1.71-1.50 (m, 5H), 1.39-1.25 (m, 14H).

### Compound 12

Compound **11** (600 mg, 0.799 mmol) was dissolved in THF (3 mL) and H₂O (1 mL), and LiOH.H₂O (134 mg, 3.20 mmol) was added. The mixture was left to react at 20 °C for 12 h. TLC analysis (DCM:MeOH = 10:1) showed the reaction had been complete. The reaction mixture was concentrated to dryness by rotary evaporation. The residue was dissolved in water (5 mL) and methanol (5 mL) and purified using a reversed-phase column (H₂O:CH₃CN = 1:1, peak at around 35%) to give the target compound **12** (460 mg, yield 100%, lithium salt).

**LCMS:** t_{R} = 1.346 min in 10-80CD_3min, MS (ESI) m/z =684.3 [M+Na]⁺.

**HPLC:** t_{R} = 1.879 min in 10-80CD_6min.

**¹H NMR:** (400 MHz, CD₃OD) *δ* ppm 7.47-7.39 (m, 2H), 7.35-7.24 (m, 6H), 7.22-7.15 (m, 1H), 6.91-6.79 (m, 4H), 4.31-4.18 (m, 2H), 4.02-3.95 (m, 1H), 3.78 (s, 6H), 3.44-3.33 (m, 2H), 3.18-3.04 (m, 2H), 2.35-2.27 (m, 1H), 2.24-2.10 (m, 4H), 1.70-1.51 (m, 5H), 1.31-1.23 (m, 12H).

### Compound 13

The compound **NAG0024** (271 mg, 0.151 mmol) was dissolved in anhydrous THF (2 mL) and anhydrous DMF (4 mL) at room temperature in a nitrogen atmosphere, and 3A molecular sieve was added. Compound **12** (100 mg, 0.151 mmol), HOBt (25 mg, 0.181 mmol), DCC (38 mg, 0.181 mmol), and DIEA (39 mg, 0.302 mmol) were then sequentially added. The reaction mixture was left to react at 45 °C for 16 h. After the reaction was complete as shown by LC-MS, the reaction mixture was quenched with water and filtered. The filtrate was concentrated, and the residue was then purified using a C18 reversed-phase column (H₂O/MeCN) to give compound **13** (210 mg, yield 57%).

### Compound NAG0052

Compound **13** (230 mg, 0.094 mmol) was dissolved in pyridine (5 mL) at room temperature, and molecular sieve was added. DMAP (12 mg, 0.283 mmol) and succinic anhydride (28 mg, 0.283 mmol) were added. The reaction mixture was stirred at 50 °C for 16 h in a nitrogen atmosphere. LCMS analysis showed the reaction had been complete. The reaction mixture was filtered and concentrated to dryness by rotary evaporation. After purification on a C18 reversed-phase column, secondary purification was performed by preparative HPLC to give the target compound NAG0052 (123 mg, 0.048 mmol, yield 51%).

MS (ESI) m/z = 2535.3 [M-1]⁻. Calculated: 2536.2.

¹H NMR (400 MHz, Acetonitrile-*d*₃) δ 7.48-7.43 (m, 2H), 7.37-7.12 (m, 11H), 7.00-6.85 (m, 10H), 6.66 (s, 1H), 5.31 (dd, *J =* 3.4, 1.1 Hz, 3H), 5.20-5.13 (m, 1H), 5.05 (dd, *J =* 11.3, 3.4 Hz, 3H), 4.56 (d, *J =* 8.5 Hz, 3H), 4.30 (dd, *J =* 7.7, 5.3 Hz, 1H), 4.18-3.93 (m, 14H), 3.79 (s, 10H), 3.65 (q, *J =* 4.7, 3.6 Hz, 13H), 3.56-3.07 (m, 24H), 2.56 (s, 6H), 2.37 (t, *J =* 5.8 Hz, 10H), 2.17 (t, *J =* 7.5 Hz, 9H), 2.02-1.96 (m, 20H), 1.88 (s, 8H), 1.82-1.73 (m, 2H), 1.60 (dt, *J =* 15.0, 7.3 Hz, 16H), 1.27 (s, 13H).

### Synthesis of L96

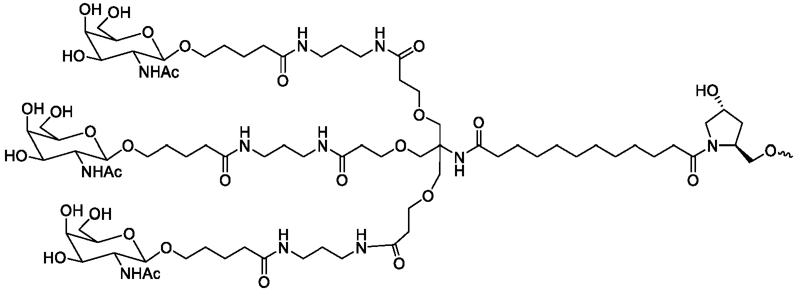

L96, represented by the formula above, was prepared by following the method described in the patent application WO2014025805A1.

### Example 4: Synthesis of dsRNAs

### 1. In-house preparation of resin with support

The carboxylic acid group-containing compound **NAG0052** (157 mg, 0.062 mmol) was dissolved in anhydrous DMF (3 mL). After the substrate was completely dissolved, anhydrous acetonitrile (4 mL), DIEA (0.03 mL, 0.154 mmol, 2.5 eq), and HBTU (35 mg, 0.093 mmol, 1.5 eq) were sequentially added. After the reaction mixture was mixed well, macroporous aminomethyl resin (476 mg, blank loading 0.41 mmol/g, target loading 0.1 mmol/g) was added. The reaction mixture was shaken overnight on a shaker (temperature: 25 °C; rotational speed: 200 rpm). The reaction mixture was filtered, and the filter cake was washed with DCM and then with anhydrous acetonitrile. The solid was collected and dried overnight under vacuum.

The solid from the previous step was dispersed in anhydrous acetonitrile (5 mL), and pyridine (0.18 mL), DMAP (3 mg), NMI (0.12 mL), and CapB1 (2.68 mL) were sequentially added. The reaction mixture was shaken on a shaker (temperature: 25 °C; rotational speed: 200 rpm) for 2 h. The reaction mixture was filtered, and the filter cake was washed with anhydrous acetonitrile. The solid was collected and dried overnight under vacuum to give a resin with a support. The loading measured 0.1 mmol/g.

2. For **NAG0052** that had been attached to the resin, the resin was used as a start, and nucleoside monomers were linked one by one in the 3'-5' direction in the order in which nucleotides were arranged. The linking of each nucleoside monomer involves four reactions: deprotection, coupling, capping, and oxidation or sulfurization. The procedure is conventional in the art.

The compound NAG0052 was attached to the sequence through solid-phase synthesis, and after aminolysis, part of the functional group of the structure of NAG0052 was removed, thus forming NAG0052'. The prepared dsRNAs had the sense and antisense strands shown in Table 1 and Table 2.

**Table 1. A list of dsRNAs**

| dsRNA No. | Sense strand No. | Antisense strand No. |
|---|---|---|
| TRD002218 | TJR4373-SS | TJR0414-AS |
| TRD007205 | TJR013485S | TJR0414-AS |

**Table 2. The nucleic acid sequences of the sense and antisense strands**

| | | SEQ ID NO | **Sequence direction 5'-3'** |
|---|---|---|---|
| **Sense strand** | TJR4373-SS | 25 | CmsAmsGm UmGfUm UfCfUf UmGmCm UmCmUm AmUmAm Am-**L96** |
| | TJR013485S | 26 | CmsAmsGm UmGfUm UfCfUf UmGmCm UmCmUm AmUmAms Ams-**NAG0052**' |
| **Antisense strand** | TJR0414-AS | 27 | UmsUfsAm UmAmGf AmGmCm AmAmGm AmAfCm AfCmUm GmsUmsUm |

In the above table, the uppercase letters G, Å, C, and U represent nucleotides containing guanine, adenine, cytosine, and uracil, respectively, the lowercase letter m represents a 2'-methoxy modification, the lowercase letter f represents a 2'-fluoro modification, and the lowercase letter s indicates that the two nucleotides adjacent to the letter s are linked by a phosphorothioate diester group; the same applies hereinafter.

### Example 5: In Vivo Inhibition of Target Gene mRNA Expression Level by dsRNAs

In this experiment, the efficiency of the dsRNAs of the present disclosure, which are conjugated differently, in inhibiting the target gene mRNA expression level in vivo was investigated.

Male C57BU6 mice, 6-8 weeks old, were randomly divided into groups of 6, with 3 mice per time point. TRD007205, the reference positive compound TRD002218, and PBS were administered to these groups of mice.

All the animals were dosed once by subcutaneous injection based on their body weight. The dsRNAs were administered at a dose of 1 mg/kg (calculated based on nucleotides without a ligand), with a volume of 5 mL/kg. The mice were sacrificed 7 days and 28 days after administration, and their livers were collected and stored with RNA later (Sigma Aldrich). Then, the liver tissue was homogenized using a tissue homogenizer, and the total RNA was extracted from the liver tissue using a tissue RNA extraction kit (FireGen Biomedicals, FG0412) by following the procedure described in the instructions. The total RNA was reverse-transcribed into cDNA, and the TTR mRNA (transthyretin mRNA) expression level in liver tissue was measured by real-time fluorescence quantitative PCR. In the fluorescence quantitative PCR method, the glyceraldehyde 3-phosphate dehydrogenase (GAPDH) gene was used as an internal reference gene, and the TTR and GAPDH mRNA expression levels were measured using Taqman probe primers for TTR and GAPDH, respectively.

**Table 3. The groups of mice for the in vivo experiment**

| No. | Dose | mRNA quantification | Number of animals | Note |
|---|---|---|---|---|
| PBS | - | D7, 28 | 6 | 3 mice per time point |
| TRD002218 | 1mpk s.c. | D7, 28 | 6 | 3 mice per time point |
| TRD007205 | 1mpk s.c. | D7, 28 | 6 | 3 mice per time point |

**Table 4. The sequences of detection primers are as follows:**

| Primer name | SEQ ID NO | Forward primer |
|---|---|---|
| mTTR-F | 28 | GGGAAGACCGCGGAGTCT |
| mTTR-R | 29 | CAGTTCTACTCTGTACACTCCTTCTACAAA |
| mTTR-P | 30 | 5'6-FAM-CTGCACGGGCTCACCACAGATGA-3'BHQ1 |
| mGAPDH-F | 31 | CGGCAAATTCAACGGCACAG |
| mGAPDH-R | 32 | CCACGACATACTCAGCACCG |
| mGAPDH-P | 33 | 5'TET-ACCATCTTCCAGGAGCGAGACCCCACT-3'BHQ2 |

The TTR mRNA expression level was calculated according to the equation below: TTR mRNA expression level = [(TTR mRNA expression level of text group/GAPDH mRNA expression level of test group)/(TTR mRNA expression level of control group/GAPDH mRNA expression level of control groups)] × 100%. TTR mRNA expression level = [(TTR mRNA expression level of test group/GAPDH mRNA expression level of test group)/(TTR mRNA expression level of control group/GAPDH mRNA expression level of control group)] × 100%.

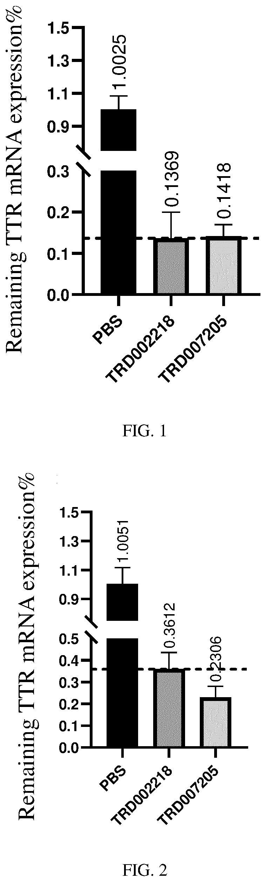

The efficiency of the dsRNAs of the present disclosure, which are conjugated with different structures, in inhibiting the target gene mRNA expression level in vivo 7 days and 28 days after administration are shown in FIG. 1 and FIG. 2, respectively. As can be seen from the results in FIG. 1, TRD007205 well inhibited TTR mRNA expression 7 days after administration. As can be seen from FIG. 2, 28 days after administration, TRD007205 inhibited the target gene mRNA expression level better than TRD002218.

### Example 6: Synthesis of dsRNAs

1. In-house preparation of resin with support.
2. A resin with **NAG0052** was used as a start, and nucleoside monomers were linked one by one in the 3'-5' direction in the order in which nucleotides were arranged. The linking of each nucleoside monomer involves four reactions: deprotection, coupling, capping, and oxidation or sulfurization.

The prepared dsRNAs had the sense and antisense strands shown in Table 5 and Table 6.

**Table 5. A list of dsRNAs**

| dsRNA No. | Sense strand No. | Antisense strand No. |
|---|---|---|
| TRD008069 | TJR015002S | TJR014946A |
| TRD008042 | TJR015002S | TJR014947A |
| TRD008070 | TJR015002S | TJR014948A |
| TRD008071 | TJR015002S | TJR014949A |
| TRD008072 | TJR015002S | TJR014950A |
| TRD008073 | TJR015002S | TJR014951A |
| TRD008074 | TJR015002S | TJR014952A |
| TRD008043 | TJR015002S | TJR014953A |
| TRD008075 | TJR015002S | TJR014954A |
| TRD008076 | TJR015002S | TJR014956A |
| TJR100383 | S0262 | A0252 |
| TJR100384 | S0263 | A0252 |
| TJR100385 | S0262 | A0253 |
| TJR100386 | S0263 | A0253 |
| TRD007972-1 | TJR014883S-1 | TJR013122A |

**Table 6. The sense and antisense strands of dsRNAs**

| | Single strand No. | SEQ ID NO | Sequence direction 5'-3' |
|---|---|---|---|
| Sense strand | TJR015002S-1 | 3 | UmsAmsUmUmCmUmCfAfGfUmGmCmUmC mUmCmCmUmAm |
| | TJR015002S | 4 | UmsAmsUmUmCmUmCfAfGfUmGmCmUmC mUmCmCmUmAm-NAG0052' |
| | S0262 | 15 | UmsAmsUmUmCfUmCfAfGfUmGmCmUmCm UmCmCmUmAm-L10 |
| | S0263 | 16 | UmsAmsUmUmCfUmCfAfGfUmGmCmUmCm |
| | | | UmCmCmUmAm-L96 |
| | TJR014883S-1 | 17 | UmsAmsUmUmCmUmCfAfGfUmGmCmUmC mUmCmCmUmAm-NAG0052' |
| | TJR014946A | 5 | UmsAfsGmGfAmGf(-)hmpNA(A)GmCmAfCm UfGmAfGmAfAmUmAmsCmsUm |
| | TJR014947A | 6 | UmsAfsGmGfAmGf(-)hmpNA(A)GmCmAfCm UmGmAfGmAfAmUfAmsCmsUm |
| | TJR014948A | 7 | UmsAfsGmGfAmGf(-)hmpNA(A)GmCmAmCm UfGmAfGmAfAmUfAmsCmsUm |
| | TJR014949A | 8 | UmsAfsGmGmAmGf(-)hmpNA(A)GmCmAfCm UfGmAfGmAfAmUfAmsCmsUm |
| | TJR014950A | 9 | UmsAfsGmGmAmGf(-)hmpNA(A)GmCmAmC mUmGmAfGmAfAmUmAmsCmsUm |
| | TJR014951A | 10 | UmsAfsGmGfAmGf(-)hmpNA(A)GmCmAmCm UmGmAfGmAfAmUmAmsCmsUm |
| Antisense strand | TJR014952A | 11 | UmsAfsGmGmAmGf(-)hmpNA(A)GmCmAfCm UmGmAfGmAfAmUmAmsCmsUm |
| | TJR014953A | 12 | UmsAfsGmGmAmGf(-)hmpNA(A)GmCmAmC mUfGmAfGmAfAmUmAmsCmsUm |
| | TJR014954A | 13 | UmsAfsGmGmAmGm(-)hmpNA(A)GfCfAmCm UmGmAfGmAfAmUmAmsCmsUm |
| | TJR014956A | 14 | UmsAfsGmGmAmGm(-)hmpNA(A)GmCmAm CmUmGmAfGmAmAmUmAmsCmsUm |
| | A0252 | is | UmsAfsGmGmAmGfAmGfCfAmCmUmGmAf GmAfAmUmAmsCmsUm |
| | A0253 | 19 | UmsAfsGmGmAmGfA(GNA)GfCfAmCmUmG mAfGmAfAmUmAmsCmsUm |
| | TJR013122A | 20 | UmsAfsGmGfAmGf(-)hmpNA(A)GmCmAfCm UfGmAfGmAfAmUfAmsCmsUm |

wherein,
(-)hmpNA(A) represents 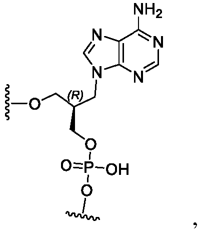 and (-)hmpNA(G) represents 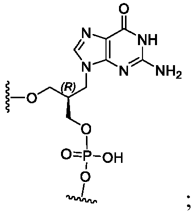
the structure of NAG0052' is: 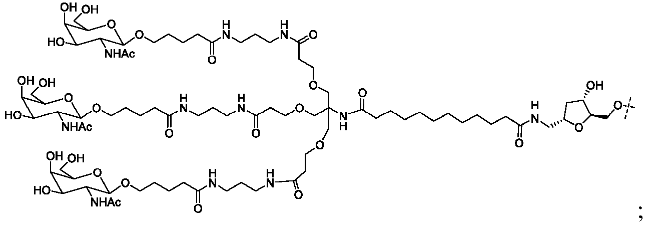
the structure of A(GNA) is: 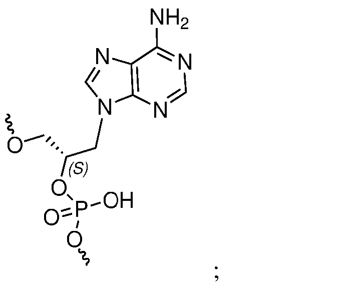
the structure of L10 is: 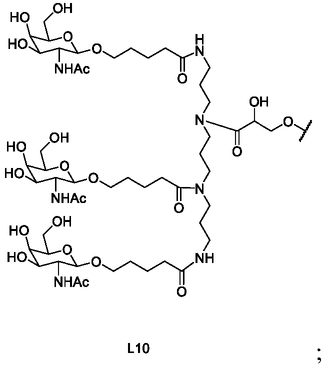
the structure of L96 is

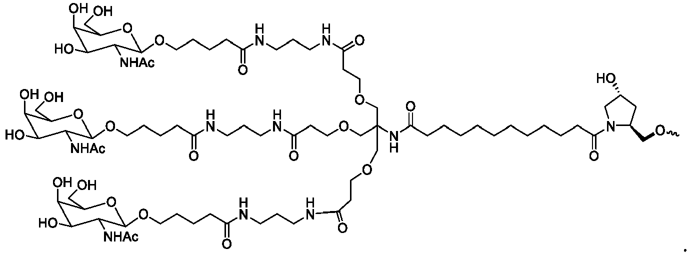

**Table 7. Naked sequences corresponding to the sense and antisense strands of dsRNAs**

| | Shi' shi single strand numbers corresponding to naked sequences | SEQ ID NO | Naked sequence, **sequence direction 5'-3**' |
|---|---|---|---|
| Sense strand | TJR015002S, TJR015002S-1, S0262, S0263, TJR014883S-1 | 1 | UAUUCUCAGUG CUCUCCUA |
| Antisense strand | TJR014946A, TJR014947A, TJR014948A, TJR014949A, TJR014950A, TJR014951A, TJR014952A, TJR014953A, TJR014954A, TJR014955A, TJR014956A, A0252, A0253, TJR013122A | 2 | UAGGAGAGCAC UGAGAAUACU |

### Example 7. Inhibition of Human APOC3 in Primary Human Hepatocytes (PHHs) - 7-Concentration-Point Inhibitory Activity

Primary human hepatocyte (PHH) activity screening was performed on dsRNAs in primary human hepatocytes (PHHs) using 7 concentration gradients. The initial final concentration for transfection of each dsRNA sample was 10 nM, and 5-fold serial dilution was carried out to obtain 7 concentration points.

The PHHs were cryopreserved in liquid nitrogen. 24 h prior to transfection, the PHHs were thawed and then seeded into a 96-well plate at a density of 3 × 10⁴ cells per well, with each well containing 80 µL of medium. The cells were transfected with the dsRNAs using Lipofectamine RNAi MAX (ThermoFisher, 13778150) by following the instruction manual of the product, with the final gradient concentrations of the dsRNAs for transfection being 10 nM, 2 nM, 0.4 nM, 0.08 nM, 0.016 nM, 0.0032 nM, and 0.00064 nM. 24 h after treatment, the total cellular RNA was extracted using a high-throughput cellular RNA extraction kit, and RNA reverse transcription and quantitative real-time PCR detection were carried out. The mRNA level of human APOC3 was measured and corrected based on the GAPDH internal reference gene level.

In the real-time quantitative PCR detection, the probe Q-PCR detection was carried out, and information about primers is shown in Table 8.

**Table 8. Taqman primers**

| Primer name | SEQ ID NO | Primer sequence |
|---|---|---|
| hAPOC3-PF | 32 | TGCCTCCCTTCTCAGCTTCA |
| hAPOC3-PR | 33 | GGGAACTGAAGCCATCGGTC |
| hAPOC3-P | 34 | ATGAAGCACGCCACCAAGACCGCCA |
| hGAPDH-PF1-MGB | 35 | GACCCCTTCATTGACCTCAACTAC |
| hGAPDH-PR1-MGB | 36 | TTGACGGTGCCATGGAATTT |
| hGAPDH-P1-MGB | 37 | TTACATGTTCCAATATGATTCC |

### Result analysis method

After the Q-PCR detection was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-△△Ct}, where △△Ct = [(target gene of Ct experimental group - internal reference of Ct experimental group) - (target gene of Ct control group - internal reference of Ct control group)]. Inhibition rate (%) = (1 - remaining level of target gene expression) × 100%.

The results are expressed relative to the remaining percentage of human APOC3 mRNA expression in cells treated with the control dsRNA. The inhibition rate IC₅₀ results are shown in Table 9. The experimental results in Table 9 show that TRD008043 is superior to the rest of the groups.

**Table 9. The multi-dose inhibitory activity of siRNAs in PHH cells**

| Compound No. | Remaining percentage of target gene mRNA (PHH) expression (mean) | | | | | | | IC₅₀ values (nM) |
|---|---|---|---|---|---|---|---|---|
| | 10 nM | 2 nM | 0.4 nM | 0.08 nM | 0.016 nM | 0.0032 nM | 0.00064 nM | |
| TRD008069 | 24.92% | 38.33% | 68.39% | 73.47% | 83.11% | 91.01 % | 92.01% | 1.0798 |
| TRD008042 | 26.53% | 37.89% | 61.49% | 72.17% | 86.28% | 89.10% | 86.96% | 0.7943 |
| TRD008070 | 23.78% | 36.48% | 56.66% | 70.28% | 84.23% | 86.48% | 91.03% | 0.6150 |
| TRD008071 | 45.02% | 31.96% | 49.62% | 68.05% | 80.10% | 84.87% | 99.95% | 0.3525 |
| TRD008072 | 19.87% | 29.88% | 44.58% | 70.06% | 86.87% | 86.88% | 79.12% | 0.3005 |
| TRD008073 | 19.79% | 28.22% | 49.97% | 66.67% | 87.28% | 94.27% | 93.77% | 0.3286 |
| TRD008074 | 22.46% | 31.73% | 56.32% | 75.03% | 94.54% | 96.84% | 95.29% | 0.5109 |
| TRD008043 | 11.76% | 24.58% | 42.58% | 80.06% | 137.04% | 142.87% | 139.52% | 0.2326 |
| TRD008075 | 22.78% | 37.10% | 53.89% | 71.67% | 89.33% | 85.35% | 90.65% | 0.5623 |
| TRD008076 | 20.26% | 40.18% | 53.04% | 73.64% | 87.25% | 89.06% | 98.43% | 0.6770 |

The difference in activity between TRD007972-1 and TJR100385 or TJR100386 was examined using the same method, and the inhibition rate IC₅₀ results are shown in Table 10.

The results in Table 10 show that TRD007972-1 exhibited a high level of on-target inhibitory activity against the APOC3 gene in PHH cells, better than TJR100385 and TJR100386.

**Table 10. The multi-dose inhibitory activity of dsRNAs in PHH cells**

| Compound No. | Remaining percentage of target gene mRNA (PHH) expression (mean) | | | | | | | IC₅₀ values (nM) |
|---|---|---|---|---|---|---|---|---|
| | 10 nM | 2 nM | 0.4 nM | 0.08 nM | 0.016 nM | 0.0032 nM | 0.00064 nM | |
| TRD007972-1 | 9.56% | 13.93% | 16.38% | 30.71% | 59.05% | 90.95% | 100.24% | 0.0257 |
| TJR100385 | 11.28% | 14.67% | 24.83% | 40.91% | 72.83% | 94.84% | 100.61% | 0.0525 |
| TJR100386 | 11.37% | 16.44% | 25.55% | 44.03% | 79.38% | 98.46% | 105.35% | 0.0652 |

### Example 8: psiCHECK Antisense Strand (AS strand) Off-Target Level Validation of dsRNAs

In vitro molecular level simulation on-target and off-target level screening was performed on dsRNAs in HEK-293A cells using 9 concentration gradients. The results show that TRD008043 has low off-target activity while having high activity.

Off-target sequences corresponding to the dsRNA sequences were constructed and inserted into psiCHECK-2 plasmids. The plasmids contained the renilla luciferase gene and the firefly luciferase gene. The plasmids were dual reporter gene systems. The target sequence of dsRNA was inserted into the 3' UTR region of the renilla luciferase gene. The activity of dsRNA for the target sequence was reflected by the measurements of renilla luciferase expression corrected with firefly luciferase. The measurements used Dual-Luciferase Reporter Assay System (Promega, E2940).

The rules for constructing GSSM target plasmids corresponding to the dsRNA sequences are as follows:
For the antisense strands of the dsRNAs, off-target plasmids (GSSM) that are completely complementary to positions 1-8 of the 5' end of the antisense strands and the bases of which at the other positions do not match at all were constructed. The corresponding rules for base mismatches were that A pairs with C and G pairs with T.

HEK293A cells were cultured at 37 °C with 5% CO₂ in a DMEM high glucose medium containing 10% fetal bovine serum. 24 h prior to transfection, the HEK293A cells were inoculated onto a 96-well plate at a density of 8 × 10³ cells per well, with each well containing 100 µL of medium.

The cells were co-transfected with dsRNA and the corresponding plasmid using Lipofectamine2000 (ThermoFisher, 11668019) according to the instructions. 0.2 µL of Lipofectamine2000 was used for each well, and the amount of plasmid for transfection was 20 ng per well. For the off-target sequence plasmids, a total of 9 concentration points were set up for the dsRNAs, with the highest concentration point final concentration being 20 nM. A 3-fold serial dilution was performed, resulting in 20 nM, 6.6667 nM, 2.2222 nM, 0.7407 nM, 0.2469 nM, 0.0823 nM, 0.0274 nM, 0.0091 nM, and 0.0030 nM. 24 h after transfection, the off-target levels were determined using Dual-Luciferase Reporter Assay System (Promega, E2940).

The results shown in Table 11 indicate that TRD008043 involves no off-target risk compared to TJR100383 and TJR100384.

**Table 11. The results of the psiCHECK off-target activity screening of the seed regions of the AS strands**

| Remaining percentage of target gene mRNA (GSSM) expression (mean) | | | | | |
|---|---|---|---|---|---|
| Double strand code | 20 nM | 6.6667 nM | 2.2222 nM | 0.7407 nM | 0.2469 nM |
| TJR100383 | 18.76% | 21.50% | 30.04% | 59.86% | 90.79% |
| TJR100384 | 20.50% | 27.59% | 34.24% | 64.27% | 102.36% |
| TRD008043 | 87.92% | 87.25% | 95.16% | 94.00% | 86.86% |

| Double strand code | 0.0823 nM | 0.0274 nM | 0.0091 nM | 0.0030 nM | IC50(nM) |
|---|---|---|---|---|---|
| TJR100383 | 100.95% | 103.06% | 89.70% | 98.64% | 1.0129 |
| TJR100384 | 98.38% | 104.87% | 104.37% | 108.00% | 1.0937 |
| TRD008043 | 93.45% | 89.65% | 89.41% | 89.24% | >20 |

### Example 9: Pharmacodynamic Study on Tg Mice

The mice used were B6;CBA-Tg(APOC3)3707Bres/J, strain: #006907 (Jackson Laboratory, 11-12 w), hereinafter referred to as APOC3 Tg mice, with 9 males and 9 females. Upon arrival, the animals were housed (N = 2/cage) and acclimatized for 10 days. Body weight was measured once a week.

Fourteen days before administration plasma samples of at least 40 µL (~90 µL of blood) were collected for the first baseline measurement of HDL, LDL, TG, TC, and ApoC3 in plasma during the acclimatization period. The second baseline measurement was conducted 7 days before administration. Plasma samples of at least 40 µL (~90 µL of blood) were collected for the second measurement of TG, TC, HDL-C, and ApoC3 in plasma. Seventeen animals were selected and divided into 3 groups based on their TG and ApoC3 (apolipoprotein C3) levels. The third baseline measurement was conducted on the morning of the administration day. Plasma samples of at least 40 µL (~90 µL of blood) were collected for the measurement of TG, TC, HDL-C, and ApoC3, and this set of data served as the time-0 (day 0) baseline.

In preparing a test sample, it was dissolved in normal saline (pH = 7.4), 5 µL of each solution was taken and diluted 10-fold, and the concentration was measured 3 times/dilution using NanoDrop. The final concentration of the administration formulation was adjusted based on the NanoDrop results. Normal saline and the test samples were all administered once by subcutaneous injection; the injection volume was calculated based on the body weight before administration.

After administration, body weight was measured once a week, and plasma samples were collected on days 7, 14, 21, 28, 35, and 42 (the animals were fasted overnight before blood collection) for the measurement of ApoC3, TG, TC, and HDL-C in plasma. Blood samples were centrifuged within half an hour of collection (centrifuge conditions: 4 °C, 3000 g, 5 min). The plasma was collected into 2 polypropylene tubes, with the volumes being 30 µL (exact volume) and 10-15 µL (the rest). The samples were flash-frozen with dry ice and stored in a -80 °C freezer before analysis. The results are shown in Tables 13-15.

**Table 12. Experimental groups and administration regimens**

| Group | Test substance | Dose | Route of administration | Blood collection point (day) | Number of animals |
|---|---|---|---|---|---|
| | | (mg/kg) | | | |
| 1 | Normal saline | / | Subcutaneous injection | -14, -7, 0, 7, 14, 21, 28, 35, 42 | 3 males + 3 females |
| 2 | TRD008043 | 3 | Subcutaneous injection | -14, -7, 0, 7, 14, 21, 28, 35,42 | 3 males + 3 females |
| 3 | TRD007972-1 | 3 | Subcutaneous injection | -14, -7, 0, 7, 14, 21, 28, 35,42 | 2 males + 3 females |

**Table 13. The percentage of APOC3 in plasma compared to the day 0 baseline after a single administration of the test substances to APOC3 Tg mice (x̅ ± SE)**

| Group | Human APOC3 (percentage compared to day 0) | | | | | |
|---|---|---|---|---|---|---|
| | Day 7 | Day 14 | Day 21 | Day 28 | Day 35 | Day 42 |
| Normal saline | 95.40±19.60 | 104.68±18.48 | 94.78±17.40 | 103.64±17.70 | 84.77±13.31 | 96.80±12.04 |
| TRD008043 3mg/kg | 9.07±2.32*** | 11.90±3.27*** | 11.32±2.93*** | 17.24±4.40*** | 20.58±5.14** | 38.18±14.08* |
| TRD007972-1 3mg/kg | 14.90±1.94*** | 37.12±10.12*** | 37.60±8.59** | 60.99±10.39 | 62.17±9.32 | 82.45±15.83 |

| | | | | | | |
|---|---|---|---|---|---|---|
| ****p* < 0.001, ***p* < 0.01, **p* < 0.05, all compared to the normal saline group. One-way ANOVA Dunnett's test. | | | | | | |

The results in Table 13 show that: after the single administration of the test samples to APOC3 Tg mice, the percentage of APOC3 in mouse plasma compared to the day 0 baseline in the TRD008043 group was significantly lower than that in the normal saline group at days 7, 14, 21, 28, 35, and 42. In contrast, the percentage of APOC3 in mouse plasma compared to the day 0 baseline in the TRD007972-1 group was not significantly different from that in the normal saline group at days 35 and 42. Also, numerically, the percentage of APOC3 in plasma compared to the day 0 baseline in the TRD008043 group was approximately 3 times lower than that in the TRD007972-1 group at D14-D35 after the single administration.

The results show that TRD008043 is more effective than TRD007972-1 in reducing human APOC3.

**Table 14. The percentage of TG in plasma compared to the day 0 baseline after a single administration of the test substances to APOC3 Tg mice (X ± SE)**

| Group | TG (percentage compared to day 0) | | | | | |
|---|---|---|---|---|---|---|
| | Day 7 | Day 14 | Day 21 | Day 28 | Day 35 | Day 42 |
| Normal saline | 86.36±8.35 | 97.92±13.02 | 106.96±9.25 | 110.12±17.72 | 98.09±12.82 | 84.22±8.90 |
| TRD008043 3mg/kg | 15.17±3.36*** | 17.02±4.53*** | 18.42±4.93*** | 21.77±5.66** | 26.17±6.95 | 36.01±6.95 |
| TRD007972-1 3mg/kg | 28.78±5.87*** | 41.20±9.06** | 49.20±9.83** | 67.68±16.12 | 114.80±40.98 | 74.58±7.84 |

| | | | | | | |
|---|---|---|---|---|---|---|
| ****p* < 0.001, ***p* < 0.01, **p* < 0.05, all compared to the normal saline group, One-way ANOVA Dunnett's test | | | | | | |

The results in Table 14 show that after the single administration of the test samples to APOC3 Tg mice, by day 28, TRD008043 still retained the effect of significantly reducing plasma TG levels compared to the normal saline group, whereas TRD007972-1 did not show a significant difference from the normal saline group anymore. Also, numerically, the percentage of TG in plasma compared to the day 0 baseline in the TRD008043 group was approximately 2-4 times lower than that in the TRD007972-1 group at D14-D35 after the single administration.

The results show that TRD008043 is more effective than TRD007972-1 in reducing TG.

**Table 15. The percentage of TC in plasma compared to the day 0 baseline after a single administration of the test substances to APOC3 Tg mice (X ± SE)**

| Group | TC (percentage compared to day 0) | | | | | |
|---|---|---|---|---|---|---|
| | Day 7 | Day 14 | Day 21 | Day 28 | Day 35 | Day 42 |
| Normal saline | 86.45±8.49 | 99.41±14.27 | 109.96±12.78 | 98.10±14.34 | 91.89±14.95 | 82.03±10.27 |
| TRD008043 3mg/kg | 42.70±9.21* | 45.45±11.65* | 47.60±11.42* | 44.92±10.51* | 42.59±11.75 | 49.61±10.11 |
| TRD007972-1 3mg/kg | 66.54±6.80 | 75.07±6.34 | 80.71±6.53 | 89.51±8.64 | 79.62±8.92 | 85.56±7.90 |

| | | | | | | |
|---|---|---|---|---|---|---|
| *p < 0.05, compared to the normal saline group, One-way ANOVA Dunnett's test | | | | | | |

The results in Table 15 show that after the single administration of the test samples to APOC3 Tg mice, by day 28, TRD008043 still retained the effect of significantly reducing plasma TC levels compared to the normal saline group, whereas TRD007972-1 did not show a significant difference from the normal saline group. Also, numerically, the percentage of TC in plasma compared to the day 0 baseline in the TRD008043 group was approximately 2 times lower than that in the TRD007972-1 group at D14-D35 after the single administration.

The results show that TRD008043 is more effective than TRD007972-1 in reducing TC.

## Claims

1. A double-stranded ribonucleic acid (dsRNA) comprising a sense strand and an antisense strand that comprise contiguous nucleotides in a direction from the 5' end to the 3' end, wherein:
the nucleotides at positions 7, 8, and 9 of the sense strand are 2'-fluoro-modified nucleotides, the nucleotide at position 5 is independently a 2'-methoxy-modified nucleotide or 2'-fluoro-modified nucleotide, and the nucleotides at the other positions are 2'-methoxy-modified nucleotides;
the nucleotides at positions 2 and 14 of the antisense strand are 2'-fluoro-modified nucleotides, the nucleotides at positions 4, 6, 8, 9, 10, 12, 16, and 18 are independently 2'-methoxy-modified or 2'-fluoro-modified nucleotides, and the nucleotides at the other positions are 2'-methoxy-modified nucleotides;
the number of 2'-fluoro-modified nucleotides in the antisense strand is 2-7;
the antisense strand comprises a chemical modification represented by formula (I), a tautomer thereof, or a pharmaceutically acceptable salt thereof at nucleotide position 7:
the chemical modification represented by formula (I) is selected from the group consisting of any one of the following structures: 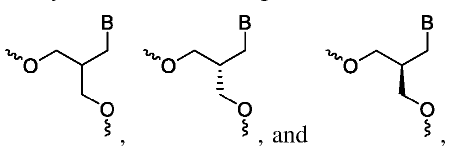 wherein B is the base of the nucleotide at position 7 of the 5' region of the antisense strand when unmodified;
and
the dsRNA inhibits the expression of apolipoprotein C3 (APOC3).

2. The dsRNA according to claim 1, wherein:
the sense strand comprises a nucleotide sequence represented by the following formula: 5'-NₐNₐNₐNₐNₐNₐN_{b}N_{b}N_{b}NₐNₐNₐNₐNₐNₐNₐNₐNₐNₐ-3';
wherein Nₐ is a 2'-methoxy-modified nucleotide, and N_{b} is a 2'-fluoro-modified nucleotide.

3. The dsRNA according to claim 1 or 2, wherein:
the antisense strand comprises a nucleotide sequence represented by the following formula:
5'-Nₐ'N_{b}'Nₐ'X'Nₐ'X'W'X'X'X'Nₐ'X'Nₐ'N_{b}'Nₐ'X'Nₐ'X'Nₐ'Nₐ'Nₐ'-3';
preferably, the antisense strand comprises a nucleotide sequence represented by the following formula:
5'-Nₐ'N_{b}'Nₐ'Nₐ'Nₐ'N_{b}'W'Nₐ'Nₐ'Nₐ'Nₐ'N_{b}'Nₐ'N_{b}'Nₐ'N_{b}'Nₐ'Nₐ'Nₐ'Nₐ'Nₐ -3^{,};
5'-Nₐ'N_{b}'Nₐ'N_{b}'Nₐ'N_{b}'W'Nₐ'Nₐ'N_{b}'Nₐ'N_{b}'Nₐ'N_{b}'Nₐ'N_{b}'Nₐ'Nₐ'Nₐ'Nₐ'Nₐ'-3';
5'-Nₐ'N_{b}'Nₐ'N_{b}'Nₐ'N_{b}'W'Nₐ'Nₐ'N_{b}'Nₐ'Nₐ'Nₐ'N_{b}'Nₐ'N_{b}'Nₐ'N_{b}'Nₐ'Nₐ'Nₐ'-3';
5'-Nₐ'N_{b}'Nₐ'N_{b}'Nₐ'N_{b}'W'Nₐ'Nₐ'Nₐ'Nₐ'N_{b}'Nₐ'N_{b}'Nₐ'N_{b}'Nₐ'N_{b}'Nₐ'Nₐ'Nₐ'-3';
5'-Nₐ'N_{b}'Nₐ'Nₐ'Nₐ'N_{b}'W'Nₐ'Nₐ'N_{b}'Nₐ'N_{b}'Nₐ'N_{b}'Nₐ'N_{b}'Nₐ'N_{b}'Nₐ'Nₐ'Nₐ'-3';
5'-Nₐ'N_{b}'Nₐ'N_{b}'Nₐ'N_{b}'W'Nₐ'Nₐ'Nₐ'Nₐ'Nₐ'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'N_{b}'W'Nₐ'Nₐ'N_{b}'Nₐ'Nₐ'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'N_{b}'W'Nₐ'Nₐ'N_{b}'Nₐ'Nₐ'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'N_{b}'W'Nₐ'Nₐ'N_{b}'Nₐ'Nₐ'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'N_{b}'W'Nₐ'Nₐ'N_{b}'Nₐ'Nₐ'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'N_{b}'W'Nₐ'Nₐ'N_{b}'Nₐ'Nₐ'Nₐ'N_{b}'Nₐ'N_{b}'Nₐ'Nₐ'Nₐ'Nₐ'Nₐ'-3';
5'-Nₐ'N_{b}'Nₐ'Nₐ'Nₐ'N_{b}'W'Nₐ'Nₐ'N_{b}'Nₐ'Nₐ'Nₐ'N_{b}'Nₐ'N_{b}'Nₐ'Nₐ'Nₐ'Nₐ'Nₐ'-3';
wherein each X' is independently Nₐ' or N_{b}'; Nₐ' is a 2'-methoxy-modified nucleotide, and N_{b}' is a 2'-fluoro-modified nucleotide;
W' represents a nucleotide comprising a chemical modification represented by formula (I), a tautomer thereof, or a pharmaceutically acceptable salt thereof.

4. The dsRNA according to any one of claims 1-3, wherein at least one phosphoester group in the sense strand and/or the antisense strand is a phosphoester group having a modification group, preferably a phosphorothioate group, and more preferably a phosphorothioate diester group.

5. The dsRNA according to claim 4, wherein the phosphorothioate diester group is present at at least one of the following positions:
between the first and second nucleotides of the 5' end of the sense strand;
between the second and third nucleotides of the 5' end of the sense strand;
between the first and second nucleotides of the 5' end of the antisense strand;
between the second and third nucleotides of the 5' end of the antisense strand;
between the first and second nucleotides of the 3' end of the antisense strand; and
between the second and third nucleotides of the 3' end of the antisense strand;
preferably, the sense strand and/or the antisense strand comprise(s) a plurality of phosphorothioate diester groups, and the phosphorothioate diester groups are present:
between the first and second nucleotides of the 5' end of the sense strand; and,
between the second and third nucleotides of the 5' end of the sense strand; and,
between the first and second nucleotides of the 5' end of the antisense strand; and,
between the second and third nucleotides of the 5' end of the antisense strand; and,
between the first and second nucleotides of the 3' end of the antisense strand; and,
between the second and third nucleotides of the 3' end of the antisense strand.

6. The dsRNA according to any one of claims 1-5, wherein:
the sense strand comprises at least 15 contiguous nucleotides that differ from the nucleotide sequence of SEQ ID NO: 1 by no more than 3 nucleotides; and
the antisense strand comprises at least 19 contiguous nucleotides that differ from the nucleotide sequence of SEQ ID NO: 2 by no more than 3 nucleotides;
preferably,
the sense strand comprises the nucleotide sequence set forth in SEQ ID NO: 1, and the antisense strand comprises the nucleotide sequence set forth in SEQ ID NO: 2.

7. The dsRNA according to any one of claims 1-6, wherein:
the antisense strand comprises the nucleotide sequence set forth in any one of SEQ ID NO: 12, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 13, and SEQ ID NO: 14;
preferably, the sense strand comprises the nucleotide sequence set forth in SEQ ID NO: 3.

8. The dsRNA according to any one of claims 1-7, wherein the dsRNA further comprises a ligand conjugated thereto, and the ligand comprises N-acetyl-galactosamine.

9. The dsRNA according to claim 8, wherein:
the ligand has the following structure or a pharmaceutically acceptable salt thereof:

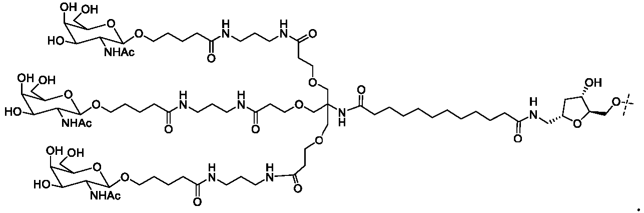

10. The dsRNA according to any one of claims 8 or 9, wherein the 3' end of the sense strand of the dsRNA is conjugated to the ligand.

11. The dsRNA according to any one of claims 8-10, wherein the ligand is linked to the 3' end of the sense strand of the dsRNA by a phosphoester group or a phosphorothioate group, preferably by a phosphodiester group or a phosphorothioate diester group, and more preferably by a phosphodiester group.

12. The dsRNA according to any one of claims 1-11, wherein:
the antisense strand comprises the nucleotide sequence set forth in any one of SEQ ID NO: 12, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 13, and SEQ ID NO: 14;
preferably, the sense strand comprises the nucleotide sequence set forth in SEQ ID NO: 4.

13. The dsRNA according to any one of claims 1-12, wherein the dsRNA has the following structure or a pharmaceutically acceptable salt thereof: 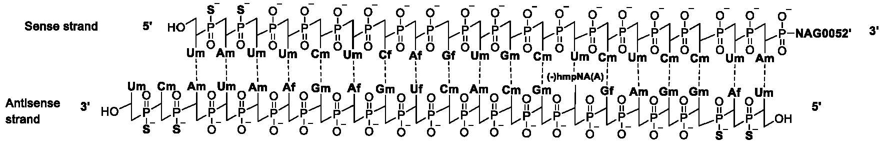
wherein Af = adenine 2'-F ribonucleoside; Cf = cytosine 2'-F ribonucleoside; Uf = uracil 2'-F ribonucleoside; Gf = guanine 2'-F ribonucleoside; Am = adenine 2'-OMe ribonucleoside; Cm = cytosine 2'-OMe ribonucleoside; Gm = guanine 2'-OMe ribonucleoside; Um = uracil 2'-OMe ribonucleoside; 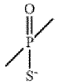
represents a phosphorothioate diester group, and 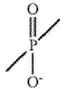
represents a phosphodiester group;
NAG0052' represents 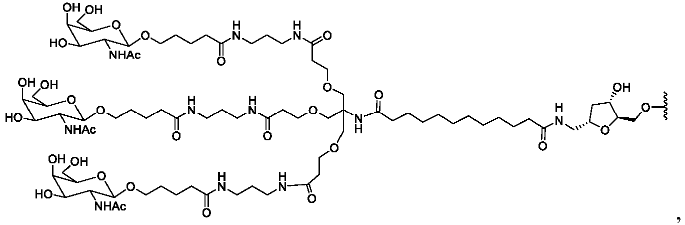 and (-)hmpNA(A) represents

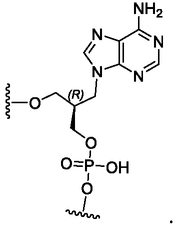

14. A pharmaceutical composition comprising the dsRNA according to any one of claims 1-13, wherein optionally, the pharmaceutical composition further comprises one or more pharmaceutically acceptable excipients.

15. A vector comprising the dsRNA according to any one of claims 1-13.

16. A cell comprising the dsRNA according to any one of claims 1-13, or the vector according to claim 15.

17. Use of the dsRNA according to any one of claims 1-13, the pharmaceutical composition according to claim 14, the vector according to claim 15, or the cell according to claim 16 in the preparation of a medicament, wherein:
the medicament is used for lowering triglyceride levels in a subject, or for preventing and/or treating a disease mediated by elevated triglyceride levels or elevated cholesterol levels; preferably, the disease mediated by elevated triglyceride levels or elevated cholesterol levels is selected from the group consisting of hypertriglyceridemia, obesity, hyperlipidemia, abnormal lipid and/or cholesterol metabolism, atherosclerosis, cardiovascular disease, coronary artery disease, hypertriglyceridemia-induced pancreatitis, metabolic syndrome, type II diabetes, familial chylomicronemia syndrome, and familial partial lipodystrophy.

18. A method for inhibiting the expression of the APOC3 gene or an mRNA thereof, comprising administering to a subject an effective amount or effective dose of the dsRNA according to any one of claims 1-13, the pharmaceutical composition according to claim 14, the vector according to claim 15, or the cell according to claim 16.

19. A method for delivering an oligonucleotide to a liver, comprising administering to a subject an effective amount or effective dose of the dsRNA according to any one of claims 8-13, the pharmaceutical composition according to claim 14, the vector according to claim 15, or the cell according to claim 16.

20. A kit comprising the dsRNA according to any one of claims 1-13, the pharmaceutical composition according to any one of claim 14, the vector according to claim 15, or the cell according to claim 16.

21. A method for preparing a dsRNA or a pharmaceutical composition, comprising: synthesizing the dsRNA according to any one of claims 1-13.
